# FASCICULE DE BREVET EUROPEEN

(11) **EP 2 521 725 B1**
(45) Date de publication et mention de la délivrance du brevet: **27.11.2013**
(21) Numéro de dépôt: 11704257.2
(22) Date de dépôt: 06.01.2011
(51) Int. Cl.: C07D 471/04, C07C 311/21, C07F 5/02, A61P 9/00, A61K 31/519

(54) **DÉRIVÉS DE PYRIDINO-PYRIDINONES ARYLSULFONAMIDES, LEUR PRÉPARATION ET LEUR APPLICATION EN THÉRAPEUTIQUE**
ARYLSULFONAMIDPYRIDIN-PYRIDINON-DERIVATE, DEREN HERSTELLUNG UND THERAPEUTISCHE VERWENDUNG DAVON
ARYLSULFONAMIDE PYRIDINE-PYRIDINONE DERIVATIVES, PREPARATION OF SAME, AND THERAPEUTIC USE THEREOF

(30) Priorité: 07.01.2010 FR 1050081
(43) Date de publication de la demande: 14.11.2012
(73) Titulaire: SANOFI, 75008 Paris (FR)
(72) Inventeur: BELLEVERGUE, Patrice, F-75013 Paris (FR); GRAILHE, Patrick, F-75013 Paris (FR); MCCORT, Gary, F-75013 Paris (FR); O'CONNOR, Stephen, F-75013 Paris (FR); DUCLOS, Olivier, F-75008 Paris (FR)
(74) Mandataire: Catillon, Marie
(86) Numéro de dépôt international: PCT/FR2011/050018
(87) Numéro de publication internationale: WO 2011/083275

(56) Documents cités:
- WO-A2-2010/004197
- FR-A1- 2 917 412
- FR-A1- 2 917 413

## Description

La présente invention se rapporte à des dérivés de pyridino-pyridinones substitués en position 7 par un arylsulfonamide, à leur préparation et à leur application en thérapeutique en tant qu'inhibiteurs de protéines kinases telles que p70S6 (S6K1) et/ou de PDGFR-TK (Platelet derived growth factors), ou d'autres kinases.

### 1) La protéine kinase p70S6K :

### I. La p70 S6Kinase ou S6K1 : généralités, structure, activation

La p70 ribosomale S6 Kinase (S6K1, précédemment p70S6K) est une sérine/thréonine kinase (de la famille des AGC kinases) de la voie PI3-Kinase/mTOR parmi les premières décrites comme activée par l'insuline et de nombreux facteurs de croissance. Cette kinase participe à la régulation de deux processus cellulaires : la synthèse protéique et la croissance cellulaire (prolifération et taille des cellules), par l'intermédiaire de son principal substrat, la protéine ribosomale S6 de la sous unité 40S. (Avruch J. 2001). Clonée en 1991 par Grove *et al*. deux isoformes, résultant d'un épissage alternatif de l'ARNm, codent pour 2 séquences protéiques: p85 S6K (α-I, 525 amino-acides) et p70S6K (α-II, 502 amino-acides) ont été identifiées. Cette dernière isoforme est principalement localisée dans le cytosol alors que l'isoforme α-I est nucléaire (présence d'un site de localisation nucléaire sur l'extension N terminale de 23 amino-acides). La S6K1 est exprimée de façon ubiquitaire.

La S6K1 présente une homologie de 70% en amino-acide avec la S6K2 (précédemment p70 beta S6 kinase), également activée par mTOR, dans laquelle 7 sites de phosphorylation (sérine ou thréonine) sont conservés.

La structure de la S6K1 comprend quatre modules: un domaine non catalytique à l'extrémité N-terminale (I), un domaine catalytique central (II), une extension du domaine kinase (III) et enfin un domaine auto-inhibiteur à l'extrémité C-terminale (IV). L'activation de cette kinase nécessite la phosphorylation séquentielle en 4 étapes de sites serine ou thréonine localisés sur différents domaines qui vont modifier sa conformation globale lui permettant d'acquérir son activité enzymatique (Pollen N. 1997, Dennis JBC1998).

### II. La S6K1 dans la voie de signalisation PI3K/ mTOR

La signalisation en amont de la S6K1 résulte de l'activation de nombreux Récepteurs Couplés aux Protéines G (RCPG), membranaires, qui contrôlent la croissance cellulaire, la prolifération et la différenciation. Après liaison de ligands tels les Facteurs de croissance (par exemple le PDGF, l'EGF), les nutriments ou hormones (par exemple les amino-acides, le glucose ou l'insuline), l'activation de leurs récepteurs aboutit au recrutement de la PI3-Kinase déclenchant une cascade de phosphorylation via PDK1 qui phosphoryle Akt, activant mTOR (via TSC1/2 et Rheb) qui enfin active la S6K1, un des deux principaux effecteurs de mTOR. Enfin, la protéine pro-apoptotique BAD est phosphorylée en S136 par la S6K1 qui l'inactive et améliore la survie cellulaire (Harada et al. PNAS 2001)

Plus récemment, la Chaperonine Contenant TCP1, CCT, a été rapportée comme substrat de S6K1 et joue un rôle dans le repliement de protéine néo-synthétisées comme l'actine, la tubuline et plusieurs protéines du cycle cellulaire suggérant également un rôle de la S6K1 dans la régulation du cycle cellulaire (Abe et al. JBC2009)

### III. Applications des inhibiteurs de S6K1

De part son activité régulatrice de la croissance cellulaire et de la synthèse protéique, la S6K1 est impliquée dans de nombreux processus physiopathologiques. Les inhibiteurs de S6K1 peuvent donc trouver des applications dans de nombreux domaines thérapeutiques: les maladies cardiovasculaires telles que l'insuffisance cardiaque consécutive à une hypertrophie du myocarde, l'athérosclérose et la re-sténose suite à une prolifération excessive des cellules musculaires lisses des artères ou l'insuffisance rénale. Les troubles métaboliques et notamment le diabète et l'obésité représentent d'autres applications thérapeutiques possibles pour des inhibiteurs de S6K1. Les maladies fibrotiques, telle que la fibrose hépatique, pancréatique, pulmonaire, cardiaque et péri-vasculaire découlant d'un excès de synthèse de matrice extracellulaire et de prolifération de fibroblastes, de cellules stellaires ou cellules musculaires lisses, régulés entre autre par l'activité de la S6K1, constituent également des indications thérapeutiques pour ces inhibiteurs. Enfin, toutes les tumeurs comportant des dérégulations de la voie PI3K/Akt/mTOR pourraient bénéficier du traitement par des inhibiteurs de la S6K1.

### IV. Rôle de la S6K1 dans le système cardiovasculaire

L'hypertrophie des cardiomyocytes due à un excès de synthèse protéique est un des mécanismes clés impliqué dans le développement de l'hypertrophie du myocarde qui aboutit à l'insuffisance cardiaque. La voie de signalisation mTOR/S6K1 est un des principaux systèmes de régulation de la croissance cellulaire en régulant la synthèse protéique et la prolifération cellulaire. De nombreuses études *in vivo* ont montré le potentiel thérapeutique d'inhibiteurs de cette voie dont la rapamycine, inhibiteur de mTOR (complexe mTORC1) qui bloque l'activation de la S6K1. La rapamycine diminue l'hypertrophie cardiaque suite à une surcharge cardiaque par constriction de l'aorte chez la souris ou le rat (Gao et al. Hypertension 2006, Boluyt M. et al. Cardiovasc. Drug Therap. 2004, Shioi et al. Circulation 2003). La rapamycine diminue l'hypertrophie du ventricule gauche, préserve la fonction contractile et diminue la fibrose cardiaque (diminution du collagène) par un mécanisme passant par la voie mTOR/S6K1 puisque la phosphorylation de la protéine ribosomale S6 et elF4E est inhibée (Gao J Hypertension 2006).

L'implication de la voie mTOR/S6K1 dans l'hyperplasie des cellules musculaires lisses d'artère est démontrée par le rôle inhibiteur de la rapamycine dans la croissance des cellules musculaires lisse d'artère *in vitro* et a été utilisé dans la prévention de la resténose de l'artère coronaire après angioplastie transluminale en utilisant des stents recouverts de rapamycine (Moses et al. N Engl. J. Med. 2003) ou après injection systémique (ORAR Trial, Rodriguez et al. J. Invasive cardiol. 2003). En particulier chez les patients diabétiques, une étude clinique a montré que les stents imprégnés de rapamycine diminuent de façon significative les risques de re-sténose après angioplastie coronaire (SIRIUS Substudy, Moussa et al. Circulation 2004). Les composés de la présente invention pourraient donc avoir une application dans la prévention de la resténose et de l'athérosclérose.

### V. Rôle de la S6K1 dans le processus de Fibrose

La réparation tissulaire excessive suite à des lésions/stimuli chroniques aboutissant à un excès de synthèse de matrice extracellulaire et différentiation de fibroblaste en myofibroblaste caractérise le processus de fibrose qui se produit dans de nombreux tissus. De part son activité de régulation de la synthèse protéique et de la croissance cellulaire, la S6K1 est très impliquée dans les fibroses; les inhibiteurs de la présente invention peuvent donc trouver des applications dans la fibrose du foie, du pancréas, de la peau, du poumon, du coeur ou du rein.
Le rôle de la S6K1 dans la fibrose hépatique et notamment dans le processus d'activation des cellules stellaires hépatiques (pour revue cf Parsons J. Gastro. Hepatol. 2007) a été mis en évidence dans des expériences *in vivo* dans le modèle de fibrose hépatique chez le rat par ligation du canal biliaire ou l'inhibition de mTOR par la rapamycine diminue l'activation de la S6K1, réduit la fibrose et améliore l'hypertension portale, effet fonctionnel accompagné d'une diminution de l'ARNm de TGFβ, CTGF, PDGFβ ainsi qu'une diminution de la S6K1 phosphorylée (Biecker et al. JPET 2005). Dans un autre modèle de fibrose du foie induite chez le rat par le tetrachlorure de carbone, la carbone, la rapamycine réduit les dépôts de collagène et l'activité trans-glutaminase *in vivo* et bloque complètement la prolifération des cellules stellaires induite par le PDGFβ (Zhu et al. Gastroenterology 1999). Dans 2 modèles de fibrose hépatique induite par ligation du canal biliaire (BDL) ou par injection de dimethylnitrosamine (DMN), une étude cinétique *ex vivo* de l'activité d'ERK1/2 et de la S6K1 a montré que l'activation de cette kinase précède l'activation et la prolifération des cellules stellaires hépatiques, le pic d'activité de S6K1 étant à 6 heures dans le modèle DMN et à 72heures dans le modèle BDL (Svegliati-Baroni et al. J. Hepatol. 2003). In vitro, l'activation des cellules stellaires par le PDGFb et l'IGF-1 implique la S6K1 et la rapamycine inhibe la prolifération des cellules stellaires et l'activation de la S6K1 (Bridle et al. JLCM 2006). Cet inhibiteur s'est également avéré capable de bloquer la surexpression de MMP13, de collagène I est l'activation de la S6K1 dans des cellules stellaires activées par le TGFβ (Lechuga et al. J. AJPGLP 2004).
Au niveau de la fibrose de la peau, une expression élevée de la S6K1 a été mise en évidence dans les cicatrices chéloïdes. La rapamycine diminue le collagène, la fibronectine, l'actine α (α SMA) (Ong et al. Exp. Dermatol. 2007).
Au niveau de la fibrose pulmonaire, la rapamycine empêche l'initiation et la progression de la fibrose pulmonaire dans un mdèle de souris transgénique sur-exprimant le TGFα dans le poumon. De plus, cet inhibiteur bloque la phosphorlyation de la S6K1 induite par le TGFα et les dépots de collagène dans le poumon (Korfhagen et al. Am. J. resp. Cell Mol. Biol. 2009)

### VI. Oncologie:

Des inhibiteurs de la P70S6K ont des applications en oncologie, notamment dans:
- le cancer du sein ou le gène de la S6K1 amplifié et surexprimé est présent dans 7.5-10.2% cancer primaire du sein (Barlund *et al.* 2000, Wu *et al.* 2000, Couch *et al.* 1999), cette surexpression est associée à un mauvais prognostic indépendant de l'amplification d'HER2 (Barlund *et al.* 2000).
- le cancer du foie (hépatocarcinome, HCC) : surexpression de la S6K1 rapportée par Sahin *et al* (2004).
- le glioblastome (Riemenschneider *et al.* 2006)
- le cancer de la thyroïde (Miyakawa *et al*. 2003)
- les cancers de l'ovaire et du col de l'utérus (Wong *et al.* 2000). La S6K1 favorise le processus de Transition des cellules Epithéliales en cellules Mésenchymateuses (EMT) dans les cellules de cancer de l'ovaire (Pon et al. Cancer Res. 2008)

### 2) Les tyrosine kinases des récepteurs PDGF-R :

Les récepteurs PDGF-R sont des membres de classe III de la famille des récepteurs à tyrosine kinases (RTK). La fixation des ligands sur les RTK induit la dimérisation des récepteurs, l'activation de leur partie tyrosine kinase qui conduit à la transphosphorylation des résidus tyrosine (Weiss & Schlessinger, 1998).
La fixation des ligands sur ces RTK induit la dimérisation des récepteurs, l'activation de leur partie tyrosine kinase qui conduit à la transphosphorylation des résidus tyrosine (Weiss & Schlessinger, 1998). Ces résidus phosphorylés servent ainsi de point d'ancrage aux protéines intracellulaires de la signalisation qui *in fine* provoquent différentes réponses cellulaires : la maintenance, la division, la prolifération, la différentiation, ou bien encore la migration cellulaire. (Claesson-Welsh, 1994).

Deux isoformes de récepteurs aux PDGFs ont été mis en évidence, la chaine PDGF-Ralpha et la chaine PDGF-Rbeta, qui suite à la fixation de leurs ligands s'homo- ou s'hétérodimérisent et induisent la signalisation intracellulaire. Les récepteurs aux PDGF sont essentiellement exprimés par les cellules d'origine mésenchymateuse et sont notamment retrouvés sur les fibroblastes, les cellules musculaires lisses, les péricytes et les cellules gliales (Ross et coll.1986, Heldin, 1992).
Le « Platelet Derived Growth Factor », PDGF, protéine de poids moléculaire d'environ 30.000 daltons, est sécrété essentiellement par les plaquettes, accessoirement par l'endothélium, les muscles lisses vasculaires et les monocytes. Il est formé de deux chaînes polypeptidiques reliées entre elles par des ponts disulfures formant soit des homodimères, soit des hétérodimères. Quatre gènes (7p22, 22q13, 4q31 et 11q22) ont été décrits comme codant pour 4 différentes chaines polypeptiques (A, B C et D), qui une fois dimérisées donnent cinq ligands biologiquement actifs PDGF-AA, BB, CC, DD et AB (pour revue, Yu et al, 2003). Une spécificité de liaison existe, dont notamment le PDGF-AA pour l'isoforme alpha du récepteur, le PDGF-D pour la forme BB, et le PDGF-C pour la forme alpha et alpha/beta. Les PDGFs ligands sont de puissants mitogènes, mais sont également impliqués dans les phénomènes de migration, survie, apoptose et transformation cellulaire.

Les inhibiteurs de la fonction de PDGF-R alpha, beta interviennent dans différents domaines thérapeutiques. Parmi les phénomènes physiopathologiques où ces récepteurs peuvent être impliqués on retrouve les cancers avec ou sans métastases ciblant les cellules tumorales et/ou les cellules de l'environnement tumoral (vasculaires, fibroblastiques), les fibroses et les maladies vasculaires :
De façon intéressante, les cellules blastiques de type LAM (leucémie aiguë myéloïde) peuvent également surexprimer d'autres récepteurs à activité kinase comme c-kit ou bien encore PDGF-R.

### Les syndromes myéloprolifératifs/dysplasiques

De façon assez fréquente, des anormalités cytogénétiques suite à des translocations chromosomiques ont été reportées dans les syndromes myéloprolifératifs. Ces réarrangements génèrent des protéines de fusion à activité tyrosine kinase dérégulées impliquées dans la prolifération des cellules myéloides blastiques.

### -protéines de fusion à activité kinase PDGF-R beta

Les protéines de fusion à activité kinase PDGF-R beta sont constituées de la partie intracellulaire de PDGF-R-beta et d'autre part d'un domaine N-ter d'une autre protéine (en général un facteur de transcription). Ont été reportés notamment dans les leucémies myélomonocytaire chronique (LMMC): Rab5/PDGF-Rbeta, H4-PDGF-Rbeta, HIP1-PDGF-RB ou bien encore Tel/PDGF-R beta. Cette dernière est la plus représentée. Elle est issue de la translocation t(5;12)(q31;p12) et code pour une protéine de fusion constituée de la partie N-terminale du facteur de transcription Tel et de la partie C-terminal de PDGF-Rbeta. Un domaine d'oligomérisation présent dans la partie Tel, conduit à une forme dimérisée de la protéine de fusion et à l'activité constitutive du domaine kinase. Cette protéine a été montrée *in vitro* comme capable de transformer des cellules hématopoïétiques à plusieurs reprises et notamment de façon détaillée dans l'article de M. Carrol et coll. (PNAS, 1996, 93, 14845-14850). *In vivo,* cette protéine de fusion conduit à un syndrome d'hyperprolifération des cellules myéloïdes (Ritchie et coll., 1999).
De plus, chez l'animal comme en clinique chez l'homme, il a été montré que des inhibiteurs de l'activité tyrosine kinase inhibent la prolifération des cellules blastiques et permettent d'enrayer le processus de leucémogénèse.

### -protéines de fusion à activité kinase PDGF-R alpha

Deux protéines de fusion impliquant PDGF-R alpha ont été reportées: bcr-PDGF-Ralpha présente dans une leucémie myéloide chronique (CML) atypique et FIP1L1-PDGF-Ralpha retrouvée dans une sous-population de leucémies, les LEC « leucémies à éosinophiles », provenant d'un syndrome d'hyper-éosinophilie (Griffin et coll. 2003). Cette protéine de fusion porte une activité constitutive du domaine kinase de PDGF-R alpha et est responsable de la prolifération anarchique de ces cellules.

Des inhibiteurs de l'activité kinase de PDGF-R alpha ont montré l'efficacité sur la prolifération de cellules FIP1L1-PDGF-R alpha positives et récemment un composé inhibiteur a reçu l'indication pour les HES/CEL.

Ainsi, inhiber l'activité kinase de PDGF-Ralpha et beta comme le font les composés de l'invention, s'avèrent d'intérêt thérapeutique pour les LAM.

### A. Cancers solides

Des inhibiteurs de l'activité tyrosine kinase des récepteurs PDGF-R alpha et beta peuvent être d'intérêt pour les cancers solides soit en ciblant directement la cellule tumorale qui par autocrinie ou paracrinie est sensible à l'activité d'inhibiteur TK de PDGF-R, soit en ciblant les cellules de l'environnement en déstabilisant le réseau pour favoriser l'association avec d'autres agents thérapeutiques. Des exemples de cancers solides sont le sarcome d'Ewing, les tumeurs stromales gastro-intestinales (GIST), les dermatofibrosarcomes, les gliomes, les glioblastomes, les hémangiomes ainsi que les tumeurs desmoïdes. Les composés de l'invention sont d'intérêt pour le traitement de tels cancers solides.

### B. Cibler PDGF-RTK dans l'environnement tumoral

### Angiogénèse

Les cellules de l'environnement de la tumeur font partie intégrante du développement du cancer que ce soit dans le cas d'une tumeur primaire ou secondaire (métastases). Parmi les cellules de l'environnement qui expriment PDGF-R et pour lesquelles le rôle de ce récepteur a été mis en évidence, on retrouve les cellules murales des vaisseaux c'est à dire les péricytes et les cellules musculaires lisses mais aussi les fibroblastes activés.
L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d'angioblastes à partir de la moelle osseuse sont observées dans les processus de néovascularisation des tumeurs. Il a été montré *in vitro* et *in vivo* que plusieurs facteurs de croissance stimulent la prolifération endothéliale comme le VEGF et les FGF. Outre ces mécanismes, il a été également mis en évidence que les cellules murales comme les péricytes et les cellules musculaires lisses participent à la stabilisation des vaisseaux néoformés. L'invalidation de PDGF-R beta cause un déficit des péricytes chez la souris et conduit à la mort des animaux en fin de gestation due à des micro-hémorragies et des oedèmes (Hellström et al, 1999, Hellström et al, 2001). Dans une élégante étude de transplantation, l'expression de PDGF-R-beta par les péricytes a été montrée nécessaire pour leur recrutement au niveau des vaisseaux tumoraux via la rétention de PDGF-B par les cellules endothéliales mais aussi par le PDGF-B sécrété par les cellules tumorales (Abramsson et al, 2003). Dans le modèle transgénique Rip1Tag2 de tumeur pancréatique, Song et coll. ont également montré l'expression de PDGF-R beta sur les progéniteurs périvasculaires dans la moelle issus de la moelle osseuse, progéniteurs qui se différencient en péricytes matures autour de la tumeur.
L'intérêt de bloquer l'activité de PDGF-R sur les péricytes tumoraux a été démontré par l'utilisation d'inhibiteur de l'activité tyrosine kinase de PDGF-R dans des modèles animaux (modèle transgénique de tumeur du pancréas et implantation de tumeur de glioma), et l'effet sur la croissance tumorale s'avère profond en association avec un inhibiteur de l'activité kinase de VEGF-R (Bergers et coll., 2003). Des données de la littérature (Cao et al, 2002, Fons et coll., 2004) ont mis en évidence l'intervention de PDGF-R alpha et du PDGF-C en angiogénèse et dans la différenciation des progéniteurs endothéliaux vers les cellules de type péricytes et cellules musculaires lisses.
Au vu des ces différents travaux, il apparaît que les composés de l'invention sont d'intérêt pour le traitement des cancers solides par leur effet sur les cellules de l'environnement et ce en association avec d'autres agents thérapeutiques comme des cytotoxiques ou des inhibiteurs de l'angiogénèse.

### Fibroblastes activés

PDGF-R est abondant dans le stroma tumoral et est retrouvé sur les fibroblastes activés (myofibroblastes). Il a été montré dans deux études que l'association d'inhibiteurs ou antagonistes de PDGF-R avec des agents cytotoxiques conduit à une diminution de la microdensité des vaisseaux des cancers de l'ovaire (Apte et coll.2004) et des cancers du pancréas (Hwang et coll., 2003). PDGF-R beta régule la pression du tissu interstitiel de la tumeur (Heuchel et coll., 1999) et la co-administration des inhibiteurs de PDGF-R et des agents de chimiothérapie améliore leur délivrance dans les cellules tumorales en diminuant la pression intra-tumorale (Griffon-Etienne, 1999). Enfin dans un modèle murin, l'administration d'un inhibiteur de l'activité kinase de PDGF-R améliore la consommation d'agents de chimiothérapie par la tumeur et ainsi augmente leur efficacité (Griffon-Etienne, 1999; Pietras et coll., 2002; Pietras et coll., 2003). Les fibroblastes activés présents dans le stroma tumoral représentent donc une nouvelle cible thérapeutique en oncologie (pour revue voir Bouzin & Feron, 2007).

### Métastases

Plusieurs travaux indiquent que le couple PDGF-R et PDGF-ligand est impliqué dans le développement des métastases, certainement par leur action sur l'angiogénèse et la metastatisation par la circulation sanguine, mais aussi par un effet direct sur la lymphangiogénèse et donc les métastases disséminées par les vaisseaux lymphatiques. Une revue documente notamment le rôle direct du PDGF-BB dans la lymphangiogénèse et les métastases lymphatiques (Cao et coll., 2005). Mais la majorité des travaux impliquent l'expression de PDGF-R dans l'environnement des métastases qui favorisent la prise et le développement des tumeurs secondaires. L'exemple le plus fréquemment rapporté est le développement des métastases osseuses, du cancer de la prostate.
Au vu des ces différents travaux, il apparaît que les composés de l'invention sont d'intérêt pour le traitement des cancers solides par leur effet sur les cellules de l'environnement et ce en association avec d'autres agents thérapeutiques comme des cytotoxiques ou des inhibiteurs de l'angiogénèse.

### C. Fibroses

Les fibroses sont souvent la cause d'un événement primaire comme un cancer, le traitement par la radiothérapie, les hépatites, l'alcoolémie. L'implication de PDGF est clairement démontrée dans la fibrose pulmonaire (incluant asbestose), rénale (glomérulonéphrite), médullaire (souvent associé aux leucémies à mégacaryocytes), induite par la radiothérapie ainsi que la fibrose hépatique et pancréatique (liée à l'alcoolémie ou à des hépatites) (pour revue voir JC Bonner, 2004). Une surexpression de PDGF a été notamment clairement montrée et des résultats dans des modèles *vivo* avec des inhibiteurs de l'activité TK de PDGF-R ont été également rapportés. Parmi ces études, celle de Einter et coll. (2002) a montré que le PDGF-CC est un puissant inducteur de fibrose rénale. Les auteurs ont testé l'efficacité d'un anticorps neutralisant dans un modèle de ligature urétrale unilatérale, où la fibrose se développe particulièrement rapidement. Ils ont observé un effet antifibrosant très marqué avec une réduction de l'accumulation de myofibroblastes, une réduction de l'accumulation de matrice extracellulaire et une réduction des dépôts de collagène IV. Une autre étude conduite dans un modèle de fibrose pulmonaire induite par la Bléomycine chez la souris a montré l'efficacité d'un inhibiteur de l'activité TK de PDGF-R sur la prévention de la fibrose par inhibition de la prolifération des cellules mésenchymales (Aono et coll., 2005). Dans un modèle de fibrose induite par l'amiante, un inhibiteur de PDGF-R TK a réduit la progression de la fibrose dans le parenchyme pulmonaire et le dépot de collagène (Vuorinen K, Gao F, Oury TD, Kinnula VL, Myllärniemi M. Imatinib mesylate inhibits fibrogenesis in asbestos-induced interstitial pneumonia. Exp Lung Res. 2007 Sep;33(7):357-73). Plusieurs équipes ont montré l'implication de PDGF-R dans la fibrose hépatique. Il est clairement montré que PDGFBB et DD possèdent des caractéristiques pro-fibrogéniques sur les cellules stellates hépatiques (Rovida et coll., 2008; Borkham-Kamphorst et coll., 2007). In vivo, un inhibiteur de PDGF-R TK est capable de réduire la fibrogénèse précoce dans un modèle de ligature du canal biliaire chez le rat (Neef et coll., 2006).
Aussi, au vue des données de la littérature, les composés de l'invention semblent d'intérêt thérapeutique pour les différents types de fibrose.

### D. Maladies vasculaires : athérosclérose & resténose, artériosclérose

La prolifération et la migration de cellules musculaires lisses vasculaires contribuent à l'hypertrophie intimale des artères et joue ainsi un rôle prépondérant dans l'athérosclérose et dans la resténose après angioplastie et endoarterectomie. Il a été clairement démontré *in vitro* et *in vivo* dans des modèles animaux, que PDGF est impliqué dans ces phénomènes. In vivo, il a été montré notamment une augmentation de l'expression de PDGF dans un modèle « Vein graft » chez le cochon. De plus, il a été également montré qu'un inhibiteur de l'activité TK de PDGF-R diminuait de façon conséquente la taille des lésions de l'artère thoracique et abdominale de souris diabetiques ApoE-KO (animaux traités à la streptozotocin), Une autre étude a montré que l'inhibition de la signalisation induite par PDGF (TK or PDGF A antisens) conduit à une diminution de la formation de la neointima formation dans les modèles « balloon injury » et de « coronary artery restenosis ». (Deguchi J, 1999, Ferns et coll. 1991, Sirois et al, 1997, Lindner et coll. 1995)

Ainsi, des inhibiteurs de l'activité tyrosine kinase de PDGF-R, tels que les composés de la présente invention représentent une thérapie de choix, soit seul, soit en association avec des composés antagonistes d'autres facteurs de croissance impliqués dans ces pathologies comme le FGF, dans le traitement des pathologies liées à la prolifération des cellules musculaires lisses vasculaires telles que l'athérosclérose, la resténose post-angioplastie ou suite à la pose de prothèses endovasculaires (stents) ou lors de pontages aorto-coronariens.

Les composés de l'invention de par leur activité inhibitrice de l'activité TK de PDGF-R s'avèrent intéressantes pour traiter ces maladies vasculaires.

### E. Autres

D'autres pathologies semblent pouvoir être des indications pour les composés de l'invention dont l'hypertension artérielle pulmonaire (HTAP) idiopathique. L'HTAP caractérisée par une élévation importante et continue de la pression dans l'artère pulmonaire, conduit à l'insuffisance ventriculaire droite et, souvent, à la mort du patient. Elle est associée à l'accroissement de la prolifération et à la migration des cellules musculaires lisses des vaisseaux pulmonaires. Schermuly et coll. (2005) ont montré que l'inhibition de l'activité tyrosine kinase des récepteurs aux PDGF améliore considérablement l'évolution de la maladie. Pour cela, ils ont utilisé entre autre un modèle d'hypertension artérielle pulmonaire expérimentale chez le rat obtenu par administration de monocrotaline pendant 28 jours. Tous les rats traités ont survécus, alors que 50% dentre eux sont morts dans le groupe témoin non traité.
FR2917412 décrit des dérivés de 7-alkynyl-1,8-naphtyridones utiles comme inhibiteur de VEGFR-3, pour utilisation médicale.

La présente invention a pour objet des composés répondant à la formule (I) : 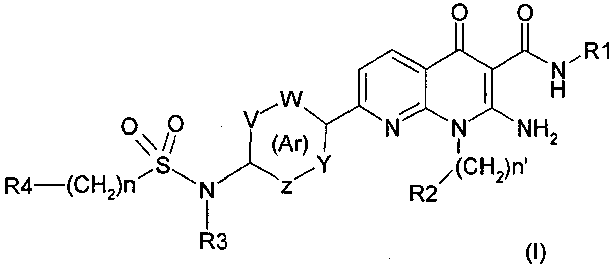 dans laquelle
- **n** représente 0, 1, 2 ou 3 ;
- **n'** représente 0, 1, 2, 3 ou 4 ;
- **R1** représente un groupe alkyle,
- **R2** représente :
   (i) un groupe cycloalkyle,
   (ii) un groupe alkyle ou
   (iii) un groupe alcoxy,
   lesdits groupes cycloalkyle, alkyle ou alcoxy étant éventuellement substitués par un ou plusieurs atomes d'halogène;
- **R3** représente :
   **(i)** un atome d'hydrogène, ou
   (ii) un groupe -C(O)alkyle;
- **Ar** représente un cycle aryle ou hétéroaryle comprenant 5 ou 6 chaînons dans lequel **Y,Z,V et W:**
   (a) représentent, indépendamment les uns des autres,
      (i) un groupe =CH-,
      (ii) un groupe =C(R5)- dans lequel **R5** représente :
         ○ un groupe alkyle,
         ○ un atome d'halogène, ou
         ○ un groupe alcoxy,
      (iii) un hétéroatome choisi parmi l'atome d'azote, l'atome de soufre et l'atome d'oxygène,
   (b) l'un au plus parmi **Y, Z, V et W** étant éventuellement absent,
   étant entendu que, lorsque **Ar** représente un hétéroaryle choisi parmi le pyrrolyle, l'imidazolyle, le pyrazolyle et les triazolyles, au moins l'un des atomes d'azote dudit hétéroaryle peut être éventuellement substitué par un groupe **R6** choisi parmi un groupe alkyle,
- **R4** représente un groupe choisi parmi:
   ○ un groupe alkyle;
   ○ un groupe alcoxyalkyle,
   ○ un groupe -NRR' avec **R et R'**, pouvant être identiques ou différents, et représentant, indépendamment l'un de l'autre, un atome d'hydrogène, un groupe groupe alkyle ou un groupe -(C3-C6)cycloalkyle,
   ○ un groupe cycloalkyle,
   ○ un groupe alcényle,
   ○un groupe aryle, lesdits groupes aryle étant éventuellement substitués par au moins un atome d'halogène, et/ou par au moins un groupe choisi parmi un groupe -(C1-C5)alkyle, halogenoalkyle, nitrile, halogenoalkyloxy, alcoxy, nitro et les groupes -NRR' avec **R et R'**, pouvant être identiques ou différents, et représentant, indépendamment l'un de l'autre, un atome d'hydrogène ou un groupe choisi parmi les groupes alkyle et les groupes -(C3-C6)cycloalkyle,
   ○ un groupe hétéroaryle, lesdits groupes comprenant au moins un hétéroatome choisi parmi l'atome d'azote ou de soufre, lesdits groupes hétéroaryles étant éventuellement substitués par au moins un groupe choisi parmi les groupes alkyle et un groupe hétérocycloalkyle comprenant au moins un hétéroatome choisi parmi l'atome d'azote et d'oxygène;
      étant entendu que, lorsque un groupe hétéroaryle est choisis parmi le pyrrolyle, l'imidazolyle, le pyrazolyle et les triazolyles, au moins l'un des atomes d'azote dudit hétéroaryle peut être substitué par un groupe **R6** choisi parmi un groupe alkyle,
   ○ un groupe hétérocycloalkyle comprenant au moins un hétéroatome choisi parmi l'atome d'azote, de soufre et d'oxygène et étant éventuellement substitué par au moins un substituant choisi parmi (i) les atomes d'halogène, , (ii) les groupes halogénoalkyles, (iii) les groupes alkyles, avantageusement les groupes -(C1-C4)alkyle, linéaires ou ramifiés et (iv) les groupes cycloalkyles,
      étant entendu que, lorsque les groupes hétérocycloalkyles sont choisis parmi le pyrrolinyle, pyrrolidinyle, imidazolidinyle, imidazolinyle, pyrazolinyle, pyrazolidinyle, piperidinyle, morpholinyle, piperazinyle et thiomorpholinyle, au moins l'un des atomes d'azote dudit hétérocycloalkyle peut être éventuellement substitué par un groupe **R6** choisi parmi un groupe alkyle,
sous forme d'acide, de base ou de sel d'addition à un acide ou à une base.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Par exemple, quand **R4** représente un hétérocycle, la configuration absolue d'un carbone substitué sur ledit hétérocycle peut être R ou S.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.
Les composés de formule (I) peuvent également exister sous forme de solvats ou d'hydrates, à savoir sous forme d'associations ou de combinaisons avec une ou plusieurs molécules de solvant ou d'eau, sous forme cristalline ou amorphe. De tels solvats et hydrates font également partie de l'invention.

L'invention a également pour objet un procédé de préparation d'un composé de formule (I) selon l'invention, caractérisé en ce que l'on fait réagir un composé de formule (IXa) : 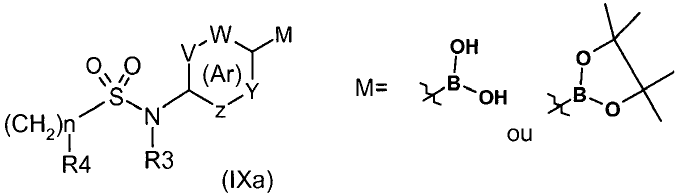 avec un composé de formule (VII), en présence d'un catalyseur de couplage et d'une base tels que définis ci-après, 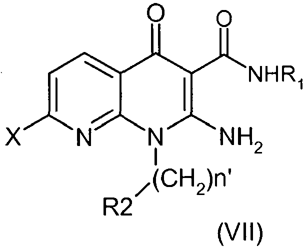 où **R1, R2, R3, R4, n, n', V, W, Y, Z et Ar** sont tels que définis précédemment, **X** représente un groupe partant défini ci-après, avantageusement **X** représente un halogène, encore plus avantageusement **X** représente un atome de chlore et **M** est tel que défini ci-dessus.

Selon un autre aspect, l'invention a aussi pour objet un procédé de préparation d'un composé de formule (I) selon l'invention, caractérisé en ce que l'on fait réagir un composé de formule (IXb) 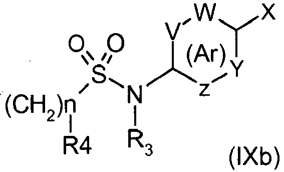 avec un composé de formule (VIII), 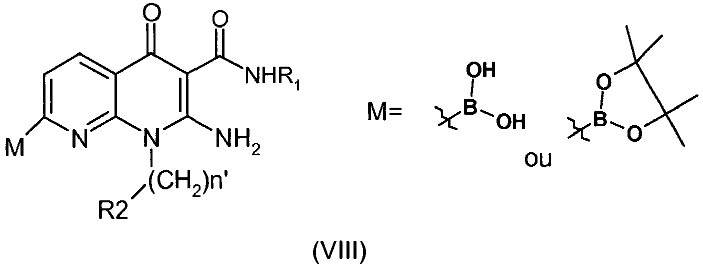 où **R1, R2, R3, R4, n, n', V, W, Y, Z et Ar** sont tels que définis précédemment, **X** représente un groupe partant défini ci-après, avantageusement **X** représente un halogène, encore plus avantageusement **X** représente un atome de brome ou d'iode et **M** est tel que défini ci-dessus.

Dans le cadre de la présente invention, et sauf mention différente dans le texte, on entend par :
- un atome d'halogène : un atome de fluor, de chlore, de brome ou d'iode ;
- un hétéroatome : un atome d'azote, d'oxygène ou de soufre ;
- un groupe alkyle : un groupe aliphatique saturé, linéaire ou ramifié, pouvant comporter 1, 2, 3, 4, 5 ou 6 atomes de carbone (abrégé par -(C1-C6)alkyle). Avantageusement, il s'agit d'un groupe -(C1-C4)alkyle. A titre d'exemples, on peut citer comme (i) groupe -C1alkyle, le groupe méthyle, comme (ii) groupe -C2alkyle, le groupe éthyle, comme (iii) groupe -C3alkyle, le groupe propyle, isopropyle, comme (iv) groupe - C4alkyle, le groupe butyle, isobutyle, tertbutyle, comme (v) groupe -C5alkyle, le groupe pentyle, isopentyle, (vi) comme groupe -C6alkyle, le groupe hexyle;
- un groupe alkylène : un groupe alkyle tel que précédemment défini, divalent saturé, linéaire ou ramifié, pouvant comporter 1, 2, 3, 4, 5 ou 6 atomes de carbone (abrégé -(C1-C6)alkylène-). A titre d'exemple, on peut citer les radicaux méthylène (ou - CH₂-), éthylène (ou -CH₂-CH₂-), propylène (-CH₂-CH₂-CH₂-) ;
- un groupe alcényle : un groupe aliphatique comprenant au moins 2 atomes de carbone et étant mono- ou poly-insaturé. Avantageusement, il s'agit d'un groupe en C2-C10 comprenant au moins une double liaison C=C, encore plus avantageusement un groupe en C2-C6 comprenant au moins une double liaison C=C.
- un groupe cycloalkyle : un groupe alkyle cyclique pouvant comporter 3, 4, 5, 6, 7, 8, 9 ou 10 atomes de carbone, abrégé également -(C3-C10)cycloalkyle. Avantageusement, il s'agit d'un groupe -(C3-C5)cycloalkyle. A titre d'exemples, on peut citer les groupes cyclopropyle, méthylcyclopropyle, cyclobutyle, cyclopentyle, cyclohexyle, cycloheptyle, adamantyle, pentalène ;
- un groupe alcoxy ou alkyloxy : un radical -O-alkyle où le groupe alkyle est tel que précédemment défini. A titre d'exemples, on peut citer les groupes -O-(C1-C5)alkyle ou -(C1-C5)alcoxy, et en particulier comme (i) groupe -O-C1 alkyle, le groupe -Ométhyle, comme (ii) groupe -O-C2alkyle, le groupe -Oéthyle, comme (iii) groupe -O-C3alkyle, le groupe -Opropyle, -Oisopropyle, comme (iv) groupe -O-C4alkyle, le groupe -Obutyle, - Oisobutyle, -Otertbutyle, comme (v) groupe -O-C5alkyle le groupe -Opentyle, - Oisopentyle;
- un groupe alcoxyalkyle : un radical de formule -alkylène-O-alkyle, où les groupes alkyle et alkylène, comprenant le même nombre de carbone ou ne comprenant pas le même nombre de carbone, sont tels que définis précédemment. A titre d'exmples, on peut citer les groupes -(C1-C6)alkylène-O-(C1-C6)alkyle, avec -(C1-C6)alkylène- et - (C1-C6)alkyle tels que définis ci-dessus ;
- un groupe halogénoalkyle : un groupe alkyle tel que défini ci-dessus substitué par 1, 2, 3, 4 ou 5 atomes d'halogène, tels que définis précédemment. On citera par exemple les groupes -halogéno(C1-C5)alkyle, avec (C1-C5)alkyle tel que défini ci-dessus, et en particulier le groupe trifluorométhyle (abrégé -CF₃) ;
- un groupe halogénoalkyloxy : un groupe halogénoalkyle-O- où le groupe halogénoalkyle est tel que défini précédemment ;
- un groupe aryle : un groupe aromatique cyclique comprenant 6, 7, 8, 9 ou 10 atomes de carbone. A titre d'exemples de groupes aryles, on peut citer le groupe phényle(abrégé Ph) ou un groupe naphtyle ;
- un groupe arylalkyle : un groupe aryle, tel que défini ci-dessus, substitué par au moins un groupe alkyle, tel que défini ci-dessus. Avantageusement, il s'agit de radicaux - alkyle-aryle. On peut citer, par exemple, le benzyle c'est-à-dire le radical -CH₂-Ph ;
- un groupe aryloxy : un radical de formule -O-aryle, où le groupe aryle est tel que défini précédemment ;
- un groupe hétéroaryles : un groupe aromatique cyclique comprenant 2, 3, 4 ou 5 atomes de carbone et comprenant 1 à 3 hétéroatomes, qui peu(ven)t être choisi(s) parmi l'atome d'azote, l'atome d'oxygène et l'atome de soufre, indépendemment l'un de l'autre, de manière à être identiques ou différents, lorsqu'ils sont au nombre de 2 ou indépendemment les uns des autres, de manière à être identiques ou différents, lorsqu'ils sont au nombre de 3. On peut citer, par exemple, les groupes pyridinyle, pyrrolyle, pyrazolyle, furanyle, pyrazinyle, pyrimidyle, imidazolyle, thiophényle, thiazolyle, 1, 2, 3 triazolyle et 1, 2, 4 triazolyle ;
- un hétérocycloalkyle : un groupe alkyle cyclique, éventuellement ponté, comprenant 5, 6 ou 7 atomes de carbone et comprenant 1, 2 ou 3 hétéroatomes qui peu(ven)t être choisi(s), indépendemment l'un de l'autre, de manière à être identiques ou différents, lorsqu'ils sont au nombre de 2 ou indépendemment les uns des autres, de manière à être identiques ou différents, lorsqu'ils sont au nombre de 3, parmi l'atome d'azote, l'atome d'oxygène et l'atome de soufre. On peut notamment citer les groupes pipéridinyle, pipérazinyle, pyrrolidinyle, hexaméthylèneimino, tétrahydrofuranyle, morpholinyle, 1,1-dioxydotetrahydrothiényle;
- un groupe protecteur Pg : un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green *et coll*., 2^{nd} Edition (John Wiley & Sons, Inc., New York), 1991 ;
- un groupe partant : un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316 ;
- un catalyseur de couplage : un complexe de métaux tels que le palladium et nickel généralement utilisés en quantité catalytique permettant la formation de liaisons carbone-carbone à partir de dérivés halogénés et de composés organometalliques dérivés de l'étain (via un « couplage de Stille »), du magnésium (via un « couplage de Corriu-Kumada »), du bore (via un « couplage de Suzuki »), du zinc (via un « couplage de Negishi »)... Des exemples de tels catalyseurs de couplage sont décrits dans « Palladium reagents and catalysts - Innovations in organic sysnthesis», J.Tsuji. (John Wiley & Sons, Inc., Chichester), 1995.

Parmi les composés de formule (I) objets de l'invention, on peut citer un groupe de composés dans lequel:
- **n** représente 0, 1, 2 ou 3 ;
   et/ou
- **n'** représente 0, 1, 2, 3 ou 4 ;
   et/ou
- **R1** représente un groupe alkyle, avantageusement un groupe -(C1-C6)alkyle, encore plus avantageusement un groupe -(C1-C4)alkyle ;
   et/ou
- **R2** représente :
   ○ un groupe cycloalkyle, avantageusement un groupe *-(C3-C10)cycloalkyle,* encore plus avantageusement un groupe *-(C3-C5)cycloalkyle,*
   ○ un groupe alkyle, avantageusement un groupe -(C1-C6)alkyle, encore plus avantageusement un groupe -(C1-C4)alkyle, ou
   ○ un groupe alcoxy, avantageusement un groupe -O-(C1-C6)alkyle, encore plus avantageusement un groupe -O-(C1-C4)alkyle,
   lesdits groupes cycloalkyle, alkyle ou alcoxy étant éventuellement substitués par un ou plusieurs atomes d'halogène, avantageusement par un ou plusieurs atomes de fluor ;
   avantageusement **R2** représente un groupe alkyle ;
   et/ou
- **R3** représente :
   ○ un atome d'hydrogène, ou
   ○ un groupe -C(O)alkyle, avantageusement un groupe 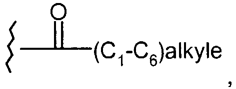 encore plus avantageusement un groupe 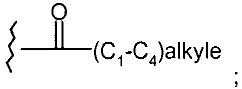
   avantageusement **R3** représente un atome d'hydrogène ;
   et/ou
- **Ar** représente un cycle aryle ou hétéroaryle comprenant 5 ou 6 chaînons et/ou
- **Y, Z, V et W** :
   a) représentent, indépendamment les uns des autres,
      i) un groupe =CH-,
      ii) un groupe =C(R5)- dans lequel R5 représente:
         - un groupe alkyle, avantageusement un groupe -(C1-C6)alkyle, encore plus avantageusement un groupe -(C1-C4)alkyle,
         - un atome d'halogène, avantageusement choisi parmi l'atome de fluor et l'atome de chlore, ou
         - un groupe alcoxy, avantageusement un groupe -O-(C1-C6)alkyle, encore plus avantageusement un groupe -O-(C1-C4)alkyle,
      iii) un hétéroatome choisi parmi l'atome d'azote, l'atome de soufre et l'atome d'oxygène, et/ou
   b) l'un au plus parmi **Y, Z, V et W** est éventuellement absent,
   étant entendu que, lorsque **Y, Z, V et W** sont compris dans un hétéroaryle choisi parmi le pyrrolyle, l'imidazolyle, le pyrazolyle et les triazolyles, au moins l'un des atomes d'azote dudit hétéroaryle peut être éventuellement substitué par un groupe **R6**,
   et/ou
- **R4** représente un groupe choisi parmi:
   ○ les groupes alkyles, avantageusement un groupe -(C1-C6)alkyle, encore plus avantageusement un groupe -(C1-C4)alkyle ;
   ○ les groupes alcoxyalkyle,
   ○ les groupes -NRR',
   ○ les groupes cycloalkyle, avantageusement les groupes -(C3-C5)cycloalkyle,
   ○ les groupes alkenyle, avantageusement les groupes en C2-C10 comprenant au moins une double liaison C=C, encore plus avantageusement les groupes en C2-C6 comprenant au moins une double liaison C=C,
   ○ les groupes aryle, avantageusement les groupes aryle comprenant 6 atomes de carbone, lesdits groupes aryle étant éventuellement substitués par au moins un atome d'halogène, avantageusement choisi parmi les atomes de fluor et de chlore, et/ou par au moins un groupe choisi parmi les groupes -(C1-C5)alkyle, halogenoalkyle, nitrile, halogenoalkyloxy, alcoxy, nitro et les groupes -NRR',
   ○ les groupes hétéroaryles, avantageusement les groupes hétéroaryle comprenant 5 ou 6 chaînons et comprenant au moins un hétéroatome choisi parmi l'atome d'azote ou de soufre, lesdits groupes hétéroaryles étant éventuellement substitués par au moins un groupe choisi parmi les groupes alkyle, avantageusement un groupe -(C1-C4)alkyle et les groupes hétérocycloalkyle comprenant au moins un hétéroatome choisi parmi l'atome d'azote et d'oxygène, avantageusement ledit groupe hétérocycloalkyle est la morpholinyle ;
      (i) étant entendu que, lorsque lesdits groupes hétéroaryles sont choisis parmi le pyrrolyle, l'imidazolyle, le pyrazolyle et les triazolyles, au moins l'un des atomes d'azote dudit hétéroaryle peut être éventuellement substitué par un groupe **R6**,
      (ii) avantageusement lesdits groupes hétéroaryles sont choisis parmi les groupes pyridinyle et imidazolyle,
   ○ les groupes hétérocycloalkyles comprenant au moins un hétéroatome choisi parmi l'atome d'azote, de soufre et d'oxygène et étant éventuellement substitués par au moins un substituant choisi parmi (i) les atomes d'halogène, avantageusement choisis parmi l'atome de fluor et de chlore, (ii) les groupes halogénoalkyles, avantageusement les groupes fluoro(C1-C4)alkyle substitués par 1, 2, 3, 4 ou 5 atomes de fluor, (iii) les groupes alkyles, avantageusement les groupes -(C1-C4)alkyle, linéaires ou ramifiés et (iv) les groupes cycloalkyles, avantageusement les groupes -(C3-C5)cycloalkyle,
      étant entendu que, lorsque les groupes hétérocycloalkyles sont choisis parmi le pyrrolinyle, pyrrolidinyle, imidazolidinyle, imidazolinyle, pyrazolinyle, pyrazolidinyle, piperidinyle, morpholinyle, piperazinyle et thiomorpholinyle, au moins l'un des atomes d'azote dudit hétérocycloalkyle peut être éventuellement substitué par un groupe **R6**,
   et/ou
- **R5** représente :
   ○ un groupe alkyle, avantageusement un groupe -(C1-C6)alkyle, encore plus avantageusement un groupe -(C1-C4)alkyle,
   ○ un atome d'halogène, avantageusment choisi parmi l'atome de fluor et l'atome de chlore, ou
   ○ un groupe alcoxy, avantageusement un groupe -O-(C1-C6)alkyle, encore plus avantageusement un groupe -O-(C1-C4)alkyle,
   et/ou
- **R**6 représente un groupe choisi parmi un groupe alkyle, avantageusement un groupe -(C1-C6)alkyle, encore plus avantageusement un groupe -(C1-C4)alkyle,
   et/ou
- **R et R',** pouvant être identiques ou différents, et représentant, indépendamment l'un de l'autre, un atome d'hydrogène, un groupe groupe alkyle ou un groupe -(C3-C6)cycloalkyle,
sous forme d'acide, de base ou de sel d'addition à un acide ou à une base.

Parmi les composés de formule (I) objets de l'invention, on peut citer un groupe de composés dans lequel:
**R1** représente un groupe-(C1-C4)alkyle,
   et/ou
**R2** représente un groupe un groupe -(C1-C4)alkyle,
   et/ou
**n'** représente 1,
   et/ou
**R3** représente un atome d'hydrogène,
   et/ou
**Ar** représente un phényle,
   et/ou
lesdits composés sont sous forme de base ou de sels d'addition à un acide, avantageusement l'acide chlorhydrique.

Parmi les composés de formule (I) objets de l'invention, on peut citer un groupe de composés dans lequel:
**R4** représente un groupe choisi parmi:
   o les groupes alkyles, avantageusement un groupe -(C1-C6)alkyle, encore plus avantageusement un groupe -(C1-C4)alkyle ;
   o les groupes -NRR', avec **R et R'**, pouvant être identiques ou différents, et représentant, indépendamment l'un de l'autre, un atome d'hydrogène, un groupe alkyle ou un groupe -(C3-C6)cycloalkyle,
   o les groupes alkenyle, avantageusement les groupes en C2-C10 comprenant au moins une double liaison C=C, encore plus avantageusement les groupes en C2-C6 comprenant au moins une double liaison C=C,
   ○ les groupes aryle, avantageusement les groupes aryle comprenant 6 atomes de carbone, lesdits groupes aryle étant éventuellement substitués par au moins un atome d'halogène, avantageusement choisi parmi les atomes de fluor et de chlore, et/ou par au moins un groupe choisi parmi les groupes alcoxy et les groupes -NRR', avec **R et R'** tels que définis ci-dessus,
   ○ les groupes hétéroaryles, avantageusement les groupes hétéroaryle comprenant 5 ou 6 chaînons et comprenant au moins un hétéroatome choisi parmi l'atome d'azote, lesdits groupes hétéroaryles étant éventuellement substitués par au moins un groupe choisi parmi les groupes alkyle, avantageusement un groupe -(C1-C4)alkyle et les groupes hétérocycloalkyle comprenant au moins un hétéroatome choisi parmi l'atome d'azote et d'oxygène, avantageusement ledit groupe hétérocycloalkyle est la morpholinyle ;
   étant entendu que, lorsque lesdits groupes hétéroaryles sont choisis parmi le pyrrolyle, l'imidazolyle, le pyrazolyle et les triazolyles, au moins l'un des atomes d'azote dudit hétéroaryle peut être éventuellement substitué par un groupe **R6**, avec **R6** représentant un groupe choisi parmi un groupe alkyle, avantageusement un groupe - (C1-C6)alkyle, encore plus avantageusement un groupe -(C1-C4)alkyle.

Parmi les composés de formule (I) objets de l'invention, on peut citer un groupe de composés dans lequel **R4** représente un groupe choisi parmi les groupes phényle, pyridinyle et imidazolyle.

Parmi les composés de formule (I) objets de l'invention, on peut citer un groupe de composés dans lequel **Y, Z, V et W** représentent chacun un groupe =CH et/ou un groupe =C(R5)- avec **R5** représentant un atome de chlore ou de fluor, **Y, Z, V et W** étant ainsi compris dans un groupe phényle éventuellement substitué.

Parmi les composés de formule (I) objets de l'invention, on peut notamment citer les composés suivants :
2-amino-1-ethyl-7-(3-fluoro-4-{[(pyridin-3-ylmethyl)sulfonyl]amino}phenyl)-N-methyl-4-oxo-1,4-dihydro-1,8-naphthyridine-3-carboxamide (composé n°1)
2-amino-1-ethyl-7-(3-fluoro-4-{[(3-fluorophenyl)sulfonyl]amino}phenyl)-N-methyl-4-oxo-1,4-dihydro-1,8-naphthyridine-3-carboxamide (composé n°2)
2-amino-7-{4-[(ethenylsulfonyl)amino]-3-fluorophenyl}-1-ethyl-N-methyl-4-oxo-1,4-dihydro-1,8-naphthyridine-3-carboxamide (composé n°3)
2-amino-7-[4-({[2-(dimethylamino)ethyl]sulfonyl}amino)-3-fluorophenyl]-1-ethyl-N-methyl-4-oxo-1,4-dihydro-1,8-naphthyridine-3-carboxamide (composé n°4)
2-amino-7-(4-{[(3-aminobenzyl)sulfonyl]amino}-3-fluorophenyl)-1-ethyl-N-methyl-4-oxo-1,4-dihydro-1,8-naphthyridine-3-carboxamide (composé n°5)
2-amino-1-éthyl-7-(3-fluoro-4-{[(1-méthyl-1H-imidazol-4-yl)sulfonyl]amino}phényl)-N-méthyl-4-oxo-1,4-dihydro-1,8-naphtyridine-3-carboxamide (composé n°6)
2-amino-7-{4-[(butylsulfonyl)amino]-3-fluorophenyl}-1-ethyl-N-methyl-4-oxo-1,4-dihydro-1,8-naphthyridine-3-carboxamide (composé n°7)
2-amino-7-(3-chloro-4-{[(2,3-dichlorophenyl)sulfonyl]amino}phenyl)-1-ethyl-N-methyl-4-oxo-1,4-dihydro-1,8-naphthyridine-3-carboxamide (composé n°8)
2-amino-7-(4-{[(2,5-dichlorophenyl)sulfonyl]amino}-3-fluorophenyl)-1-ethyl-N-methyl-4-oxo-1,4-dihydro-1,8-naphthyridine-3-carboxamide (composé n°9)
2-amino-1-éthyl-N-méthyl-4-oxo-7-{4-[(pyridin-3-ylsulfonyl)amino]phényl}-1,4-dihydro-1,8-naphtyridine-3-carboxamide (composé n°10)
2-amino-7-(4-{[(2,6-dichlorophenyl)sulfonyl]amino}-3-fluorophenyl)-1-ethyl-N-methyl-4-oxo-1,4-dihydro-1,8-naphthyridine-3-carboxamide (composé n°11)
2-amino-7-(2-chloro-4-{[(2,5-dichlorophenyl)sulfonyl]amino}phenyl)-1-ethyl-N-methyl-4-oxo-1,4-dihydro-1,8-naphthyridine-3-carboxamide (composé n°12)
2-amino-7-(2-chloro-4-{[(2,3-dichlorophenyl)sulfonyl]amino}phenyl)-1-ethyl-N-methyl-4-oxo-1,4-dihydro-1,8-naphthyridine-3-carboxamide (composé n° 13)
2-amino-7-(4-{[(2,3-dichlorophenyl)sulfonyl]amino}-2-fluorophenyl)-1-ethyl-N-methyl-4-oxo-1,4-dihydro-1 ,8-naphthyridine-3-carboxamide (composé n°14)
2-amino-7-(4-{[(2,3-dichlorophenyl)sulfonyl]amino}-3-methylphenyl)-1-ethyl-N-methyl-4-oxo-1,4-dihydro-1,8-naphthyridine-3-carboxamide (composé n°15)
2-amino-1-éthyl-N-methyl-7-{4-[(methylsulfonyl)amino]phenyl}-4-oxo-1,4-dihydro-1,8-naphthyridine-3-carboxamide (composé n°16)
2-amino-7-(4-{[(2,3-dichlorophenyl)sulfonyl]amino}phenyl)-1-ethyl-N-methyl-4-oxo-1,4-dihydro-1,8-naphthyridine-3-carboxamide (composé n°17)
2-amino-7-(4-{[(2,3-dichlorophenyl)sulfonyl]amino}-3-fluorophenyl)-1-ethyl-N-methyl-4-oxo-1,4-dihydro-1,8-naphthyridine-3-carboxamide (composé n°18)
2-amino-7-(4-{[(2-chlorophenyl)sulfonyl]amino}phenyl)-1-ethyl-N-methyl-4-oxo-1,4-dihydro-1,8-naphthyridine-3-carboxamide (composé n°19)
2-amino-1-ethyl-7-(3-fluoro-4-{[(2-fluorophenyl)sulfonyl]amino}phenyl)-N-methyl-4-oxo-1,4-dihydro-1,8-naphthyridine-3-carboxamide (composé n°20)
2-amino-7-(4-{[(4-chlorophenyl)sulfonyl]amino}-3-fluorophenyl)-1-ethyl-N-methyl-4-oxo-1,4-dihydro-1,8-naphthyridine-3-carboxamide (composé n°21)
2-amino-7-(4-{[(3-chlorophenyl)sulfonyl]amino}-3-fluorophenyl)-1-ethyl-N-methyl-4-oxo-1,4-dihydro-1,8-naphthyridine-3-carboxamide (composé n°22)
2-amino-7-(4-{[(3,4-difluorophenyl)sulfonyl]amino}-3-fluorophenyl)-1-ethyl-N-methyl-4-oxo-1,4-dihydro-1,8-naphthyridine-3-carboxamide (composé n°23)
2-amino-1-ethyl-7-(3-fluoro-4-{[(4-fluorophenyl)sulfonyl]amino}phenyl)-N-methyl-4-oxo-1,4-dihydro-1,8-naphthyridine-3-carboxamide (composé n°24)
2-amino-1-ethyl-7-(3-fluoro-4-{[(3-methoxyphenyl)sulfonyl]amino}phenyl)-N-methyl-4-oxo-1,4-dihydro-1,8-naphthyridine-3-carboxamide (composé n°25)
2-amino-1-ethyl-7-[3-fluoro-4-({[6-(morpholin-4-yl)pyridin-3-yl]sulfonyl}amino)phenyl]-N-methyl-4-oxo-1,4-dihydro-1,8-naphthyridine-3-carboxamide (composé n°26)
2-amino-1-ethyl-7-(3-fluoro-4-{[(pyridin-2-ylmethyl)sulfonyl]amino}phenyl)-N-methyl-4-oxo-1,4-dihydro-1,8-naphthyridine-3-carboxamide (composé n°27).

Il est à noter que les composés ci-dessus ont été nommés en nomenclature IUPAC par l'intermédiaire du logiciel ACDLABS 10.0 ACD/name (Advanced Chemistry development).

Conformément à l'invention, on peut préparer les composés de formule générale (I) selon le procédé qui suit.

Selon le **schéma 1**, un acide 2,6-dihalogénonicotinique de formule (II), où X et X' représentent indépendamment l'un de l'autre un atome d'halogène choisi avantageusement parmi l'atome de F, de Cl et de Br, avantageusement X et X' représentent un atome de chlore, est mono-substitué en position 2 par une amine de formule R2-(CH2)n'-NH₂ dans laquelle R2 et n' sont tels que définis précédemment en rapport avec les composés de formule (I), objets de l'invention. Cette réaction peut avoir lieu à température ambiante, ou à une température de 50°C à 100°C, en chauffage classique ou par micro-onde et dans un solvant protique tel qu'un alcool par exemple éthanol, n-butanol, tert-butanol ou l'eau. L'acide (III), issu de l'étape (i), est ensuite activé en dérivé de formule (IV).

Ce dérivé (IV) peut être soit sous forme de fluorure d'acide avec A = F par l'action du fluorure de cyanuryle à température ambiante, en présence d'une base telle que la triéthylamine ou la pyridine et dans un solvant tel que le dichlorométhane ou le THF, comme décrit par G. OLAH et coll. dans Synthesis (1973), 487, ou soit sous forme d'imidazolide avec A = imidazolyle par l'action du carbodiimidazole dans un solvant tel que le DMF ou le THF ou par d'autres méthodes connues de l'Homme de l'art, telles que celles décrites par MUKAIYAMA et TANAKA dans Chem. Lett. (1976), 303 ou par ISHIKAWA et SASAKI dans Chem. Lett. (1976), 1407.

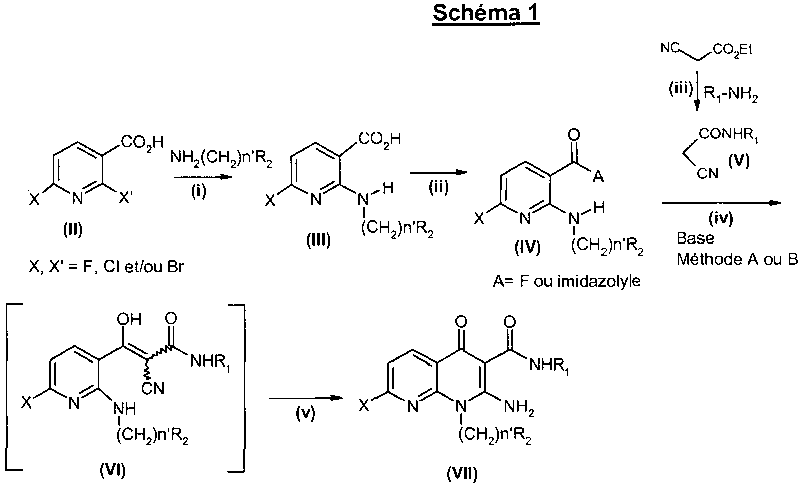

Le fluorure d'acide (composé de formule (IV) avec **A** = F, **X** = halogène, avantageusement X = Cl et avec n' et R2 tels que définis ci-dessus) ou l'imidazolide (composé de formule (IV) avec A = imidazolyle, X = halogène, avantageusement X = Cl et avec n' et R2 tels que définis ci-dessus) de formule (IV) obtenus à l'issue de l'étape (ii) sont très réactifs mais stables. Ils peuvent être mis ensuite en réaction avec un cyanoacétamide N-substitué de formule (V) selon les methodes A ou B décrites ci-après.

Suivant la méthode A, deux équivalents d'une base telle que l'hydrure de sodium ou le tert-butoxyde de potassium, sont utilisés pour l'étape (iv) de condensation du dérivé cyanoacétamide N-substitué (V), avec un composé de formule (IV), après une nuit à température ambiante, on obtient un β-céto-cyanoacétamide de formule (VI), qui est ensuite cyclisé en pyridino-pyridinone de formule (VII) dans lequel X = halogène, avantageusement X = Cl et R1, R2, n' sont tels que définis ci-dessus par chauffage à une température comprise entre 90 et 125°C dans un solvant polaire tel que le n-butanol, le DMSO ou le DMF.

La methode B est similaire à la méthode A pour l'étape de condensation (iv) mais au mélange réactionnel, un troisième équivalent de la base utilisée est additionné et le composé de formule (VI) formé, se cyclise in situ, à température ambiante, pour fournir directement le composé pyridino-pyridinone de formule (VII) dans lequel X = halogène, avantageusement X = Cl et R1, R2, n' sont tels que définis ci-dessus.

Les N-alkylcyanoacétamides de formule (V) sont préparés selon l'étape (iii) en faisant réagir le cyanoacétate d'ethyle avec un excès d'amine de formule R₁-NH₂ (où R₁ est tel que défini précédemment en rapport avec les composés de formule (I) objets de l'invention) dans un solvant tel que le THF ou l'éthanol, à température allant de la température ambiante à la température de reflux du solvant.

Pour l'obtention des composés de formule (I) objet de la présente invention deux méthodes sont utilisables à partir de l'intermédiaire halogéné de formule (VII) décrit précédemment.

Suivant la voie 1 représentée dans le **schéma 2**, l'intermédiaire (VII) dans lequel X représente un groupe partant avantageusement un atome d'halogène, avantageusement un atome choisi parmi F, Cl et Br, encore plus avantageusement un atome de Cl et dans lequel n', R1 et R2 sont tels que définis ci-dessus conformément à l'invention, est engagé à l'étape (vi) dans une réaction de couplage de SUZUKI avec un acide boronique ou un ester boronique de bispinacol (IXa) dans lequel n, R3, R4, V, W, Y et Z sont tels que définis précédemment en rapport avec les composés de formule (I) objets de l'invention, M étant tel que défini dans le schéma 2 et étant entendu que le cycle (Ar), défini ci-dessus conformément à l'invention, doit comprendre entre 5 et 6 chaînons. Cette réaction (vi) est faite en présence d'un catalyseur tel qu'un complexe de palladium (à l'état d'oxydation (0) ou (II)) tel que par exemple Pd(PPh₃)₄, PdCl₂(PPh₃)₂, Pd₂dba₃, Xphos ou PdCl₂(dppf), dans un solvant polaire, protique ou non tel que le DME, l'éthanol, le DMF, le dioxanne, ou des mélanges de ces solvants, en présence d'une base telle que le carbonate de césium, l'hydrogénocarbonate de sodium aqueux, ou K₃PO₄, en chauffant classiquement entre 80 et 120 °C ou bien sous l'action des micro-ondes entre 130 et 170°C.

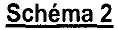

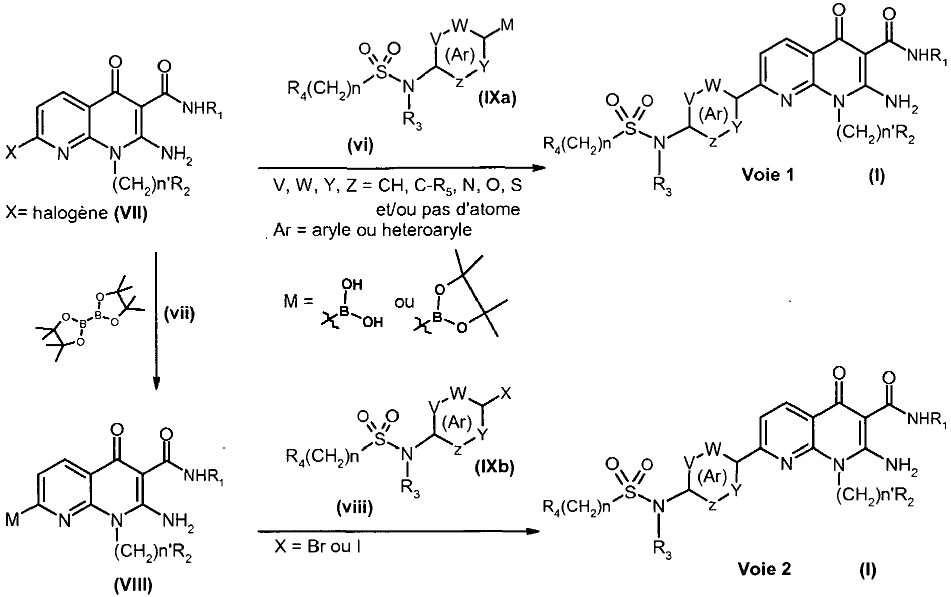

Pour l'obtention des composés de formule (I) objet de la présente invention, une seconde voie est utilisable à partir de l'intermédiaire halogéné de formule (VII), cette voie 2 est décrite dans le schéma 2. L'intermédiaire halogéné de formule (VII), tel que défini précédemment, peut être transformé en acide boronique de formule (VIII), dans lequel M étant tel que défini dans le schéma 2 et R1, R2, n'étant tels que définis ci-dessus conformément à l'invention, suivant l'étape (vii), par réaction avec le bis(pinacolato)diborane en présence de [1,1'-bis(diphénylphosphino)ferrocène] dichloropalladium (II) et d'acétate de potassium ou de carbonate de potassium dans un solvant polaire tel que le DMSO, le DMF, le DME ou le dioxanne, à une température comprise entre 50 et 100°C, selon la méthodologie décrite par ISHIYAMA, T. et coll. dans J. Org. Chem., 1995, 60, 7508-7510 et GIROUX, A. et coll. dans Tet. Lett., 1997, 38, 3841-3844. Dans l'étape (viii) suivante, ledit composé acide boronique (VIII) est engagé dans une réaction de type Suzuki, avec un composé aromatique halogéné de formule (IXb) dans lequel X représente un groupe partant, avantageusement un atome d'halogène, avantageusement X est choisi parmi les atomes de brome et d'iode et où R₃, R₄ , V, W, Y et Z sont tels que définis précédemment en rapport avec les composés de formule (I) objets de l'invention, étant entendu que le cycle (Ar), aryle ou hétéroaryle, doit comprendre entre 5 et 6 chaînons.

Quand les modes de préparation, des composés de départ, des réactifs, tels que les composés de formule (IX), utilisés dans les schémas 1 et 2 ne sont pas décrits, ils sont soit disponibles commercialement ou bien peuvent être préparés selon des méthodes qui sont décrites dans la littérature ou connues de l'Homme du métier.

Si nécessaire, certaines fonctions réactives situées dans les groupes, tels que par exemple dans les groupes R₁, R₂, R₃ et R₄ en particulier dans les groupes R₅ et/ou R₆, conformes à l'invention, peuvent être protégées au cours de ces réactions par des groupes protecteurs, tels que décrits dans « Protective Groups in Organic Synthesis », Green et coll., 2nd Edition (John Wiley & Sons, Inc., New York).

Les exemples suivants illustrent la préparation de certains composés conformes à l'invention. Ces exemples ne sont pas limitatifs et ne font qu'illuster la présente invention. Les numéros des composés exemplifiés renvoient à ceux donnés dans le tableau ci-après, qui illustre les structures chimiques et les propriétes physiques de quelques composés selon l'invention.

Les abréviations et formules brutes suivantes sont utilisées :
- AcOEt: Acétate d'éthyle
- DCM: Dichlorométhane
- °C: degré Celsius
- DME: Diméthoxyéthane
- DMF: Diméthylformamide
- DMSO: Diméthyl sulfoxide
- h: Heure(s)
- HCl: Acide chlorhydrique
- NaHCO₃: Hydrogénocarbonate de sodium
- Na₂SO₄: Sulfate de sodium
- NaCl: Chlorure de sodium
- NaOH: Hydroxyde de sodium
- Na₂SO₄: Sulfate de sodium
- min.: minutes
- ml: millilitre
- P₂O₅: di-phosphore pentaoxide
- THF: Tétrahydrofurane

Les Condition d'analyse des exemples décrits ci-dessous ainsi que du tableau ci-dessous sont les suivantes:
➢ **Conditions de couplage LC/UV/MS :**
   - Conditions A :
      Instrument (Micromass): chaîne HPLC : Gilson, Spectromètre de masse ZMD (Micromass).
      LC/UV
      Colonne : XTerra C18 3.5 µm (4.6 x 50 mm) (Waters), Temp. colonne :25°C,
      Détection UV : 220 nm.
      Gradient : 15 minutes
      Eluants : A: H₂O + 0,1 % HCOOH / B : CH₃CN + 0,1 % HCOOH, Débit: 1 ml/min.
      Gradient : 0 à 15 min de 5 à 95% B.
   - Conditions B :
      Instrument (Micromass) : chaîne HPLC : Waters, Spectromètre de masse platform II (Micromass).
      LC/UV
      Colonne : XTerra MSC18 3.5 µm (4.6 x 150 mm) (Waters), Temp. colonne : 20°C,
      Détection UV : 220 nm.
      Gradient : 11 minutes
      Eluants : A: CH3COONH4 5mM + 3% CH3CN / B : CH₃CN, Débit: 0.5 ml/min.
      Gradient : 0 à 8 min de 10 à 90% B ; 8 à 11min 90%B.
➢ **MS**
   Mode d'ionisation : Electrospray mode positif ESI+, Gamme de masse : 90-1500 uma ou APCI+
➢ **RMN**
   Les spectres RMN ¹H ont été obtenu en utilisant des Spectromètres RMN Bruker 200 ou 400 MHz dans du CDCl3 ou du DMSO-d6, en utilisant le pic du CHCl3 ou du DMSO-d5 comme référence. Les déplacements chimiques δ sont exprimés en partie par million (ppm). Les signaux observés sont exprimés ainsi : s = singulet ; d = doublet ; t = triplet ; m = massif ou large singulet ; H = proton.
➢ **Point de FUSION**
   Les points de fusions inférieurs à 260°C ont été mesurés avec un appareil banc Koffler et les points de fusions supérieurs à 260°C ont été mesurés avec un appareil Buchi B-545.

### Exemple 1 : 2-amino-7-(4-{[(2,3-dichlorophenyl)sulfonyl]amino}phenyl)-1-ethyl-N-methyl-4-oxo-1,4-dihydro-1,8-naphthyridine-3-carboxamide

### 1.1 : 6-chloro-2-(ethylamino)pyridine-3-carboxylique acide :

Une solution de 18,0 g (84,4 mmol) d'acide 2,6-dichloronicotinique dans 180 ml (3.45 mol) d'une solution d'éthylamine à 70 % dans l'eau est chauffée à 50°C pendant 10 heures. L'excès d'amine est alors évaporé sous pression réduite, puis une solution aqueuse d'acide acétique à 10 % est additionnée jusqu'à la précipitation du produit. Le solide beige est essoré, rincé avec de l'eau froide et séché à l'étuve. On obtient 10,5 g du produit attendu. Rendement = 62 %. Point de fusion : 158-160°C. MH⁺: 201,1 (tr: 7.7 min, condition A).

### 1.2 : 6-chloro-2-(ethylamino)pyridine-3-carbonyl fluoride

A une suspension de 10,5 g (52,3 mmol) du composé obtenu à l'issue de l'étape 1.1 dans le dichlorométhane (250 ml), 4,2 ml (52,3 mmol) de pyridine et 8,4 ml (99,6 mmol) de fluorure cyanurique sont ajoutés successivement. Le mélange est agité pendant 3 heures à température ambiante puis filtré. Le solide est rincé au dichlorométhane (100 ml) et le filtrat est lavé deux fois à l'eau glacée (60 ml). La phase organique est séchée sur Na₂SO₄ puis concentrée sous pression réduite. 10,44 g de produit sont obtenus, sous forme d'huile orange. Rendement = 99 %. Le produit est utilisé sans purification dans l'étape suivante.

### 1.3: 2-cyano-N-methylacetamide

A 10,9 g (353,6 mmol) d'une solution de méthylamine dans le THF refroidie à 0°C, 20 g (176,8 mmol) d'éthyle cyanoacétate sont ajoutés goutte à goutte puis le mélange réactionnel est agité à température ambiante pendant une nuit. Les solvants sont évaporés sous pression réduite et le produit est purifié par recristallisation dans le toluène. 16,8 g de produit sont obtenus, sous forme d'un solide beige. Rendement = 96 %. Point de fusion = 99°C.

### ➢ Selon la Méthode A du schéma 1 ci-dessus (étapes 1.4 et 1.5 ci-après).

### 1.4 : 3-[6-chloro-2-(ethylamino)pyridin-3-yl1-2-cyano-3-hydroxy-N-methylprop-2-enamide

A une solution, refroidie à 0-5°C, de 9,80 g (100 mmol), du composé obtenu à l'issue de l'étape 1.3, dans 100 ml de DMF anhydre, 3,98 g (100 mmol) d'hydrure de sodium à 60 % dans de l'huile minérale sont ajoutés par petites quantités. Après la fin du dégagement d'hydrogène, le mélange est agité pendant 10 minutes à température ambiante, puis refroidit à nouveau à 0-5°C. Une solution de 10,1 g (49,8 mmol) du composé obtenu à l'issue de l'étape 1.2, dans 60 ml de DMF, est additionnée et le mélange est agité à température ambiante pendant une nuit puis 2,85 ml (49,8 mmol) d'acide acétique sont ajoutés. Le DMF est évaporé sous pression réduite puis le résidu est repris dans de l'eau et le produit est extrait deux fois avec un mélange dichlorométhane : méthanol dans des proportions de 95 pour 5, puis une fois avec un mélange acétate d'éthyle : THF (2:1). Les phases organiques combinées sont séchées sur MgSO₄, puis les solvants sont évaporés sous pression réduite. On obtient 19,0 g de produit utilisé tel quel dans l'étape suivante.

### 1.5 : 2-amino-7-chloro-1-ethyl-N-methyl-4-oxo-1.4-dihydro-1.8-naphthyridine-3-carboxamide

Une solution de 19,0 g (49,8 mmol) du produit brut obtenu à l'issue de l'étape 1.4 dans 600 ml de n-butanol est chauffée pendant 48 heures à 110°C. Le solvant est évaporé sous pression réduite et le solide obtenu est trituré dans du méthanol. Le solide est ensuite filtré et séché à l'étuve. On obtient 7,9 g du produit attendu sous forme de solide jaune pâle. Rendement = 57 %. Point de fusion : 283-286°C. MH⁺: 281.2 (tr= 6,99 min, condition A)

### ➢ Selon la Méthode B du shéma 1 ci-dessus (étapes 1.6 ci-après au lieu des étapes 1.4 et 1.5 précédentes).

### 1.6 : 2-amino-7-chloro-1-éthyl-N-méthyl-4-oxo-1.4-dihydro-1_{.}8-naphthyridine-3-carboxamide

A une solution refroidie à 0-5 °C de 0,48 g (4,9 mmol) du composé obtenu à l'issue de l'étape 1.3, dans du DMF anhydre (7 ml), 0,4 g (9,95 mmol) d'hydrure de sodium à 60 % dans l'huile minérale sont ajoutés par petites portions. Le mélange est agité à cette température pendant dix minutes puis une solution de 1,0 g (4,93 mmol) du composé obtenu à l'issue de l'étape 1.2 dans du DMF anhydre (5 ml) est ajoutée. Le mélange réactionnel est agité pendant 1 nuit à température ambiante puis 0,2g (4,9 mmol) d'hydrure de sodium à 60 % sont rajoutés par petites portions. L'agitation est poursuivie à cette température pendant 30 minutes puis 0,56 ml (9,8 mmol) d'acide acétique sont additionnés, suivi de 60 ml d'eau et le solide est filtré, rincé à l'eau puis sèché à l'étuve. On obtient 1,30 g du produit attendu. Rendement = 94 %. Point de fusion : 283-284°C. MH⁺: 281,2 (tr= 6,99 min, condition A)

### 1.7: [7-amino-8-ethyl-6-(methylcarbamoyl)-5-oxo-5,8-dihydro-1.8-naphthyridin-2-yl]boronique acide

Une suspension de 8 g (0,03 mol) du composé obtenu à l'issue de l'étape 1.5 ou 1.6 (selon que la méthode A ou B a été employée), de 8,0 g (0,03 mol) de bis(pinacolato) diborane et de 8,5 g (0,08 mol) d'acétate de potassium dans le DMSO (130 ml), est (130 ml), est dégazée à l'argon pendant 15 minutes. 1,4 g (1,7 mmol) de [1,1'-bis(diphenylphosphino)ferrocène]dichloropalladium(II), complexé avec du dichlorométhane (1 :1) est additionné et le mélange est chauffé à 80°C pendant 30 minutes, sous argon, puis refroidi et dilué avec 1,1 l d'eau et acidifié à pH = 4 par ajout d'acide acétique (50 ml). Le mélange est filtré et le précipité noir est lavé à l'eau (40 ml) puis à l'éther (60 ml). Le résidu noir est repris dans 575 ml d'une solution NaOH (1N) et le mélange est filtré sur célite 545. Le filtrat est acidifié avec 60 ml d'acide acétique et le précipité est filtré, lavé à l'eau et à l'éther puis séché à l'étuve. 6,85 g de produit sont obtenu sous forme d'une poudre blanche. Rendement = 83%. Point de fusion : 335°C. MH⁺: 291.2 (tr= 5.3 min, condition A)
RMN ¹H (250MHZ, DMSO-d₆), δ (ppm) : 11,69 (s, 1H); 11,12 (q, 1H, 4,67 Hz); 8,47 (s, 2H); 8,44 (d, 1H, 7,7Hz); 7,9 (s, 1H); 7,75 (d, 1 H, 7,7Hz); 4,72 (m, 2H); 2,8 (d, 3H, 4,67 Hz); 1,22 (t, 3H, 6,9 Hz).

### 1.8:2-amino-7-(4-{[(2,3-dichlorophenyl)sulfonyl]amino}phenyl)-1-ethyl-N-methyl -4-oxo-1,4-dihydro-1,8-naphthyridine-3-carboxamide

### 1.8.1 : 2,3-dichloro-N-[4-(4,4,5,5-tetramethyl-1,3,2-dioxaborolan-2-yl)phenyl] benzenesulfonamide

A une solution de 1,1 g (5 mmol) de 4-(4,4,5,5-tetramethyl-1,3,2-dioxaborolan-2-yl)aniline dans 10 ml de de pyridine, on ajoute par portions 1,23 g (5 mmol) de chlorure de 2,3-dichlorobenzenesulfonyle et laisse agiter 15 h à température ambiante. Le solvant est évaporé et le résidu est repris avec 20 ml d'acétate d'éthyle, lavé avec HCl 1 N puis à l'eau et avec une solution aqueuse saturée de chlorure de sodium. La phase organique est séchée sur Na₂SO₄ et évaporée à sec. On obtient 2,1 g du composé sous la forme de cristaux rouge foncé. Rendement = 100 %. Point de fusion : 235°C
RMN¹H (400MHz; CDCl₃): δ (ppm) : 1,2 (s;12H; 7,0 (br s;1H); 7,05 (d;2H;8Hz); 7,2 (t;1H; 8Hz); 7,55 (d;1H;8Hz); 7,6 (d;2M;8Hz); 7,9 (d;1h;8Hz).

### 1.8.2: 2-amino-7-(4-{[(2,3-dichlorophenyl)sulfonyl]amino}phenyl)-1-ethyl-N-methyl-4-oxo-1,4-dihydro-1,8-naphthyridine-3-carboxamide

280 mg (1mmol) de chloronaphtyridine obtenue en 1.6 et 450 mg (1,05 mmol) du boronate obtenu dans l'étape précédente sont dissous dans 12 ml de DME et 3 ml d'éthanol. On ajoute 8 ml d'une solution aqueuse saturée de NaHCO₃ puis on fait barboter de l'argon pendant 10 minutes. On ajoute alors 85 mg (0,073 mmol) de tetrakis(triphenylphosphine)palladium(0) et chauffe le milieu réactionnel à 90°C sous atmosphère d'argon. Après 3h le milieu est filtré à chaud, le précipité obtenu après refroidissement est filtré, lavé à l'eau puis à l'éthanol et enfin à l'éther éthylique. Le solide obtenu est recristallisé dans l'éthanol et séché à l'étuve. On obtient 205 mg de produit sous la forme d'une poudre jaune pâle. Rendement = 37%. Point de fusion = 210°C
RMN(200MHz;DMSOd6): δ(ppm) :1 ,2 (t;3H;7Hz); 2,8 (d;3H;4,5Hz); 4,55 (q;2H;7Hz); 7,2 (d;2H.8Hz); 7,55 (t;1H;8Hz); 7,7-7,9 (m;4H); 8,1-8,2 (m;3H);8,4 (d;1H;8Hz); 11,1 (q;1H;4,5Hz); 11,65 (br s;1H)
LCMS : MH⁺ : 546 (tr : 6.58 min, condition B).

### Exemple 2: 2-amino-7-(4-{[(2,3-dichlorophenyl)sulfonyl]amino}-3-fluorophenyl)-1-ethyl-N-methyl-4-oxo-1,4-dihydro-1,8-naphthyridine-3-carboxamide

### ➢ Selon la Méthode A du schéma 1 ci-dessus:

### 2.1: 2-fluoro-4-(4,4,5,5-tetramethyl-1,3,2-dioxaborolan-2-yl)aniline

5,0 g (21,1 mmol) de 2-fluoro-4-iodoaniline et 5,89 g (23,2 mmol) de bis(pinacolato) diborane sont dissous dans 130 ml de DMSO. On ajoute 6,21 g (63,3 mmol) d'acétate de potassium et fait barboter de l'argon pendant 10 min. 1,21 g (1.50 mmol) de [1,1'-bis(diphenylphosphino)ferrocène]dichloropalladium(II), complexé avec du dichlorométhane (1 :1) est additionné et le mélange est chauffé à 85°C pendant 4,5 heures , sous argon, puis refroidi et dilué avec 500 ml d'eau. Le mélange est extrait avec l'acétate d'éthyle (3X200 ml), les phases organiques sont lavées à l'eau, séchées sur Na₂SO₄ puis évaporées à sec. Le produit brut est purifié par chromatographie sur silice (éluant : cyclohexane/acétate d'éthyle 90/10). On obtient 3,73 g de produit sous la forme de poudre blanche. Rendement = 75%. Point de fusion : 112°C.
RMN¹H(400MHz;CDCl₃): δ(ppm) : 1,2 (s ;12H) ; 3,8 (br s ;2H) ; 6,55 (t ;1 H ;7Hz) ; 7,25-7,35 (m ;2H).

### 2.2: 2.3-dichloro-N-[2-fluoro-4-(4,4,5,5-tetramethyl-1,3_{.}2-dioxaborolan-2-yl) phenyl]benzenesulfonamide

Sous atmosphère inerte, à une solution de 1,0 g (4,22 mmol) du composé obtenu à l'issue de l'étape 2.1, dans 40 ml de pyridine anhydre, 1,24 g de chlorure de 2,3-dichlorobenzenesulfonyle sont ajoutés par portions, puis le mélange réactionnel est laissé sous agitation pendant 18 heures. Le solvant est évaporé et le résidu est repris avec 20 ml d'acétate d'éthyle, lavé avec HCl 1N puis à l'eau et avec une solution aqueuse saturée de chlorure de sodium. La phase organique est séchée sur Na₂SO₄ et évaporée à sec et le résidu est recristallisé dans le cyclohexane. On obtient 1.13 g de produit sous forme de cristaux blancs. Rendement : 60%. MH⁺: 445 (tr: 8,43 min, condition A).

### 2.3 : 2-amino-7-(4-{[(2,3-dichloroehenyl)sulfonyllamino}-3-fluorophenyl)-1-ethyl -N-methyl-4-oxo-1.4-dihydro-1,8-naphthyridine-3-carboxamide

1,13 g (2,53 mmol) du composé obtenu à l'issue de l'étape 2.2 et 0,65 g (2,32 mmol) de chloronaphtyridine obtenue en 1.6 sont dissous dans 18 ml de dimethoxyethane et 7 ml d'éthanol. On ajoute 16 ml d'une solution aqueuse saturée de NaHCO₃ et on fait barboter de l'argon pendant 10 minutes. 0,134 g (0,12 mM) de tetrakis(triphenylphosphine)palladium(0) sont ajoutés et le milieu réactionnel est chauffé à 100°C sous argon pendant 4h puis filtré et le résidu obtenu après évaporation du filtrat est trituré dans l'eau. Le précipité est filtré, lavé à l'eau et séché. Puis purifié par chromatographie sur silice en éluant avec un gradient de méthanol dans le dichlorométhane. On obtient 740 mg de produit sous forme de poudre blanche. Rendement : 57%. Point de fusion : 333°C
RMN(200MHz ;DMSOd6) : δ(ppm) :1,2 (t ;3H ;7Hz) ; 2,7 (s ;3H) ;4,5 (q ;2H ;7Hz) ; 7,35 (t ;1H ;8Hz) ; 7,5 (t ;1H ;8Hz) ; 7,8-8,0 (m ;6H) ; 8,45 (d ;1H ;8Hz) ; 10,8 (s ;1H) ; 11,1 (q ;1H ;4,5Hz) ; 11,7 (br s ; 1H).
LCMS : MH⁺: 563,9 (tr: 7,544 min, condition A)

### ➢ Selon la Méthode B du schéma 1 ci-dessus:

### 2.4 : 2,3-dichloro-N-(2-fluoro-4-iodophenyl)benzenesulfonamide

A une solution de 2-fluoro-5-iodo aniline (2,8 g; 11,8 mmol) dans 30 ml de pyridine anhydre, on ajoute par portions 2,96 g (11,8 mmol) de chlorure de 2,3-dichlorobenzenesulfonyle et on laisse agiter 24 heures à 20°C. La pyridine est évaporée, le résidu est repris avec 50 ml d'acétate d'éthyle et lavé à l'eau puis avec une solution aqueuse saturée de NaCl. La phase organique est séchée sur Na₂SO₄ puis évaporée à sec. Le produit est recristallisé dans le cyclohexane. On obtient 4,51 g de produit sous forme de poudre blanche. Rendement: 86%; LCMS:(M-H)⁻: 444 (tr: 7,90 min, condition A).

### 2.5 : 2-amino-7-(4-{[(2,3-dichlorophenyl)sulfonyl]amino}-3-fluorophenyl)-1-ethyl -N-methyl-4-oxo-1,4-dihydro-1,8-naphthyridine-3-carboxamide

A une solution de 1,0 g (2,24 mmol) du composé obtenu à l'issue de l'étape 2.4 et de 1,0 g (3,45 mmol) de l'acide boronique obtenu à l'issue de l'étape 1.7 dans 16 ml de DMF, 4,5 ml d'une solution aqueuse saturée de NaHCO₃ sont ajoutés et on fait barboter de l'argon pendant 10 min. On ajoute alors 144 mg (0,16 mmol) de tris(dibenzylidèneacétone)dipalladium(0) et le mélange est chauffé à 85°C pendant 5 heures. Le milieu réactionnel est filtré à chaud puis le filtrat évaporé à sec et le résidu trituré dans l'eau (20 ml). Le précipité est filtré lavé à l'eau puis séché à l'étuve sous vide. Le produit brut est purifié par chromatographie sur silice. On obtient 504 mg de produit sous forme de poudre blanche. Rendement : 40%. RMN et LCMS identiques à celles du produit obtenu par la méthode A.

### Exemple 3: 2-amino-7-(4-{[(2,5-dichlorophenyl)sulfonyl]amino}-3-fluorophenyl)-1-ethyl-N-methyl-4-oxo-1,4-dihydro-1,8-naphthyridine-3-carboxamide :

### 3.1 : 2,5-dichloro-N-(2-fluoro-4-iodophenyl)benzenesulfonamide

Préparé selon la méthode décrite en 2.4 (méthode B) à partir de 2,0 g (8,44 mmol) de 2-fluoro-4-iodoaniline et de 2,11 g (8,44 mmol) de chlorure de 2,5-dichlorobenzenesulfonyle dans 21 ml de pyridine.
On obtient 3,20 g de produit sous forme de poudre blanche. Rendement : 85%. LCMS :(M-H)⁻: 444 (tr: 7,88 min, condition A).

### 3.2 : 2-amino-7-(4-{[(2,5-dichloroohenyl)sulfonyllamino}-3-fluorophenyl)-1-ethyl -N-methyl-4-oxo-1,4-dihydro-1,8-naphthyridine-3-carboxamide

Préparé selon la méthode décrite en 2.5 (méthode B) à partir de 1,24 g (2,78 mmol) du produit issu de l'étape 3.1 et de 1,24 g (4,28 mmol) de l'acide boronique issu de l'étape 1.7.
On obtient 610 mg de produit sous forme de poudre blanche. Rendement : 39%. Point de fusion: 230°C
RMN¹H (200MHZ,DMSO-d₆): δ(ppm) :1,3 (t ;3H ;7Hz) ;2,8 (d ;3H ;4,5Hz) ; 4,55 (q ;2H ;7Hz) ; 7,40 (t ;1H ;8H); 7,75 (s ;2H) ; 7,85-8,2 (m ;5H) ; 8,50 (d ;1H ;8H); 10,85 (s ;1 H) ; 11,05 (q ;1 H ;4,5Hz) ; 11,7 (brs; 1 H).
LCMS : MH⁺ : 564 (tr: 7,32 min, condition A)

### Exemple 4: 2-amino-1-ethyl-N-methyl-7-{4-[(methylsulfonyl)amino]phenyl}-4-oxo-1,4-dihydro-1,8-naphthyridine-3-carboxamide

### 4.1 : {4-[(methylsulfonyl)aminolohenyl}boronique acide

On refroidit dans la glace une suspension de 1,0 g (5,77 mmol) de chlorhydrate de l'acide 4-aminobenzeneboronique dans 10 ml d'une solution aqueuse saturée de NaHCO₃ et on ajoute 2 ml (25,8 mmol) de chlorure de méthanesulfonyle et ajuste le pH à 7,2 en ajoutant environ 10 ml d'une solution aqueuse saturée de NaHCO₃ et laisse agiter 2h à 5°C. On rajoute 1 ml de chlorure de méthanesulfonyle et 5 ml d'une solution aqueuse saturée de NaHCO₃. On laisse la température du milieu remonter à 20°C et ajoute HCl 3N jusqu'à pH=2 puis évapore le tout à sec. On ajoute 20 ml d'eau. Le précipité est filtré, lavé avec un minimum d'eau puis à l'éther éthylique. Le produit est séché à l'étuve sous vide à 40°C. On obtient 0,45 g de poudre blanche utilisée sans purification supplémentaire.

### 4.2: 2-amino-1-ethyl-N-methyl-7-{4-[(methylsulfonyl)aminolphenyl}-4-oxo-1,4-dihydro-1,8-naphthyridine-3-carboxamide

On dissout 0,33 g (1,16 mmol) de chloronaphtyridine obtenue à l'issue de l'étape 1.6 dans 16 ml de diméthoyéthane et 8 ml d'éthanol et fait barboter de l'azote. On ajoute l'acide boronique obtenu à l'issue de l'étape précédente ainsi que 8 ml d'une solution aqueuse saturée de NaHCO₃. On introduit 67 mg (0,06 mmol) de tetrakis(triphenylphosphine)palladium(0) et chauffe le milieu à 110°C pendant 3h. Après refroidissement on filtre sur papier et le filtrat est concentré à sec. Le résidu est repris dans l'eau et le précipité obtenu est filtré, lavé à l'eau et séché à l'étuve sous vide sur P2O5 puis purifié par chromatographie sur silice (éluant : CH₂Cl₂/MeOH :95/5). On obtient 450 mg de produit sous forme de poudre. Rendement : (93%). Point de fusion :>300°C.
RMN¹H (200MHZ, DMSO-d₆): δ(ppm) :1,3 (t ;3H ;7Hz) ;2,8 (d ;3H ;4,5Hz) ; 3,1 (s ;3H) ; 4,6 (q ;2H ;7Hz) ; 7,3 (d ;2H ;8Hz) ; 7,9 (d ; 1 H ; 8Hz) ; 8,2(d ;2H ;8Hz) ; 8,50 (d ;1H ;8Hz) ; 10,1 (s ;1H) ; 11,1 (q ;1H ;4,5Hz) ; 11,7 (br s ; 1H).
MH⁺: 416 (tr: 5,05 min, condition B).

### Exemple 5 : chlorhydrate de 2-amino-1-ethyl-7-(3-fluoro-4-{[(pyridin-3-ylmethyl)sulfonyl]amino}phenyl)-N-methyl-4-oxo-1,4-dihydro-1,8-naphthyridine-3-carboxamide

### 5.1 : N-[2-fluoro-4-(4,4,5,5-tetramethyl-1,3,2-dioxaborolan-2-yl)ohenyl]-1-(pyridin -3-yl)methanesulfonamide

Sous atmosphère inerte, à une solution de 0,28 g (1,18 mmol) du composé obtenu à l'issue de l'étape 2.1, dans 12 ml de pyridine anhydre, 0,493 g (1,30 mmol) de chlorure de pyridin-3-ylméthanesulfonyle trifluoromethanesulfonate sont introduits par portions et le milieu réactionnel est laissé sous agitation pendant 18 heures. On rajoute 0,1 equivalent de chlorure de sulfonyle et laisse agiter 24 heures. La pyridine est évaporée complètement (deux chasses au toluène). Le résidu est redissous dans l'acétate d'éthyle, lavé à l'eau puis séché sur Na₂SO₄ et concentré à sec. Le solide obtenu est recristallisé dans le cyclohexane. On isole 330 mg de poudre blanche. Rendement : 75%. Point de fusion : 206°C.
RMN¹H(400MHz;DMSOd6): δ(ppm) : 1,15 (s ;12H) ; 4,5 (s ;2H); 7,2-7,3 (m ;4H); 7,6 (d;1H;8Hz); 8,35-8,45 (m;2H); 9,8 (s;1H).

### 5.2 : 2-amino-1-ethyl-7-(3-fluoro-4-{[(pyridin-3-ylmethyl)sulfonyl]aminol}phenyl)-N-methyl-4-oxo-1,4-dihydro-1,8-naphthyridine-3-carboxamide

Ce produit a été préparé selon le protocole décrit au paragraphe 2.3 (méthode A) à partir de 0,300 g (0,76 mmol) du composé issu de l'étape précédente et de 0,195 g (0,69 mmol) de chloronaphtyridine issue de l'étape 1.6. On obtient 200 mg de produit sous forme de poudre blanche. Rendement : 56%
RMN¹H (400MHZ,DMSO-d₆): δ(ppm) : 1,2 (t ;3H ;7Hz) ;2,7 (d ;3H ;4,5Hz) ; 4,5 (m ;4H) ; 7,3 (m ;1 H) ; 7,4 (t ;1 H ;8H);7,7 (d ;1 H ;8Hz) ; 7,8-8,0 (m ;3H) ; 8,35 (m ;3H) ; 9,95 (s ;1 H) ; 11,0 (q ;1 H ;4,5Hz) ; 11,6 (br s ; 1H).

### 5.3 : chlorhydrate de 2-amino-1-ethyl-7-(3-fluoro-4-{[(pyridin-3-ylmethyl) sulfonyl]amino}phenyl)-N-methyl-4-oxo-1,4-dihydro-1,8-naphthyridine-3-carboxamide

A une suspension de 0.200 g (0,39 mmol) de produit issu de l'étape précédente dans 10 ml de dichlorométhane, on ajoute goutte à goutte 0,2 ml d'une solution de HCl 2N dans l'éther éthylique. On laisse agiter 10 minutes à 20°C et le précipité est filtré, lavé à l'éther éthylique et séché à l'étuve sous vide. On obtient 202 mg de produit sous forme de poudre blanche. Rendement : 94%. Point de fusion : 220-223°C.
RMN¹H (200MHZ,DMSO-d₆): δ(ppm) :1,3 (t ;3H ;7Hz) ;2,8(s ;3H) ; 4,6 (m ;2H) ; 4,8 (s ;2H) ;7,55 (t ;1H ;8H);7,65 (t ;1H ;8H);7,9-8,2 (m ;4H) ;8,5 (d ;1 H ;8Hz) ; 8,7 (m ;2H); 10,1 (s;1H);11,1 (s; 1H).11,7 (brs;1H).
LCMS : MH+ :511 (tr: 5, 68 min ; condition A).

### Exemple 6 : 2-amino-1-ethyl-7-(3-fluoro-4-{[(3-fluorophenyl)sulfonyl]amino} phenyl)-N-methyl-4-oxo-1,4-dihydro-1,8-naphthyridine-3-carboxamide

### 6.1 : 3-fluoro-N-12-fluoro-4-(4,4,5,5-tetramethyl-1,3,2-dioxaborolan-2-yl)phenyl] benzenesulfonamide :

Ce produit a été préparé selon le protocole décrit au paragraphe 2.2 (méthode A) à partir de 0,5 g (2,11 mmol) du composé issu de l'étape 2.1 et de 0,635 g (3,16 mmol) de chlorure de 3-fluorobenzenesulfonyle. On obtient 0,524 g de produit sous forme de poudre blanche. Rendement: 63%
RMN¹H (400MHZ, DMSO-d₆): δ(ppm) :1,25 (s ;12H) ; 7,3-7,5 (m ;3H) ;7,6-7,9 (m;4H) ;10,55 (s ;1 H).

### 6.2 : 2-amino-1-ethyl-7-(3-fluoro-4-{[(3-fluorophenyl)sulfonyl]amino}phenyl)-N-methyl-4-oxo-1,4-dihydro-1,8-naphthyridine-3-carboxamide:

Ce produit a été préparé selon le protocole décrit au paragraphe 2.3 (méthode A) à partir de 0,496 g (1,25 mmol) du composé issu de l'étape précédente et de 0,320 g (1,14 mmol) de chloronaphtyridine issue de l'étape 1.6. On obtient 0,287 g de produit sous forme de poudre beige. Rendement : 49%. Point de fusion : 256°C
RMN¹H (200MHZ,DMSO-d₆): δ(ppm) :1,3 (t ;3H ;7Hz) ; 2,8 (d ;3H ;4,5Hz) ; 4,55 (q ;2H ;7Hz) ; 7,4 (t ;1 H ;8H); 7,5-7,7 (m ;4H) ; 7,9-8,1 (m ;4H) ; 8,5(d ;1 H ;8Hz); 10,6 (s ;1H) ; 11,1 (q ;1H ;4,5Hz) ; 11,7 (br s ; 1 H).
LCMS : MH+ ;514 (tr :7,38 min ; condition B).

### Exemple 7: 2-amino-1-ethyl-7-(3-fluoro-4-{[(3-methoxyphenyl)sulfonyl]amino} phenyl)-N-methyl-4-oxo-1,4-dihydro-1,8-naphthyridine-3-carboxamide

### 7.1 : N-[2-fluoro-4-(4,4,5,5-tetramethyl-1,3,2-dioxaborolan-2-yl)phenyl]-3-methoxybenzenesulfonamide

Ce produit a été préparé selon le protocole décrit au paragraphe 2.2 (méthode A) à partir de 0,5 g (2,11 mmol) du composé issu de l'étape 2.1 et de 0,674 g (3,16 mmol) de chlorure de 3-methoxybenzenesulfonyle. On obtient 0,454 g de produit sous forme de poudre blanche. Rendement : 53%
RMN¹H (400MHZ,DMSO-d₆): δ(ppm) : 1,25 (s ;12H) ; 3,8 (s.3H); 7,2 (d;1 H;8Hz); 7,25-7,4 (m ;5H) ; 7,6 (t;1H;8Hz) ; 10,4 (s ;1H).

### 7.2: 2-amino-1-ethyl-7-(3-fluoro-4-{[(3-methoxyphenyl)sulfonyl]amino}phenyl)-N-methyl-4-oxo-1,4-dihydro-1,8-naphthyridine-3-carboxamide

Ce produit a été préparé selon le protocole décrit au paragraphe 2.3 (méthode A) à partir de 0,447 g (1,1 mmol) du composé issu de l'étape précédente et de 0,280 g (1,0 mmol) de chloronaphtyridine issue de l'étape 1.6 .On obtient 0,19 g de produit sous forme de poudre blanche. Rendement :36%. Point de fusion :>260°C.
RMN¹H (200MHZ, DMSO-d₆): δ(ppm) :1,25 (t ;3H ;7Hz) ;2,8 (d ;3H ;4,5Hz) ; 4,55 (q ;2H ;7Hz) ;7,2 (d ;1 H ;8Hz) ;7,3-7,5 (m ;4H) ;7,8-8,0 (m ;4H) ;8,5 (d ;1 H ;8Hz) ; 10,4(s ;1H) ; 11,1 (q ;1H ;4,5Hz) ; 11,7 (br s ; 1H).
LCMS : MH+ :526 (tr: 7,56 min ; condition B).

### Exemple 8: 2-amino-1-ethyl-7-(3-fluoro-4-{[(4-fluorophenyl)sulfonyl]amino} phenyl)-N-methyl-4-oxo-1,4-dihydro-1,8-naphthyridine-3-carboxamide :

### 8.1 : 4-fluoro-N-[2-fluoro-4-(4,4,5,5-tetramethyl-1,3,2-dioxaborolan-2-yl)phenyl]benzenesulfonamide

Ce produit a été préparé selon le protocole décrit au paragraphe 2.2 (méthode A) à partir de 0,6 g (2,53 mmol) du composé issu de l'étape 2.1 et de 0,739 g (3,80 mmol) de chlorure de 4-fluorobenzenesulfonyle. On obtient 0,736 g de produit sous forme de poudre blanche. Rendement: 53%
RMN¹H (400MHZ, DMSO-d₆): δ(ppm) :1,1 (s ;12H); 7,0-7,25 (m;5H); 7,65 (m ;2H) ; 10,25 (s ;1H).

### 8.2: 2-amino-1-ethyl-7-(3-fluoro-4-{[(4-fluorophenyl)sulfonyl]amino}phenyl)-N-methyl-4-oxo-1,4-dihydro-1,8-naphthyridine-3-carboxamide

Ce produit a été préparé selon le protocole décrit au paragraphe 2.3 (méthode A) à partir de 0,542 g (1,37 mmol) du composé issu de l'étape précédente et de 0,350 g (1,25 mmol) de chloronaphtyridine issue de l'étape 1.6. On obtient 0,186 g de produit sous forme de poudre blanche. Rendement : 29%. Point de fusion :>260°C.
RMN¹H (200MHZ,DMSO-d₆): δ(ppm) : 1,3 (t ;3H ;7Hz) ; 2,8 (d ;3H ;4,5Hz) ; 4,55 (q ;2H ;7Hz) ; 7,3-7,45 (m ;3H) ; 7,8-8,1 (m ;6H) ; 8,5 (d ;1H ;8Hz) ;10,45 (s ;1H) ; 11,1 (q ;1 H ;4,5Hz) ; 11,7 (br s ; 1 H).
LCMS : MH+ :514 (tr : 7,47 min ; condition B).

### Exemple 9: 2-amino-7-(4-{[(3-chlorophenyl)sulfonyl]amino}-3-fluorophenyl)-1-ethyl-N-methyl-4-oxo-1,4-dihydro-1,8-naphthyridine-3-carboxamide

### 9.1 : 3-chloro-N-[2-fluoro-4-(4,4,5,5-tetramethyl-1,3,2-dioxaborolan-2-yl)phenyl] benzenesulfonamide

Ce produit a été préparé selon le protocole décrit au paragraphe 2.2 (méthode A) à partir de 0,5 g (2,11 mmol) du composé issu de l'étape 2.1 et de 0,579 g (2,74 mmol) de chlorure de 3-chlorobenzenesulfonyle. On obtient 0,408 g de produit sous forme de poudre blanche. Rendement: 47%
RMN¹H (400MHZ, DMSO-d₆): δ(ppm) : 1,3 (s ;12H); 7,3-7,5 (m;3H); 7,6-7,9 (m ;4H) ; 10,6 (s ;1 H).

### 9.2: 2-amino-7-(-4-{[(3-chloroohenyl)sulfonyl]amino}-3-fluorophenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,408 g (0,99 mmol) du composé issu de l'étape précédente et de 0,253 g (0.90 mmol) de chloronaphtyridine issue de l'étape 1.6. On obtient 0,402 g de produit sous forme de poudre jaune. Rendement : 84%. Point de fusion :>260°C.
RMN¹H (200MHZ,DMSO-d₆):δ(ppm) : 1,3 (t ;3H ;7Hz) ; 2,8 (d ;3H ;4,5Hz) ; 4,55 (q ;2H ;7Hz) ; 7,2 (t ;1 H ;8H); 7,3-7,4 (m ;2H) ; 7,55-8,0 (m ;6H) ; 8,35 (d ;1 H ;8H); 10,45 (s ;1H); 11,1 (q ;1 H ;4,5Hz) ; 11,6 (brs ; 1 H).
LCMS : MH+ :530 (tr :7,69 min ; condition B).

### Exemple 10: 2-amino-1-ethyl-7-(3-fluoro-4-{[(2-fluorophenyl)sulfonyl]amino} phenyl)-N-methyl-4-oxo-1,4-dihydro-1,8-naphthyridine-3-carboxamide

### 10.1 : 2-fluoro-N-[2-fluoro-4-(4,4,5,5-tetramethyl-1,3,2-dioxaborolan-2-yl) phenyl]benzenesulfonamide

Ce produit a été préparé selon le protocole décrit au paragraphe 2.2 (méthode A) à partir de 0,5 g (2,11 mmol) du composé issu de l'étape 2.1 et de 0,451 g (2,32 mmol) de chlorure de 2-fluorobenzenesulfonyle. On obtient 0,528 g de produit sous forme de poudre rosée. Rendement : 63%.
RMN¹H (400MHZ,DMSO-d₆): δ(ppm): 1,2 (s ;12H); 7,25-7,5 (m;5H); 7,7-7,8 (m ;2H) ; 11.65(s ;1 H).

### 10.2: 2-amino-1-ethyl-7-(3-fluoro-4-{[(2-fluorophenyl)sulfonyl]amino}phenyl)-N-methyl-4-oxo-1,4-dihydro-1,8-naphthyridine-3-carboxamide

Ce produit a été préparé selon le protocole décrit au paragraphe 2.3 (méthode A) à partir de 0,528 g (1,34 mmol) du composé issu de l'étape précédente et de 0,341 g (1,21 mmol) de chloronaphtyridine issue de l'étape 1.6. On obtient 0,097 g de produit sous forme de poudre jaune pâle. Rendement : 16%. Point de fusion :>260°C.
RMN¹H (200MHZ, DMSO-d₆): δ(ppm) :1,25 (t ;3H ;7Hz) ;2,8 (s ;3H) ; 4,5 (q ;2H ;7Hz) ; 7,2-7,45(m ;3H) ; 7,7-8,0 (m ;6H) ; 8,5 (d ;1H ;8Hz) ; 10,65 (s ;1H) ; 11,1 (q ;1H ;4,5Hz) ; 11,7 (br s ; 1 H).
LCMS : MH+ :514 (tr : 7,25 min ; condition B).

### Exemple 11: 2-amino-7-(4-{[(2,6-dichlorophenyl)sulfonyl]amino}-3-fluorophenyl)-1-ethyl-N-methyl-4-oxo-1,4-dihydro-1,8-naphthyridine-3-carboxamide :

### 11.1 : 2,6-dichloro-N-(2-fluoro-4-iodophenyl)benzenesulfonamide

Ce produit a été préparé selon le protocole décrit au paragraphe 2.4 (méthode B) à partir de 2,8 g (11,81 mmol) de 2-fluoro-4-iodoaniline et de 2,99 g (11,81 mmol) de chlorure de 2,6-dichlorobenzenesulfonyle. On obtient 4,43 g de produit sous forme de poudre jaune. Rendement: 84%.
LCMS : MH+ :446 (tr : 7,55 min ; condition A).

### 11.2 : 2-amino-7-(4-{[(2,6-dichlorophenyl)sulfonyl]amino-3-fluorophenyl)-1-ethyl-N-methyl-4-oxo-1,4₋dihydro-1,8-naphthyridine-3-carboxamide

Ce produit a été préparé selon le protocole décrit au paragraphe 2.5 (méthode B) à partir de 2,0 g (4,48 mmol) du composé issu de l'étape précédente et de 2,0 g (6,89 mmol) de l'acide boronique issu de l'étape 1.7. On obtient 0,700 g de produit sous forme de poudre blanche. Rendement : 28%. Point de fusion : 321 °C.
RMN¹H (200MHZ,DMSO-d₆): δ (ppm) : 1,3 (t ;3H ;7Hz) ; 2,8 (d ;3H ;4,5Hz) ; 4,55 (q ;2H ;7Hz) ; 7,35 (t ;1H ;8Hz) ; 7,45-7,7 (m ;3H) ; 7,8-8,2 (m ;4H) ; 8,5 (d ;1 H ;8Hz) ; 10,9 (s ;1H) ; 11,1 (q ;1 H ;4,5Hz) ; 11,7 (br s ; 1 H).
LCMS : MH+ :564 (tr :14,33 min ; condition A).

### Exemple 12: 2-amino-7-{2-chloro-4-{[(2,3-dichlorophenyl)sulfonyl]amino} phenyl)-1-ethyl-N-methyl-4-oxo-1,4-dihydro-1,8-naphthyridine-3-carboxamide

### 12.1 : 3-chloro-4-(4,4,5,5-tetramethyl-1,3,2-dioxaborolan-2-yl)aniline

Ce produit a été préparé selon le protocole décrit au paragraphe 2.1 (méthode A) à partir de 3,0 g (11,84 mmol) de 3-chloro-4-iodoaniline et de 3,31 g (13,0 mmol) de bis(pinacolato) diborane. On isole 1,51 g de produit sous forme de solide blanc.
Rendement : 50%
RMN¹H(400MHz; CDCl₃): δ(ppm) : 1,4 (s ;12H) ; 3,95(br s ;2H) ; 6,55 (d ;1 H ;8Hz) ; 6,7 (s;1 H); 7,6 (d ;1H;8Hz).

### 12.2 : 2,3-dichloro-N-[3-chloro-4-(4,4,5,5-tetramethyl-1,3,2-dioxaborolan-2-yl)phenyl]benzenesulfonamide

Ce produit a été préparé selon le protocole décrit au paragraphe 2.2 (méthode A) à partir de 0,6 g (2,37 mmol) du composé issu de l'étape précédente et de 0,593 g (2,37 mmol) de chlorure de 2,3-dichlorobenzenesulfonyle. On obtient 0,944 g de produit sous forme de poudre beige clair. Rendement : 86%.
RMN¹H (400MHZ,DMSO-d₆): δ(ppm) : 1,2 (s ;12H); 7,0 (d;1H;8Hz);7,1 (s;1H); 7,5 (d;1H;8Hz); 7,6 (t;1 H;8Hz); 7,95 (d;1H;8Hz); 8,10 (d;1H;8Hz); 11.25 (s ;1 H).

### 12.3 : 2-amino-7-(2-chloro-4-{[(2,3-dichlorophenyl)sulfonyl]amino}phenyl)-1-ethyl-N-methyl-4-oxo-1,4-dihydro-1,8-naphthyridine-3-carboxa-mide

Ce produit a été préparé selon le protocole décrit au paragraphe 2.3 (méthode A) à partir de 0,943 g (2,04 mmol) du composé issu de l'étape précédente et de 0,515 g (1,84 mmol) de chloronaphtyridine issue de l'étape 1.6. On obtient 0,630 g de produit sous forme de poudre beige. Rendement : 53%. Point de fusion : 239°C.
RMN¹H (200MHZ,DMSO-d₆):δ(ppm) :1,2(t ;3H ;7Hz) ;2,8(d;3H ;4,5Hz) ; 4,5(q ;2H ;7Hz) ;7,1-7,3(m ;2H) ;7,5-7,8(m ;4H) ;8,0(s ;1H) ;8,1(d ;1H ;2Hz) ; 8,5(d ;1H ;8Hz) ; 11,05(q ;1H ;4,5Hz) ; 11,3(s ;1H) 11,7(br s ; 1 H).
LCMS : MH+ :580 (tr : 7,74 min ; condition A).

### Exemple 13: 2-amino-7-(4-{[(4-chlorophenyl)sulfonyl]amino}-3-fluorophenyl)-1-ethyl-N-methyl-4-oxo-1,4-dihydro-1,8-naphthyridine-3-carboxamide

### 13.1 : 4-chloro-N-[2-fluoro-4-(4,4,5,5-tetramethyl-1,3,2-dioxaborolan-2-yl) phenyl]benzenesulfonamide

Ce produit a été préparé selon le protocole décrit au paragraphe 2.2 (méthode A) à partir de 0,5 g (2,11 mmol) du composé issu de l'étape 2.1 et de 0,468 g (2,15 mmol) de chlorure de 4-chlorobenzenesulfonyle. On obtient 0,645 g de produit sous forme de poudre rose. Rendement : 75%. Point de fusion : 196°C
RMN¹H (400MHZ,DMSO-d₆):δ(ppm) :1,3(s ;12H);7,35-7,45(m;2H);7,50(d;1H;8Hz); 7,7(d ;2H;8Hz) ; 7,8(d ;2H ;8Hz) ; 10,55(s ;1 H).

### 13.2 : 2-amino-7-(4-{[(4-chlorophenyl)sulfonyl]amino}-3-fluorophenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,613 g (1,49 mmol) du composé issu de l'étape précédente et de 0,380 g (1,35 mmol) de chloronaphtyridine issue de l'étape 1.6. On obtient 0,407 g de produit sous forme de poudre jaune. Rendement : 57%. Point de fusion :>260°C.
RMN¹H (200MHZ,DMSO-d₆):δ(ppm) :1,3(t ;3H ;7Hz) ;2,8(d ;3H ;4,5Hz) ; 4,55(q ;2H ;7Hz) ;7,4(t ;1H ;8Hz) ;7,7(d ;2H ;8Hz) ;7,8(d ;2H ;8Hz) ;7,8-8,1(m ;4H) ; 8,5(d ;1H ;8Hz) ;10,55(s ;1H) ; 11,1 (q ;1H ;4,5Hz); 11,7(br s ; 1 H).
LCMS : MH+ :530 (tr :7,91 min ; condition A).

### Exemple 14: 2-amino-7-(4-{[(3,4-difluorophenyl)sulfonyl]amino)-3-fluorophenyl)-1-ethyl-N-methyl-4-oxo-1,4-dihydro-1,8-naphthyridine-3-carboxamide

### 14.1 : 3.4-difluoro-N-[2-fluoro-4-(4,4,5,5-tetramethyl-1,3,2-dioxaborolan-2-yl)phenyl]benzenesulfonamide

Ce produit a été préparé selon le protocole décrit au paragraphe 2.2 (méthode A) à partir de 0,4 g (1,69 mmol) du composé issu de l'étape 2.1 et de 0,518 g (2,36 mmol) de chlorure de 3,4-difluorobenzenesulfonyle. On obtient 0,437 g de produit sous forme de poudre blanche. Rendement :63%. Point de fusion :114°C
RMN¹H (400MHZ,DMSO-d₈):δ(ppm) :1,2(s ;12H);7,2(m;2H);7,3(d ;1 H ;8Hz) ;7,5-7,65(m ;2H) ;7,7(t;1H;8Hz);10,4(s ;1 H).

### 14.2 : 2-amino-7-(4-{[(3,4-difluorophenyl)sulfonyllamino}-3-fluorophenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,400 g (0,97 mmol) du composé issu de l'étape précédente et de 0,259 g (0,92 mmol) de chloronaphtyridine issue de l'étape 1.6. On obtient 0,228 g de produit sous forme de poudre blanche. Rendement :46%. Point de fusion :>260°C.
RMN¹H (200MHZ,DMSO-d₆):δ(ppm) :1,3(t ;3H ;7Hz) ;2,8(s ;3H) ; 4,55(q ;2H ;7Hz) ;7,4(t ;1H ;8Hz) ;7,5-7,7(m ;2H) ;7,8-8,1(m ;5H) ; 8,5(d ;1H ;8H) ;10,6(s ;1 H) ; 11,05(q ;1H ;4,5Hz) ; 11,7(br s ; 1 H).
LCMS : MH+ :532 (tr :7,55 min ; condition A).

### Exemple 15: chlorhydrate de 2-amino-1-ethyl-7-[3-fluoro-4-({[6-(morpholin-4-yl)pyridin-3-yl]sulfonyl}amino)phenyl]-N-methyl-4-oxo-1,4-dihydro-1,8-naphthyridine-3-carboxamide

### 15.1 : N-12-fluoro-4-(4,4,5,5-tetramethyl-1,3,2-dioxaborolan-2-yl)phenyl]-6-(morpholin-4-yl)pyridine-3-sulfonamide

Ce produit a été préparé selon le protocole décrit au paragraphe 5.1 à partir de 0,5 g (2,11 mmol) du composé issu de l'étape 2.1 et de 0,743 g (2,74 mmol) de chlorure de 6-(morpholin-4-yl)pyridine-3-sulfonyle. On obtient 0,614 g de produit sous forme de poudre blanche. Rendement : 70%. Point de fusion : 206°C
RMN¹H (400MHZ,DMSO-d₆):δ(ppm) :1,2(s ;12H); 3,45(s ;4H) ;3,55(s;4H);6,8(m;1 H); 7,2(m;1H); 7,3(m ;2H) ; 7,65(m ;1H) ;8,25(m;1H);10,1(s ;1H).

### 15.2 : 2-amino-1-ethyl-7-[3-fluoro-4-({[6-(morpholin-4-yl)pyridin-3-yl]sulfonyl} amino)phenyl]-N-methyl-4-oxo-1,4-dihydro-1,8-naphthyridine-3-carboxamide

Ce produit a été préparé selon le protocole décrit au paragraphe 2.3 (méthode A) à partir de 0,599 g (1,29 mmol) du composé issu de l'étape précédente et de 0,330 g (1,18 mmol) de chloronaphtyridine issue de l'étape 1.6. On obtient 0,360 g de produit sous forme de poudre jaune. Rendement : 53%. Point de fusion : 260°C.
RMN¹H (200MHZ, DMSO-d₆): δ(ppm) : 1,25 (t ;3H ;7Hz) ; 2,8 (d;3H ;4,5Hz) ; 3,5-3,75 (m ;8H) ; 4,55 (q ;2H ;7Hz) ; 6,90 (d ;1H ;8Hz) ; 7,5 (t ;1H ;8Hz) ; 7,6 (brs ;1H) ; 7,8 (dd ;1H ;8Hz et 2Hz); 7,9-8,1 (m ;3H) ; 8,4 (d ;1H ;2Hz) ; 8,5 (d ;1H ;8H) ;10,3 (s ;1H) ; 11,05 (q ;1 H ;4,5Hz) ; 11,7 (br s ; 1 H).

### 15.3 : chlorhydrate de 2-amino-1-ethyl-7-[3-fluoro-4-({[6-(moroholin-4-yl) pyridin-3-yllsulfonyl}amino)phenyl]-N-methyl-4-oxo-1,4-dihydro-1,8-naphthyridine-3-carboxamide

Le produit issu de la réaction précédente (0,2 g -0,34 mmol) est salifié selon le protocole utilisé au paragraphe 5.3 en utilisant 0,17 ml d'une solution 2N de HCl dans l'éther. On isole 0,203 g de produit sous forme de poudre jaune. Rendement : 95% ; Point de fusion :>260°C.
RMN¹H (200MHZ, DMSO-d₆): δ(ppm) :1,25 (t ;3H ;7Hz) ; 2,8 (d;3H ;4,5Hz) ; 3,5-3,8 (m ;8H) ; 4,55 (q ;2H ;7Hz) ; 6,90 (d ;1 H ;8H); 7,5 (t ;1H ;8H); 7,8 (dd ;1 H ;8Hz et 2Hz); 7,85-8,1 (m ;4H) ; 8,4 (d ;1H ;2Hz) ; 8,5 (d ;1H ;8H); 10,3 (s ;1H) ; 11,1 (q ;1 H ;4,5Hz) ; 11,7 (br s ; 1 H).
LCMS : MH+ :582 (tr :7,07 min ; condition B).

### Exemple 16: chlorhydrate de 2-amino-1-ethyl-7-(3-fluoro-4-{[(pyridin-2-ylmethyl)sulfonyl]amino}phenyl)-N-methyl-4-oxo-1,4-dihydro-1,8-naphthyridine-3-carboxamide

### 16.1 : N-[2-fluoro-4-(4,4,5,5-tetramethyl-1.3,2-dioxaborolan-2-yl)phenyl]-1-(pyridin-2-yl)methanesulfonamide

Ce produit a été préparé selon le protocole décrit au paragraphe 5.1 à partir de 028 g (1,18 mmol) du composé issu de l'étape 2.1 et de 0,493 g (1,30 mmol) de chlorure de pyridin-2-ylméthanesulfonyle trifluorométhanesulfonate. On obtient 0,441 g de produit sous forme de poudre blanche. Rendement: 95%. Point de fusion:152°C
RMN¹H (400MHZ, DMSO-d₆):δ(ppm) :1,3 (s ;12H); 4,7 (s ;2H) ; 7,35-7,45 (m;4H); 7,5(d;1 H;8Hz); 7,8 (t;1 H;8Hz) ; 8,55 (d;1 H;2Hz); 9,95 (s ;1 H).

### 16.2 : 2-amino-1-ethyl-7-(3-fluoro-4-{[(pyridin-2-ylmethyl)sulfonyl]amino} phenyl)-N-methyl-4-oxo-1,4-dihydro-1,8-naphthyridine-3-carboxamide

Ce produit a été préparé selon le protocole décrit au paragraphe 2.3 (méthode A) à partir de 0,400 g (1,02 mmol) du composé issu de l'étape précédente et de 0,260 g (0,93 mmol) de chloronaphtyridine issue de l'étape 1.6. On obtient 0,204 g de produit sous forme de poudre jaune. Rendement : 43%.
RMN¹H (400MHZ, DMSO-d₆): δ(ppm) :1,3 (t ;3H ;7Hz) ; 2,8 (d;3H ;4,5Hz) ; 4,6 (q ;2H ;7Hz) ; 4,7 (s ;2H) ; 7,35 (m ;1H) ; 7,5 (d ;1H ;8H);7,6 (t ;1H ;8Hz) ; 7,8 (t ;1H ;8Hz); 7,95-8,1 (m ;4H) ; 8,55 (m ;2H) ; 9,95 (s ;1 H) ; 11,1 (q ;1H ;4,5Hz) ; 11,7 (br s ; 1H).

### 16.3 : chlorhydrate de 2-amino-1-ethyl-7-(3-fluoro-4-{[(pyridin-2-ylmethyl) sulfonyl]amino}phenyl)-N-methyl-4-oxo-1,4-dihydro-1,8-naphthyridine-3-carboxamide

Le produit issu de la réaction précédente (0,204 g -0,4 mmol) est salifié selon le protocole utilisé au paragraphe 5.3 en utilisant 0,2 ml d'une solution 2N de HCl dans l'éther. On isole 0,202 g de produit sous forme de poudre jaune. Rendement :92% ; Point de fusion :260°C.
RMN¹H (200MHZ, DMSO-d₆): δ (ppm) :1,3 (t ;3H ;7Hz) ; 2,8 (d ;3H ;4,5Hz); 4,6 (m ;2H) ; 4,75 (s ;2H) ; 7,4 (m ;1H) ; 7,55 (t ;1 H ;8Hz) ;7,85 (t ;1 H ;8Hz);7,9-8,1 (m ;5H) ; 8,55 (m;2H); 10,0 (s ;1 H) ; 11,1 (q ;1 H ;4,5Hz) ; 11,7 (br s ; 1 H).
LCMS : MH+ :511 (tr: 6,33 min ; condition A).

### Exemple 17: 2-amino-7-{4-[(ethenylsulfonyl)amino]-3-fluorophenyl}-1-ethyl-N-methyl-4-oxo-1,4-dihydro-1,8-naphthyridine-3-carboxamide :

### 17.1 : N-[2-fluoro-(4,4,5,5-tetramethyl-1,3,2-dioxaborolan-2-yl)phenyl] ethenesulfonamide

Ce produit a été préparé selon le protocole décrit au paragraphe 2.2 (méthode A) à partir de 0,5 g (2,11 mmol) du composé issu de l'étape 2.1 et de 0,425 g (2,53 mmol) de chlorure de 2-chloroéthanesulfonyle. On obtient 0,390 g de produit sous forme d'une huile rosée. Rendement : 56%.
RMN¹H (400MHZ, DMSO-d₆): δ(ppm) : 1,4(s,;12H); 6,2 (m ;2H) ; 7,0(m;1H); 7,5-7,7 (m ;3H) ; 10,2 (s ;1H).

### 17.2 : 2-amino-7-{4-[(ethenylsulfonyl)amino]-3-fluorophenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,390 g (1,2 mmol) du composé issu de l'étape précédente et de 0,280 g (1,0 mmol) de chloronaphtyridine issue de l'étape 1.6. On obtient 0,095 g de produit sous forme de poudre blanche. Rendement: 21 %. Point de fusion :>260°C.
RMN¹H (200MHZ, DMSO-d₆): δ(ppm) :1,3 (t ;3H ;7Hz) ; 2,8 (d;3H ;4,5Hz) ; 4,6(q ;2H ;7Hz) ; 6,05 (d ;1H ;12Hz) ; 6,1 (d ;1 H ;16Hz) ; 6,85 (dd ;1 H ;12 et 16Hz) ; 7,5 (t ;1 H ;8Hz) ; 7,8-8,2 (m ;5H) ; 8,5 (d ;1 H ;8H) ;10,1 (s ;1 H) ; 11,0 (q ;1 H ;4,5Hz) ; 11,7 (br s ; 1 H).
LCMS : MH+ :446 (tr: 6,75 min ; condition A).

### Exemple 18: chlorhydrate de 2-amino-7-[4-({[2-(dimethylamino)ethyl] sulfonyl}amino)-3-fluorophenyl]-1-ethyl-N-methyl-4-oxo-1,4-dihydro-1,8-naphthyridine-3-carboxamide :

### 18.1 : 2-(dimethylamino)-N-[2-fluoro-4-(4,4,5,5-tetramethyl-1,3,2-dioxaborolan-2-yl)phenyl]ethanesulfonamide

A une solution de 0,23 g (0,7 mmol) du produit issu de l'étape 17.1 dans 7 ml de toluène, sont ajoutés goute à goutte 0,35 ml d'une solution 2N de diméthylamine dans le THF et l'agitation est maintenue 3 heures à 20°C. Le milieu réactionnel est évaporé à sec et le résidu repris dans 20 ml d'acétate d'éthyle, lavé avec 20 ml d'eau puis séché sur Na₂SO₄ et concentré à sec. On isole 0,261 g de produit sous forme d'une cire blanche.
Rendement:100%
RMN¹H (400MHZ, DMSO-d₆): δ(ppm) : 1,3 (s ;12H); 2,25 (s;6H); 2,8 (m;2H); 3,35 (m;2H); 7,4 (d;1H;8Hz); 7,5 (m ;2H).
LCMS: MH+:373 (tr: 5,29 min ; condition A)

### 18.2: 2-amino-7-[4-({[2-(dimethylamino)ethyl}sulfonyl}amino)-3-fluorophenyl]-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,261 g (0,70 mmol) du composé issu de l'étape précédente et de 0,179 g (0,64 mmol) de chloronaphtyridine issue de l'étape 1.6. On obtient 0,200 g de produit sous forme de poudre jaune. Rendement : 64%.
LCMS : MH+ :491 (tr :5,22 min ; condition A).

### 18.3 : chlorhydrate de 2-amino-7-[4-({[2-(dimethylamino)ethyl]sulfonyl}amino)-3-fluorophenyl]-1-ethyl-N-methyl-4-oxo-1,4-dihydro-1,8-naphthyridine-3-carboxamide

Le produit issu de la réaction précédente (0,200 g -0,41 mmol) est salifié selon le protocole utilisé au paragraphe 5.3 en utilisant 0,2 ml d'une solution 2N de HCl dans l'éther. On isole 0,055 g de produit sous forme de poudre jaune. Rendement : 25% ; Point de fusion : 268-270°C.
RMN¹H (200MHZ, DMSO-d₆): δ(ppm) :1,3 (t ;3H ;7Hz) ; 2,25 (s ;6H) ; 2,8 (m ;5H) ; 3,3 (m ;2H) ; 4,6 (m ;2H); 7,55 (t ;1H ;8Hz); 7,8-8,1 (m ;4H) ; 8,5 (d;1H ;8Hz); 11,05 (m;1H); 11,7 (br s; 1H).
LCMS : MH+ :491 (tr :4,99 min condition A).

### Exemple 19: chlorhydrate de 2-amino-1-éthyl-7-(3-fluoro-4-{[(1-méthyl-1H-imidazol-4-yl)sulfonyl]amino}phényl)-N-méthyl-4-oxo-1,4-dihydro-1,8-naphtyridine-3-carboxamide

### 19.1 : N-[2-fluoro-4-(4,4,5,5-tétraméthyl-1,3,2-dioxaborolan-2-yl)phényl]-1-méthyl-1H-imidazole-4-sulfonamide

Ce produit a été préparé selon le protocole décrit au paragraphe 2.2 (méthode A) à partir de 0,5 g (2,11 mmol) du composé issu de l'étape 2.1 et de 0,481 g (2,53 mmol) de chlorure de 1-méthyl-1H-imidazole-4-sulfonyle. On obtient 0,574 g de produit sous forme d'une poudre blanche. Rendement : 71%. Point de fusion: 230°C.
RMN¹H (400MHZ, DMSO-d₆): δ(ppm) : 1,1 (s ;12H); 3,4 (s ;3H); 7,05 (s;1 H); 7,1 (s;1H); 7,1 (s;1H); 7,55 (m ;2H); 9,9 (s ;1H).

### 19.2 : 2-amino-1-éthyl-7-(3-fluoro-4-{[(1-méthyl-1H-imidazol-4-yl)sulfonyl] amino}phényl)-N-méthyl-4-oxo-1,4-dihydro-1,8-naphtyridine-3-carboxamide

Ce produit a été préparé selon le protocole décrit au paragraphe 2.3 (méthode A) à partir de 0,574 g (1,50 mmol) du composé issu de l'étape précédente et de 0,384 g (1,37 mmol) de chloronaphtyridine issue de l'étape 1.6. On obtient 0,202 g de produit sous forme de poudre jaune. Rendement : 30%.
LCMS : MH+ :500 (tr : 6,01 min ; condition A).

### 19.3 : chlorhydrate de 2-amino-1-éthyl-7-(3-fluoro-4-{[(1-méthyl-1H-imidazol-4-yl)sulfonyl]aminolphényl)-N-méthyl-4-oxo-1,4-dihydro-1,8-naphtyridine-3-carboxamide

Le produit issu de la réaction précédente (0,200 g -0,40 mmol) est salifié selon le protocole utilisé au paragraphe 5.3 en utilisant 0,2 ml d'une solution 2N de HCl dans l'éther. On isole 0,178 g de produit sous forme de poudre blanche. Rendement :82% ; Point de fusion :>300°C.
RMN¹H (200MHZ, DMSO-d₆): δ(ppm) : 1,35(t ;3H ;7Hz) ; 2,8 (d ;3H ;4,5Hz) ; 3,7 (s ;3H) ; 4,6 (m ;2H) ; 7,65 (t ;1 H ;8Hz) ; 7,8 (s ;1H) ; 7,85 (s ;1H);7,9-8,1 (m ;4H) ; 8,5 (d;1 H ;8Hz); 10,25 (s ;1 H) ; 11,1 (q;1 H ;4,5Hz) ; 11,7 (br s ; 1H).
LCMS : MH+ :500 (tr : 6,06 min ; condition A).

### Exemple 20: 2-amino-7-{4-[(butylsulfonyl)amino]-3-fluorophenyl}-1-ethyl-N-methyl-4-oxo-1,4-dihydro-1,8-naphthyridine-3-carboxamide :

### 20.1 : N-(2-fluoro-4-(4,4,5,5-tetramethyl-1,3,2-dioxaborolan-2-yl)ahenyllbutane-1-sulfonamide

Ce produit a été préparé selon le protocole décrit au paragraphe 2.2 (méthode A) à partir de 0,400 g (1,69 mmol) du composé issu de l'étape 2.1 et de 0,31 ml (2,36 mmol) de chlorure de butane-1-sulfonyle. On obtient 0,652 g de produit sous forme d'une huile orangée. Rendement : 100%.
RMN¹H (400MHZ, DMSO-d₆): δ (ppm) : 0,8 (t;3H;7Hz); 1,3 (s ;12H); 1,4 (m;2H); 1,7(m;2H); 3,15 (m;2H); 7,4 (d ;1H ;8Hz) ; 7,5 (s;2H); 9,8 (s ;1H).

### 20.2 : 2-amino-7-{4-[(butylsulfonyl)amino]-3-fluorophenyl}-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,636 g (1,34 mmol) du composé issu de l'étape précédente et de 0,326 g (1,16 mmol) de chloronaphtyridine issue de l'étape 1.6. On obtient 0,200 g de produit sous forme de poudre blanche. Rendement : 36%. Point de fusion : 165-167°C.
RMN¹H (200MHZ,DMSO-d₆): δ(ppm) :0,9 (t ;3H ;7Hz) ; 1,2-1,4 (m ;5H) ; 1,75 (m ;2H) ;2,8 (d;3H ; 4,5Hz) ; 3,2 (m ;2H) ; 4,6 (m ;2H) ; 7,65 (t ;1H ;8Hz) ; 7,8-8,2 (m ;4H) ; 8,5 (d ;1H ;8Hz) ; 9,9 (s ;1H) ; 11,0 (q ;1H ;4,5Hz) ; 11,7 (br s ; 1H).
LCMS : MH+ :476 (tr : 7,43 min ; condition A).

### Exemple 21: chlorhydrate de 2-amino-7-(4-{[(3-aminobenzyl)sulfonyl]amino}-3-fluorophenyl)-1-ethyl-N-methyl-4-oxo-1,4-dihydro-1,8-naphthyridine-3-carboxamide

### 21.1 : N-[2-fluoro-4-(4,4,5,5-tetramethyl-1,3,2-dioxaborolan-2-yl)phenyl]-1-(3-nitrophenyl)methanesulfonamide

Ce produit a été préparé selon le protocole décrit au paragraphe 2.2 (méthode A) à partir de 0,400 g (1 ,69 mmol) du composé issu de l'étape 2.1 et de 0,502 g (2,02 mmol) de chlorure de (3-nitrophényl)méthanesulfonyle. On obtient 0,584 g de produit sous forme d'une poudre beige. Rendement: 80%. Point de fusion: 178°C.
RMN¹H (400MHZ, DMSO-d₆): δ(ppm) : 1,3 (s ;12H); 4,8 (s ;2H); 7,3-7,45 (m;3H); 7,65 (m;1H); 7,8 (m;1H); 8,2 (m ;2H); 10,0 (s ;1H).

### 21.2 : 1-(3-aminophenyl)-N-[2-fluoro-4-(4,4,5,5-tetramethyl-1,3,2-dioxaborolan-2-yl)phenyl]methanesulfonamide

0,58 g (1,33 mmol) du prduit issu de l'étape précédente sont dissous dans 6 ml d'acétate d'éthyle et 1,38 g (6,65 mmol) de chlorure d'étain(II) dihydrate sont ajoutés à 20°C. Le milieu réactionnel est maintenu sous agitation pendant 15 heures et 0,276 g de chlorure d'étain(II) dihydrate sont ajoutés. Le milieu réactionnel est chauffé à 80°C pendant 3 heureset laissé 15 heures à 20°C. Cette solution est versée lentement sur une solution aqueuse de NaHCO₃ (4,18 g dans 34 ml d'eau) et l'insoluble est filtré. Le filtrat est extrait avec 3X100 ml d'acétate d'éthyle, lavé à l'eau puis séché sur Na₂SO₄ et évaporé à sec. On obtient 0,42 g de produit sous forme de cire blanche. Rendement: 78%.
RMN¹H (400MHZ, DMSO-d₆): δ(ppm) : 1,3 (s ;12H); 4,35 (s ;2H); 5,15 (s;2H); 6,5(m;1 H); 6,6 (m;2H); 7,0(m;1 H); 7,4 (m ;3H); 9,9 (s ;1 H).

### 21.3 : 2-amino-7-(4-{[(3-aminobenzyl)sulfonyllamino}-3-fluoroohenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,414 g (1,02 mmol) du composé issu de l'étape précédente et de 0,260 g (0,93 mmol) de chloronaphtyridine issue de l'étape 1.6. On obtient 0,160 g de produit sous forme de poudre jaune. Rendement : 33%.
LCMS : MH+ :525 (tr : 6,11 min ; condition A).

### 21.4 : chlorhydrate de 2-amino-7-(4-{[(3-aminobenzyl]sulfonyl]amino}-3-fluorophenyl)-1-ethyl-N-methyl-4-oxo-1,4-dihydro-1,8-naphthyridine-3-carboxamide

Le produit issu de la réaction précédente (0,160 g -0,31 mmol) est salifié selon le protocole utilisé au paragraphe 5.3 en utilisant 0,15 ml d'une solution 2N de HCl dans l'éther. On isole 0,100 g de produit sous forme de poudre beige. Rendement :58% ; Point de fusion :>260°C.
RMN¹H (200MHZ, DMSO-d₆): δ(ppm) :1,3 (t ;3H ;7Hz) ; 2,8 (d ;3H ;4,5Hz); 4,4 (s ;2H) ; 4,6 (m ;2H) ; 6,6-6,8 (m ;3H) ;7,05 (t ;1 H ;8Hz) ; 7,5 (t ;1H ;8Hz) ; 7,9-8,1 (m ;4H) ; 8,5 (d;1 H ;8Hz); 9,9 (s ;1 H) ; 11,1 (q;1 H ;4,5Hz) ; 11,7 (br s ; 1H).
LCMS : MH+ :525 (tr : 6,02 min ; condition A).

### Exemple 22: 2-amino-7-(4-{[(2-chlorophenyl)sulfonyl]amino}phenyl)-1-ethyl-N-methyl-4-oxo-1,4-dihydro-1,8-naphthyridine-3-carboxamide

### 22.1 : 2-chloro-N-[4-(4,4,5,5-tetramethyl-1,3,2-dioxaborolan-2-yl)phenyl] benzenesulfonamide

Ce produit a été préparé selon le protocole décrit au paragraphe 1.8-A à partir de 0,660 g (3,01 mmol) de 4-(4,4,5,5-tetramethyl-1,3,2-dioxaborolan-2-yl) aniline et de 0,635 g (3,01 mmol) de chlorure de 2-chlorobenzenesulfonylesulfonyle. On obtient 1,13 g de produit sous forme d'une poudre rouge foncé. Rendement: 96%. Point de fusion: 198°C
RMN¹H (400MHZ, DMSO-d₆): δ (ppm); 1,25 (s ;12H); 7,2 (d;2H;8Hz); 7,4 (m;1 H); 7,5 (m;2H); 7,6 (d ;1H ;8H); 8,1 (d ;1H ;8Hz).

### 22.2 : 2-amino-7-(4-{[(2-chlorophenyl)sulfonyl]aminolphenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,413 g (1,05 mmol) du composé issu de l'étape précédente et de 0,280 g (1,0 mmol) de chloronaphtyridine issue de l'étape 1.6. On obtient 0,180 g de produit sous forme de poudre blanche. Rendement : 35%. Point de fusion : >260°C.
RMN¹H (200MHZ, DMSO-d₆): δ (ppm) : 1,85(t ;3H ;7Hz); 2,8 (d;3H ;4,5Hz) ; 4,55 (m ;2H); 7,2 (d ;2H ;8Hz) ; 7,4-7,7 (m ; 3H) ; 7,8 (d ;1 H ; 8Hz) ; 7,8-8,2 (m; 3H) ; 7,9(s ;1 H) ; 8,45 (d ;1 H ;8Hz) ; 10,9 (s ;1 H) ;11,05 (q ;1 H ;4,5Hz) ; 11,7 (br s ; 1H).
LCMS : MH+ : 512 (tr : 6,12 min ; condition B).

### Exemple 23: 2-amino-7-(3-chloro-4-{[(2,3-dichlorophenyl)sulfonyl]amino} phenyl)-1-ethyl-N-methyl-4-oxo-1,4-dihydro-1,8-naphthyridine-3-carboxamide

### 23.1 : 2-chloro-4-(4,4,5,5-tetramethyl-1,3,2-dioxaborolan-2-yl)aniline

Ce produit a été préparé selon le protocole décrit au paragraphe 2.1 (méthode A) à partir de 5,0 g (24,22 mmol) de 4-bromo-2-chloro aniline et de 6,76 g (26,64 mmol) de bis(pinacolato)diborane. On obtient 3,14 g de produit sous forme d'une huile marron utilisée dans l'étape suivante sans purification supplémentaire.

### 23.2 : 2,3-dichloro-N-[2-chloro-4-(4,4,5,5-tetramethyl-1,3,2-dioxaborolan-2-yl) phenyl]benzenesulfonamide

Ce produit a été préparé selon le protocole décrit au paragraphe 2.2 (méthode A) à partir de 1,5 g (5,92 mmol) du composé issu de l'étape précédente et de 2,18 g (8,87 mmol) de chlorure de 2,3-dichlorobenzenesulfonyle. On obtient 1,57 g de produit sous forme d'une poudre beige. Rendement: 57%. Point de fusion:156°C.

### 23.3 : (3-chloro-4-{[(2,3dichlorophenyl)sulfonyl]amino}phenyl)boronique acide

0,3 g (6,09 mmol) du produit issu de l'étape précédente sont dissous dans 100 ml de dichlorométhane anhydre et cette solution est refroidie à -78°C. 20,0 ml d'une solution 1 M de trichlorure de bore dans le dichlorométhane sont ajoutés goutte à goutte et le milieu réactionnel est maintenu à -78°C pendant 3 heures puis pendant 15h à 20°C. A 0°C sont ajoutés 11 ml de méthanol puis le milieu réactionnel est évaporé à sec. Le résidu est repris dans 20 ml de dichlorométhane et extrait avec une solution de soude 0,5N puis la phase aqueuse est acidifiée à pH=1 par ajout de HCl 1N. Le précipité formé est filtré, lavé avec un peu d'eau et séché à l'étuve sous vide sur P₂O₅. On obtient 1,03 g de produit sous forme de poudre blanche. Rendement :42%. Point de fusion :90°C.
RMN¹H (400MHZ, DMSO-d₆): δ(ppm) : 7,4 (d;1H;8Hz);7,55 (t;1H;8Hz);7,8 (d ;1H;8Hz); 7,95 (s;1H); 8,0 (d ;1H;8Hz); 8,1 (d ;1H;8Hz); 8,3 (s ;2H); 10,6 (s ;1H).

### 23.4 : 2-amino-7-(3-chloro-4-{[(2,3dichlorophenyl)sulfonyl]amino}phenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 1,02 g (2,68 mmol) du composé issu de l'étape précédente et de 0,565 g (2,24 mmol) de chloronaphtyridine issue de l'étape 1.6 .On obtient 0,698 g de produit sous forme de poudre jaune clair. Rendement: 54%. Point de fusion : 311°C.
RMN¹H (400MHZ, DMSO-d₆): δ(ppm) : 1,3 (t ;3H ;7Hz) ; 2,8 (d ; 3H ; 4,5Hz); 4,55 (q ;2H ; 7Hz) ; 7,45 (d;1H; 8Hz); 7,55 (t;1H;8Hz); 7,9 (d ;1 H; 8Hz); 7,95 (m;2H); 8,0 (s ; 1 H); 8,15 (d ;1H;8Hz) ; 8,25 (s;1H); 8,5 (d ;1H;8Hz) ; 10,65 (s ;1H). 11,05 (q ; 1 H ; 4,5Hz) ;11,7 (br s ;1 H).
LCMS : MH+ :582 (tr : 8,58 min ; condition A).

### Exemple 24: 2-amino-7-(4-{[(2,3-dichlorophenyl)sulfonyl]amino}-2-fluorophenyl)-1-ethyl-N-methyl-4-oxo-1,4-dihydro-1,8-naphthyridine-3-carboxamide

### 24.1 : 3-fluoro-4-(4,4,5,5-tetramethyl-1,3,2-dioxaborolan-2-yl)aniline

Ce produit a été préparé selon le protocole décrit au paragraphe 2.1 (méthode A) à partir de 5,0 g (26,3 mmol) de 4-bromo-3-fluoro aniline et de 7.34 g (28,9 mmol) de bis(pinacolato)diborane. On obtient 2,45 g de produit sous forme d'une huile marron utilisée dans l'étape suivante sans purification supplémentaire.

### 24.2 : 2,3-dichloro-N-[3-fluoro-4-(4,4,5,5-tetramethyl-1,3,2-dioxaborolan-2-yl) phenyl]benzenesulfonamide

Ce produit a été préparé selon le protocole décrit au paragraphe 2.2 (méthode A) à partir de 2,5 g (15,4 mmol) du composé issu de l'étape précédente et de 3,88 g (15,8 mmol) de chlorure de 2,3-dichlorobenzenesulfonyle. On obtient 2,67 g de produit sous forme d'une poudre beige. Rendement: 57%.
RMN¹H (400MHz ; CDCl3): δ(ppm) :1,2 (s ;12H); 6,75 (d;1 H;7Hz); 6,8 (d;1 H; 12Hz) ; 7,1 (s;1H); 7,25 (t;1H ;7Hz); 7,5 (t ; 1 H ;7Hz) ; 7,55 (d ;1H ;7Hz) ; 7,9 (d ;1H ;7Hz).

### 24.3 : 2-amino-7-(4-{[(2,3-dichlorophenyl)sulfonyl]amino}-2-fluorophenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 2,6 g (5,82 mmol) du composé issu de l'étape précédente et de 1,36 g (4,84 mmol) de chloronaphtyridine issue de l'étape 1.6 .On obtient 1,89 g de produit sous forme de poudre jaune clair. Rendement : 69%. Point de fusion : 340°C.
RMN¹H (200MHZ,DMSO-d₆): δ(ppm) : 1,2 (t ;3H ;7Hz) ; 2,8 (d ;3H ;4,5Hz); 4,5 (q ;2H ;7Hz) ; 7,05 (d;1H;12Hz); 7,1 (d ;1H;7Hz); 7,6 (d ;1H;7Hz); 7,65 (d ;1H;7Hz);7,8-8,2 (m;4H); 8,15 (d ;1H ;7Hz); 8,45 (d ;1H;7Hz) ; 11,0 (q ;1H ;4,5Hz);11,4 (s ;1H) ; 11,7 (brs ;1H).
LCMS : MH+ :564 (tr: 8,18 min ; condition A).

### Exemple 25 : 2-amino-7-(4-{[(2,3-dichlorophenyl)sulfonyl]amino}-3-methylphenyl) -1-ethyl-N-methyl-4-oxo-1,4-dihydro-1,8-naphthyridine-3-carboxamide

### 25.1 : 2-methyl-4-(4,4,5,5-tetramethyl-1,3,2-dioxaborolan-2-yl)aniline

Ce produit a été préparé selon le protocole décrit au paragraphe 2.1 (méthode A) à partir de 5,0 g (26,8 mmol) de 4-bromo-2-methyl aniline et de 7.5 g (29,5 mmol) de bis(pinacolato)diborane. On obtient 0,992 g de produit tutilisé dans l'étape suivante sans purification supplémentaire.
RMN¹H (400MHz ;CDCl3):δ(ppm) :;1,4(s ;12H);2,25(s ;3H) ;3,9(s ;2H) ; 6,7(d;1H;7Hz); 7,55(m ;2H).

### 25.2: 2,3-dichloro-N-[2-methyl-4-(4,4,5,5-tetramethyl-1,3,2-dioxaborolan-2-yl) phenyl]benzenesulfonamide

Ce produit a été préparé selon le protocole décrit au paragraphe 2.2 (méthode A) à partir de 1,35 g (5,79 mmol) du composé issu de l'étape précédente et de 2,13 g (8,67 mmol) de chlorure de 2,3-dichlorobenzenesulfonyle. On obtient 2,1 g de produit sous forme d'une poudre beige. Rendement : 82%.
RMN¹H (400MHz ; CDCl3):δ(ppm) : 1,2 (s ;12H); 3,15 (s ;3H) ; 6,8 (s;1H); 7,1 (d;1 H;7Hz) ; 7,15 (t;1 H ;7Hz); 7,35 (d;1 H ;7Hz); 7,45 (s ;1 H) ; 7,50 (d ;1 H ;7Hz) ; 7,9 (d ;1 H ;7Hz).

### 25.3 : 2-amino-7-(4-{[(2,3-dichlorophenyl)sulfonyl]amino}-3-methylphenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,700 g (1,58 mmol) du composé issu de l'étape précédente et de 0,370 g (1,32 mmol) de chloronaphtyridine issue de l'étape 1.6 .On obtient 0,224 g de produit sous forme de poudre. Rendement : 30%. Point de fusion : 299°C.
RMN¹H (200MHZ, DMSO-d₆): δ(ppm) : 1,25 (t ;3H ;7Hz) ; 2,25 (s ;3H) ; 2,8 (d ;3H ;4,5Hz); 4,55 (q ;2H ;7Hz) ; 7,1 (d;1H;8Hz); 7,5 (t ;1H;8Hz); 7,7-8,1 (m;6H); 8,5 (d ;1H;8Hz); 10,2(s;1H);11,05(q;1H ;4,5Hz); 11,7 (brs ;1H).
LCMS : MH+ :560 (tr : 8,36 min ; condition A).

### Exemple 26: 2-amino-7-(2-chloro-4-{[(2,5-dichlorophenyl)sulfonyl]amino}phenyl) -1-ethyl-N-methyl-4-oxo-1,4-dihydro-1,8-naphthyridine-3-carboxamide

### 26.1 : 2,5-dichloro-N-[3-chloro-4-(4,4,5,5-tetramethyl-1,3,2-dioxaborolan-2-yl) phenyl]benzenesulfonamide

Ce produit a été préparé selon le protocole décrit au paragraphe 2.2 (méthode A) à partir de 0 ,500 g (1,97 mmol) du composé issu de l'étape 12.1 et de 0,484 g (1,97 mmol) de chlorure de 2,5-dichlorobenzenesulfonyle. On obtient 0,704 g de produit sous forme d'une poudre beige. Rendement : 77%.
RMN¹H (400MHz ;CDCl3): δ(ppm) :1,25 (s ;12H); 7,0-7,15 (m ;2H) ; 7,50 (d ;1H ;8Hz); 7,65 (d ;1H ;8Hz); 7,80 (d ;1H ;8Hz); 8,1 (s ;1H); 11,2 (s ;1H).

### 26.2: 2-amino-7-(2-chloro-4-{[(2,5-dichlorophenyl)sulfonyl]amino}phenyl)-1-ethyl-N-methyl-4-oxo-1,4-dihydro-1,8-naphthyridine-3-carboxamide

Ce produit a été préparé selon le protocole décrit au paragraphe 2.3 (méthode A) à partir de 0,703g (1,52 mmol) du composé issu de l'étape précédente et de 0,388 g (1,38 mmol) de chloronaphtyridine issue de l'étape 1.6 .On obtient 0,224 g de produit sous forme de poudre blanche. Rendement : 28%. Point de fusion : 323°C.
RMN¹H (200MHZ, DMSO-d₆):δ (ppm) : 1,2 (t ;3H ;7Hz) ; 2,8 (d ;3H ;4,5Hz); 4,5 (q ;2H ;7Hz) ; 7,1-7,3 (m ;2H); 7,5-7,8 (m;4H;); 8,0 (s;1 H); 8,1(s;1H) ;8,5 (d ;1H;8Hz); 11,0 (q;1H ;4,5Hz); 11,3 (s;1H) ; 11,7 (br s ;1H).
LCMS : MH+ :580 (tr : 5,15 min ; condition A).

Le **tableau 1** qui suit illustre des composés de formule (I) selon l'invention pour lesquels **R1** et **R2** représentent un méthyle (abrégé Me), **n'** représente 1, **R3** représente un atome d'hydrogène et **Ar** représente un phényle, ces composés sont appelés ci-après composés de formule (I').

### Dans ce tableau :

- dans la colonne « Forme », « - » signifie que le composé concerné se trouve sous forme de base libre, alors que « HCl » signifie que le composé concerné se trouve sous forme de sel de chlorhydrate.
- Le signe « ¤ » » signifie que les données ne sont pas disponibles.

**Tableau 1**

| | | | | | | | | | | |
|---|---|---|---|---|---|---|---|---|---|---|
| 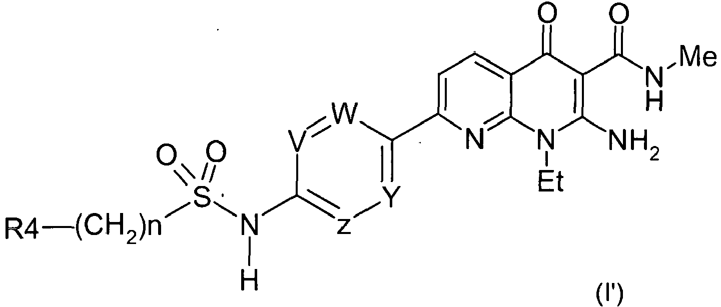 | | | | | | | | | | |

| **N°** | **R4** | **n** | **V** | **W** | **Y** | **Z** | **Forme** | LCMS En tr min (conditions) | **MH+** | **Point de fusion (°C)** |
|---|---|---|---|---|---|---|---|---|---|---|
| **1** | 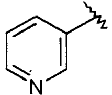 | 1 | CH | CH | CH | C-F | HCl | 5,68 (A) | 511 | 220-223 |
| **2** | 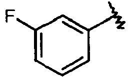 | 0 | CH | CH | CH | C-F | - | 7,38 (B) | 514 | 256 |
| **3** | 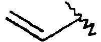 | 0 | CH | CH | CH | C-F | - | 6,75 (A) | 446 | >260 |
| **4** | 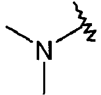 | 2 | CH | CH | CH | C-F | HCl | 4,99 (A) | 491 | 268-270 |
| **5** | 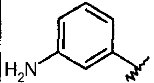 | 1 | CH | CH | CH | C-F | HCl | 6,02 (A) | 525 | >260 |
| **6** | 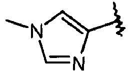 | 0 | CH | CH | CH | C-F | HCl | 6,06 (A) | 500 | >300 |
| **7** | Me | 3 | CH | CH | CH | C-F | - | 7,43 (A) | 476 | 165-167 |
| **8** | 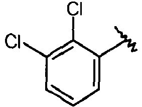 | 0 | CH | CH | CH | C-Cl | - | 8,58 (A) | 582 | 311 |
| **9** | 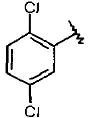 | 0 | CH | CH | CH | C-F | - | 7,32 (A) | 564 | 230 |
| **10** | 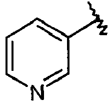 | 0 | CH | CH | CH | CH | - | ¤ | ¤ | ¤ |
| **11** | 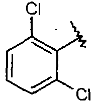 | 0 | CH | CH | CH | C-F | - | 14,33 (A) | 564 | 321 |
| **12** | 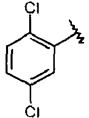 | 0 | CH | CH | C-Cl | CH | - | 5,15 (A) | 580 | 323 |
| **13** | 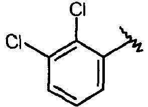 | 0 | CH | CH | C-Cl | CH | - | 7,74 (A) | 580 | 239 |
| **14** | 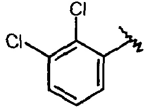 | 0 | CH | CH | C-F | CH | - | 8,18 (A) | 564 | 340 |
| **15** | 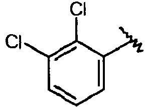 | 0 | CH | CH | CH | C-CH₃ | - | 8,36 (A) | 560 | ¤ |
| **16** | Me | 0 | CH | CH | CH | CH | - | 5,05 (B) | 416 | >300 |
| **17** | 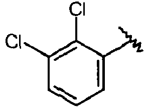 | 0 | CH | CH | CH | CH | - | 6,58 (B) | 546 | 210 |
| **18** | 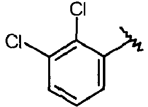 | 0 | CH | CH | CH | C-F | - | 7,54 (A) | 564 | 333 |
| **19** | 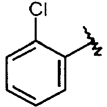 | 0 | CH | CH | CH | CH | - | 6,12 (B) | 512 | >260 |
| **20** | 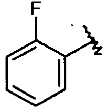 | 0 | CH | CH | CH | C-F | - | 7,25 (B) | 514 | >260 |
| **21** | 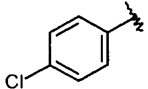 | 0 | CH | CH | CH | C-F | - | 7,91 (A) | 530 | >260 |
| **22** | 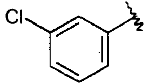 | 0 | CH | CH | CH | C-F | - | 7,69 (B) | 530 | >260 |
| **23** | 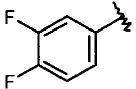 | 0 | CH | CH | CH | C-F | - | 7,55 (A) | 532 | >260 |
| **24** | 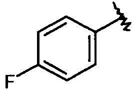 | 0 | CH | CH | CH | C-F | - | 7,47 (B) | 514 | >260 |
| **25** | 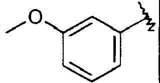 | 0 | CH | CH | CH | C-F | - | 7,56 (B) | 526 | >260 |
| **26** | 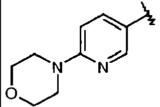 | 0 | CH | CH | CH | C-F | HCl | 7,07 (B) | 582 | >260 |
| **27** | 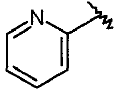 | 1 | CH | CH | CH | C-F | HCl | 6,33 (A) | 511 | 260 |

Les composés selon l'invention ont fait l'objet d'essais pharmacologiques permettant de déterminer leur effet inhibiteur de protéines kinases.
A titre d'exemple, leurs effets inhibiteurs de l'activité sérine/thréonine kinase de p70S6 et/ou tyrosine kinase de PDGF-R ont été mesurés in vitro dans des tests biochimiques.

L'activité inhibitrice vis à vis des kinases des recepteurs PDGF est donnée par la concentration qui inhibe 50% de l'activité de prolifération respectivement des cellules Baf3 tel/PDGF. L'activité inhibitrice vis à vis de la p70S6 kinase est donnée par la concentration qui inhibe 50% de la phosphorylation du substrat peptidique dérivé de la protéine ribosomale S6 (AKRRRLSSLRA, Upstate).

### Mesure de l'inhibition de l'activité tyrosine kinase du récepteur au PDGF beta (PDGF-Rβ) (Baf-3 tel/PDGFRβ) :

Ce test consiste à évaluer les effets des composés sur l'activité tyrosine kinase du récepteur PDGF beta.

L'effet d'inhibition des composés selon l'invention envers l'activité tyrosine kinase du récepteur PDGF-R a été évalué sur la lignée cellulaire murine hématopoïétique BaF/3 transfectée avec un plasmide codant pour la protéine de fusion Tel/PDGF-R beta. Cette protéine de fusion est retrouvée dans les leucémies chroniques myéloïdes myélomonocytaires (CMML). Elle comprend la partie N-Terminale du facteur de transcription Tel et la partie transmembranaire et intracellulaire du récepteur PDGF-R beta. Cette protéine de fusion est présente sous forme dimérisée (présence d'un domaine d'oligomérisation dans la partie N-Terminale de Tel) et de ce fait conduit à l'activité constitutive du domaine kinase de PDGF-R beta. Cette lignée BaF3 Tel/PDGF a été décrite dans la littérature à plusieurs reprises et notamment de façon détaillée dans l'article de M CARROLL et coll, PNAS,1996, 93, 14845-14850, M CARROLL et coll.,Blood 2002, 99, 14845-14850.

Les cellules BaF3 Tel/PDGF sont lavées par du tampon phosphate et ensemencées dans des plaques de 96 puits, à la densité de 5.10⁴ cellules/ml (100 ml par puits), dans du RPMI 1640 contenant 10% de SVF, en présence ou en absence des composés à tester. Après 72 h d'incubation, les cellules viables sont quantifiées par mesure de l'ATP cellulaire grâce à l'utilisation du kit CellTiter-Glo^{®} (Promega, Cat G7571). Les cellules sont traitées selon les instructions données par le fournisseur du kit et la luminescence est mesurée à l'aide d'un Luminoskan (Ascent, Labsystem) avec les paramètres suivants : mesure : unique ; temps d'intégration : 1000 ms, lag time : 5 s. Il apparaît ainsi que les composés selon l'invention ont une activité inhibitrice sur l'activité tyrosine kinase de PDGF-R beta. Cette activité est donnée par la concentration qui inhibe 50% de la prolifération des cellules Baf3 tel/PDGF (Cl₅₀). Les Cl₅₀ des composés selon l'invention sont inférieurs à 10,0 µM.

Par exemple, les composés n° 2, 18, 20 et 24 ont montré une Cl₅₀ de respectivement 36, 12, 280 et 24 nM dans le test de mesure de l'activité inhibitrice de la tyrosine kinase du récepteur PDGF.

### Mesure de l'inhibition de l'activité kinase de p70S6 :

La S6K1 recombinante mutée active (1-421, T412E) (ref. 14-333, Upstate USA, Inc. Charlottesville VA) (activité spécifique 298 U/mg) est incubée (20 mU/ 10 µl) avec 8 concentrations d'inhibiteurs solubilisés à 1 mM en DMSO en présence du substrat peptidique dérivé de la protéine ribosomale S6 (AKRRRLSSLRA, Upstate) (50 µM final) et d'un mélange ATP froid (100µM) et de 1 µCi/puits de [γ-33]ATP (NEN, Courtaboeuf, France). La réaction enzymatique est réalisée dans un volume final de 50 µl dans une plaque filtre 96 puits (plaque opaque MultiScreen TM-PH avec Phospho-Cellulose cat # MAPHNOB, Millipore) préalablement mouillée avec 100 µl tampon Tris 1M pH 7,4 en ajoutant les réactifs du kit S6Kinase Assay (# 17-136, Upstate) dans l'ordre suivant : 10 µl de DMSO à 5% ou des différents inhibiteurs à une concentration 5X 30 µl de mélange réactionnel contenant le tampon ADBI (# 20-108 Upstate, composé de MOPS 20 mM pH 7.2, béta glycérol phosphate 25 mM, EGTA 5 mM, sodium orthovanadate 1 mM, dithiothreitol 1 mM), la S6K1 (20 mU) et le substrat peptidique [AKRRRLSSLRA] 250 µM en tampon ADBI (# 20-122, Upstate). La réaction est déclenchée en ajoutant 10 µl de mélange ATP froid/³³γ ATP (1 µCi/50 µl en ATP 500 µM dans le tampon ADBI, MgCl₂ 75 mM) puis incubée 20 minutes à 30°C avant d'être stoppée par ajout de 20 µl d'acide phosphorique 7.5%, Le mélange réactionnel est filtré par aspiration sous vide (Vacuum manifold, Millipore), les puits sont rincés 2 fois avec 200 µl d'acide Phosphorique 7.5 % (2 minutes) puis 2 fois avec 200 µl d'H₂O distillée (2 minutes). Après séchage de la plaque, 25 µl/puits de scintillant (Optiphase Super Mix, Wallac) sont ajoutés et la radioactivité détectée par le lecteur à scintillation Micro-Béta (Wallac). Un contrôle négatif (tous les réactifs sans substrat peptidique) est réalisé pour déterminer la liaison non spécifique de ³³γ ATP au filtre phospho-cellulose qui est soustrait des résultats expérimentaux.

Il apparaît ainsi que les composés selon l'invention ont une activité inhibitrice sur l'activité kinase de p70S6. Cette activité est donnée par la concentration qui inhibe 50% de la phosphorylation du substrat peptidique dérivé de la protéine ribosomale S6 (AKRRRLSSLRA, Upstate). Les Cl₅₀ des composés selon l'invention sont inférieures à 10,0 µM.
Par exemple, les composés n° 8, 9,14 et 18 ont montré une Cl₅₀ de respectivement 412, 240, 224 et 132 nM dans le test de mesure de l'activité inhibitrice de la p70S6 kinase.

Les composés selon l'invention sont donc des inhibiteurs de protéines kinases, notamment des récepteurs tyrosine kinase PDGF et, pour certains d'entre eux, également de la p70S6 kinase.

Les composés selon l'invention peuvent donc être utilisés pour la préparation de médicaments destinés au traitement et/ou à la prévention des maladies liées à l'activté des proteines kinases, en particulier de médicaments inhibiteurs de protéine kinases.

Il s'agit de médicaments inhibiteurs de protéine kinase, notamment de médicaments inhibiteurs du recepteur PDGF-R tyrosine kinase et éventuellement de la p70S6 kinase.

Ainsi, selon un autre de ses aspects, l'invention a pour objet des médicaments qui comprennent un composé de formule (I), ou un sel d'addition de ce dernier à un acide pharmaceutiquement acceptable, ou encore un un solvat du composé de formule (I).

Ces médicaments trouvent leur emploi en thérapeutique, notamment dans le traitement et/ou la prévention des maladies liées à l'activité des protéines kinases et notamment des maladies prolifératives telles que les cancers par exemple les cancers du poumon (NSCLC), des os, du pancréas, de la peau, syndrome de Kaposi, les mélanomes intraoculaires, les cancers du sein, de l'utérus, du col de l'utérus, des ovaires, de l'endomètre, du vagin, de la vulve, de l'urètre, du pénis, de la prostate, les carcinomes des trompes de Fallope, les cancers tel que les GIST et de la région anale, du rectum, de l'intestin grêle, du colon, de l'estomac, de l'oesophage, des glandes endocrines, thyroïdes, parathyroïdes ou surrénales, les sarcomes des tissus mous, sarcomes d'Ewing, des ostésarcomes, dermatofibrosarcome et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases, les leucémies chroniques ou aigues, les lymphomes lymphocitaires, la maladie de Hodgkin et les syndromes myeloproliferatifs, et les syndromes myelodysplasiques.

Un autre aspect de l'invention comprend une association entre au moins un composé selon l'invention et au moins un agent de chimiothérapie.

En effet, les composés de la présente invention peuvent être utilisés seuls ou en mélange avec au moins un agent de chimiothérapie pouvant être choisi parmi des agents cytotoxiques et/ou des antiangiogènes. Par exemple, les agents antiangiogènes peuvent être un composé inhibiteur de l'activité kinase de VEGF-R ou un composé antagoniste d'un facteur de croissance.

Il est également possible de combiner les composés selon l'invention avec un traitement par radiations.

Les combinaisons des composés de l'invention avec les agents de chimiothérapie cités ci-dessus et/ou les radiations sont un autre objet de la présente invention.

Les agents de chimiothérapie cités ci-dessus et/ou les radiations peuvent être administrés simultanément, séparément ou séquentiellement. Le traitement sera adapté par le praticien en fonction du malade à traiter.

Ces médicaments trouvent également leur emploi en thérapeutique, dans des maladies prolifératives non-malignes, comme par exemple la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression (rejet de greffes par exemple).

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable de ce dernier, ou encore un un solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, intratrachéale, intranasale, transdermique ou rectale, le principe actif de formule (I) ci-dessus, ou son sel ou solvat éventuel, peut être administré sous forme unitaire d'administration, en mélange avec des excipients pharmaceutiques classiques, aux animaux et aux êtres humains pour la prophylaxie ou le traitement et/ou à la prévention des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également l'utilisation d'un composé de formule (I) pour la préparation d'un médicament destiné au traitement et/ou à la prévention des maladies liées à l'activité des protéines kinases, au traitement et/ou à la prévention des maladies prolifératives telles que les cancers, les leucémies chroniques ou aiguës, les lymphomes lymphocytaires, la maladie de Hodgkin, et les syndromes myeloprolifératifs, et les syndromes myelodysplasiques, au traitement et/ou à la prévention des maladies prolifératives telles que les cancers à tumeurs solides par exemple cancers du poumon (NSCLC), des os, du pancréas, de la peau, syndrome de Kaposi, les mélanomes intraoculaires, les cancers du sein, de l'utérus, du col de l'utérus, des ovaires, de l'endomètre, du vagin, de la vulve, de l'urètre, du pénis, de la prostate, les carcinomes des trompes de Fallope, les cancers tel que les GIST et de la région anale, du rectum, de l'intestin grêle, du colon, de l'estomac, de l'oesophage, des glandes endocrines, thyroïdes, parathyroïdes ou surrénales, les sarcomes des tissus mous, sarcomes d'Ewing, des ostésarcomes, dermatofibrosarcome et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases, les leucémies chroniques ou aigues, les lymphomes lymphocitaires, la maladie de Hodgkin et les syndromes myeloproliferatifs, et les syndromes myelodysplasiques, ou au traitement et/ou à la prévention de maladies prolifératives non-malignes telles que la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression.

## Revendications

1. Composé répondant à la formule (I) : 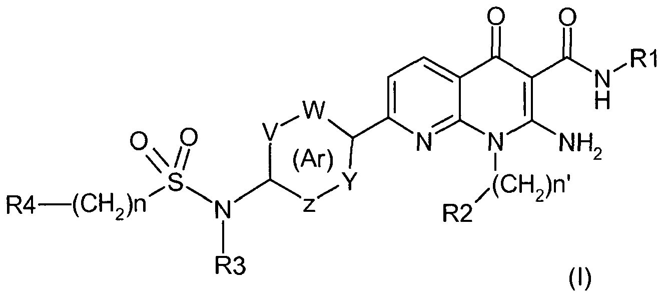 dans laquelle
• **n** représente 0, 1, 2 ou 3 ;
• **n'** représente 0, 1, 2, 3 ou 4 ;
• **R1** représente un groupe alkyle;
• **R2** représente :
(i) un groupe cycloalkyle,
(ii) un groupe alkyle, ou
(iii) un groupe alcoxy,
lesdits groupes cycloalkyle, alkyle ou alcoxy étant éventuellement substitués par un ou plusieurs atomes d'halogène;
• **R3** représente :
i) un atome d'hydrogène, ou
ii) un groupe -C(O)alkyle;
• **Ar** représente un cycle aryle ou hétéroaryle comprenant 5 ou 6 chaînons dans lequel **Y, Z, V et W:**
(a) représentent, indépendamment les uns des autres,
(i) un groupe =CH-,
(ii) un groupé =C(R5)- dans lequel **R5** représente :
○ un groupe alkyle,
○ un atome d'halogène, ou
○ un groupe alcoxy,
(iii) un hétéroatome choisi parmi l'atome d'azote, l'atome de soufre et l'atome d'oxygène,
(b) l'un au plus parmi **Y, Z, V et W** étant éventuellement absent,
étant entendu que, lorsque **Ar** représente un hétéroaryle choisi parmi le pyrrolyle, l'imidazolyle, le pyrazolyle et les triazolyles, au moins l'un des atomes d'azote dudit hétéroaryle peut être éventuellement substitué par un groupe **R6** choisi parmi un groupe alkyle,
• **R4** représente un groupe choisi parmi:
o un groupe alkyle,
o un groupe alcoxyalkyle,
o un groupe -NRR' avec **R et R'**, pouvant être identiques ou différents, et représentant, indépendamment l'un de l'autre, un atome d'hydrogène, un groupe alkyle ou un groupe -(C3-C6)cycloalkyle,
o un groupe cycloalkyle,
o un groupe alkenyle,
o un groupe aryle, ledit groupe étant éventuellement substitué par au moins un atome d'halogène, et/ou par au moins un groupe choisi parmi les groupes - (C1-C5)alkyle, halogenoalkyle, nitrile, halogenoalkyloxy, alcoxy, nitro et les groupes -NRR' avec **R et R'**, pouvant être identiques ou différents, et représentant, indépendamment l'un de l'autre, un atome d'hydrogène ou un groupe choisi parmi les groupes alkyle et les groupes -(C3-C6)cycloalkyle,
o un groupe hétéroaryle, ledit groupe comprenant au moins un hétéroatome choisi parmi l'atome d'azote ou de soufre, lesdits groupes hétéroaryles étant éventuellement substitués par au moins un groupe choisi parmi les groupes alkyle et les groupes hétérocycloalkyle comprenant au moins un hétéroatome choisi parmi l'atome d'azote et d'oxygène;
étant entendu que, lorsque le groupe hétéroaryle est choisi parmi le pyrrolyle, l'imidazolyle, le pyrazolyle et les triazolyles, au moins l'un des atomes d'azote dudit hétéroaryle peut être éventuellement substitué par un groupe **R6** choisi parmi un groupe alkyle,
o un groupe hétérocycloalkyles comprenant au moins un hétéroatome choisi parmi l'atome d'azote, de soufre et d'oxygène et étant éventuellement substitué par au moins un substituant choisi parmi (i) les atomes d'halogène, (ii) les groupes halogénoalkyles, (iii) les groupes alkyles, linéaires ou ramifiés et (iv) les groupes cycloalkyles,
étant entendu que, lorsque les groupes hétérocycloalkyles sont choisis parmi le pyrrolinyle, pyrrolidinyle, imidazolidinyle, imidazolinyle, pyrazolinyle, pyrazolidinyle, piperidinyle, morpholinyle, piperazinyle et thiomorpholinyle, au moins l'un des atomes d'azote dudit hétérocycloalkyle peut être éventuellement substitué par un groupe **R6** choisi parmi un groupe alkyle,
sous forme d'acide, de base ou de sel d'addition à un acide ou à une base.

2. Composé de formule (I) selon la revendication 1, **caractérisé en ce que** :
**R1** représente un groupe -(C1-C4)alkyle,
lesdits composés sont sous forme de base ou de sels d'addition à un acide.

3. Composé de formule (I) selon l'une des revendications 1 ou 2, **caractérisé en ce que** :
**R2** représente un groupe un groupe -(C1-C4)alkyle,
lesdits composés sont sous forme de base ou de sels d'addition à un acide.

4. Composé de formule (I) selon l'une quelconques des revendications 1 à 3, **caractérisé en ce que** :
**n'** représente 1,
lesdits composés sont sous forme de base ou de sels d'addition à un acide.

5. Composé de formule (I) selon l'une quelconques des revendications 1 à 4, **caractérisé en ce que** :
**R3** représente un atome d'hydrogène,
lesdits composés sont sous forme de base ou de sels d'addition à un acide.

6. Composé de formule (I) selon l'une quelconques des revendications 1 à 5, **caractérisé en ce que** :
**Ar** représente un phényle,
lesdits composés sont sous forme de base ou de sels d'addition à un acide.

7. Composé de formule (I) selon l'une quelconque des revendications 1 à 6, **caractérisé en ce que** :
**R4** représente un groupe choisi parmi:
○ un groupe alkyle ;
○ un groupe -NRR', avec **R et R'**, pouvant être identiques ou différents, et représentant, indépendamment l'un de l'autre, un atome d'hydrogène, un groupe alkyle ou un groupe -(C3-C6)cycloalkyle,
○ un groupe alcényle,
○ un groupe aryle, ledit groupe étant éventuellement substitués par au moins un atome d'halogène, et/ou par au moins un groupe choisi parmi les groupes alcoxy et les groupes -NRR', avec **R et R'** tels que définis ci-dessus,
o un groupe hétéroaryle, ledit groupe hétéroaryle étant éventuellement substitués par au moins un groupe choisi parmi les groupes alkyles et les groupes hétérocycloalkyles comprenant au moins un hétéroatome choisi parmi l'atome d'azote et d'oxygène;
étant entendu que, lorsque lesdit groupe hétéroaryle est choisi parmi le pyrrolyle, l'imidazolyle, le pyrazolyle et les triazolyles, au moins l'un des atomes d'azote dudit hétéroaryle peut être éventuellement substitué par un groupe **R6**, avec **R6** représentant un groupe choisi parmi un groupe alkyle.

8. Composé de formule (I) selon l'une quelconque des revendications 1 à 7, **caractérisé en ce que R4** représente un groupe choisi parmi les groupes phényle, pyridinyle et imidazolyle.

9. Composé de formule (I) selon l'une quelconque des revendications 1 à 8, **caractérisé en ce que Y, Z, V et W** représentent chacun un groupe =CH et/ou un groupe =C(R5)-avec **R5** représentant un atome de chlore ou de fluor, **Y, Z, V et W** étant ainsi compris dans un groupe phényle éventuellement substitué.

10. Composé de formule (I) selon l'une quelconque des revendications 1 à 9, choisi parmi les composés suivants :
2-amino-1-ethyl-7-(3-fluoro-4-{[(pyridin-3-ylmethyl)sulfonyl]amino}phenyl)-N-methyl-4-oxo-1,4-dihydro-1,8-naphthyridine-3-carboxamide;
2-amino-1-ethyl-7-(3-fluoro-4-{[(3-fluorophenyl)sulfonyl]amino}phenyl)-N-methyl-4-oxo-1,4-dihydro-1,8-naphthyridine-3-carboxamide ;
2-amino-7-{4-[(ethenylsulfonyl)amino]-3-fluorophenyl}-1-ethyl-N-methyl-4-oxo-1,4-dihydro-1,8-naphthyridine-3-carboxamide ;
2-amino-7-[4-({[2-(dimethylamino)ethyl]sulfonyl}amino)-3-fluorophenyl]-1-ethyl-N-methyl-4-oxo-1,4-dihydro-1,8-naphthyridine-3-carboxamide;
2-amino-7-(4-{[(3-aminobenzyl)sulfonyl]amino}-3-fluorophenyl)-1-ethyl-N-methyl-4-oxo-1,4-dihydro-1,8-naphthyridine-3-carboxamide;
2-amino-1-éthyl-7-(3-fluoro-4-{[(1-méthyl-1H-imidazol-4-yl)sulfonyl]amino}phényl)-N-méthyl-4-oxo-1,4-dihydro-1,8-naphtyridine-3-carboxamide;
2-amino-7-{4-[(butylsulfonyl)amino]-3-fluorophenyl}-1-ethyl-N-methyl-4-oxo-1,4-dihydro-1,8-naphthyridine-3-carboxamide;
2-amino-7-(3-chloro-4-{[(2,3-dichlorophenyl)sulfonyl]amino}phenyl)-1-ethyl-N-methyl-4-oxo-1,4-dihydro-1,8-naphthyridine-3-carboxamide ;
2-amino-7-(4-{[(2,5-dichlorophenyl)sulfonyl]amino}-3-fluorophenyl)-1-ethyl-N-methyl-4-oxo-1,4-dihydro-1,8-naphthyridine-3-carboxamide;
2-amino-1-éthyl-N-méthyl-4-oxo-7-{4-[(pyridin-3-ylsulfonyl)amino]phényl}-1,4-dihydro-1,8-naphtyridine-3-carboxamide;
2-amino-7-(4-{[(2,6-dichlorophenyl)sulfonyl]amino}-3-fluorophenyl)-1-ethyl-N-methyl-4-oxo-1,4-dihydro-1,8-naphthyridine-3-carboxamide;
2-amino-7-(2-chloro-4-{[(2,5-dichlorophenyl)sulfonyl]amino}phenyl)-1-ethyl-N-methyl-4-oxo-1,4-dihydro-1,8-naphthyridine-3-carboxamide;
2-amino-7-(2-chloro-4-{[(2,3-dichlorophenyl)sulfonyl]amino}phényl)-1-ethyl-N-methyl-4-oxo-1,4-dihydro-1,8-naphthyridine-3-carboxamide;
2-amino-7-(4-{[(2,3-dichlorophenyl)sulfonyl]amino}-2-fluorophenyl)-1-ethyl-N-methyl-4-oxo-1,4-dihydro-1,8-naphthyridine-3-carboxamide;
2-amino-7-(4-{[(2,3-dichlorophenyl)sulfonyl]amino}-3-methylphenyl)-1-ethyl-N-methyl-4-oxo-1,4-dihydro-1,8-naphthyridine-3-carboxamide;
2-amino-1-ethyl-N-methyl-7-{4-[(methylsulfonyl)amino]phenyl}-4-oxo-1,4-dihydro-1,8-naphthyridine-3-carboxamide;
2-amino-7-(4-{[(2,3-dichlorophenyl)sulfonyl]amino}phenyl)-1-ethyl-N-methyl-4-oxo-1,4-dihydro-1,8-naphthyridine-3-carboxamide;
2-amino-7-(4-{[(2,3-dichlorophenyl)sulfonyl]amino}-3-fluorophenyl)-1-ethyl-N-methyl-4-oxo-1,4-dihydro-1,8-naphthyridine-3-carboxamide;
2-amino-7-(4-{[(2-chlorophenyl)sulfonyl]amino}phenyl)-1-ethyl-N-methyl-4-oxo-1,4-dihydro-1,8-naphthyridine-3-carboxamide;
2-amino-1-ethyl-7-(3-fluoro-4-{[(2-fluorophenyl)sulfonyl]amino}phenyl)-N-methyl-4-oxo-1,4-dihydro-1,8-naphthyridine-3-carboxamide;
2-amino-7-(4-{[(4-chlorophenyl)sulfonyl]amino}-3-fluorophenyl)-1-ethyl-N-methyl-4-oxo-1,4-dihydro-1,8-naphthyridine-3-carboxamide;
2-amino-7-(4-{[(3-chlorophenyl)sulfonyl]amino}-3-fluorophenyl)-1-ethyl-N-methyl-4-oxo-1,4-dihydro-1,8-naphthyridine-3-carboxamide;
2-amino-7-(4-{[(3,4-difluorophenyl)sulfonyl]amino}-3-fluorophenyl)-1-ethyl-N-methyl-4-oxo-1,4-dihydro-1,8-naphthyridine-3-carboxamide;
2-amino-1-ethyl-7-(3-fluoro-4-{[(4-fluorophenyl)sulfonyllamino}phenyl)-N-methyl-4-oxo-1,4-dihydro-1,8-naphthyridine-3-carboxamide;
2-amino-1-ethyl-7-(3-fluoro-4-{[(3-methoxyphenyl)sulfonyl]amino}phenyl)-N-methyl-4-oxo-1,4-dihydro-1,8-naphthyridine-3-carboxamide;
2-amino-1-ethyl-7-[3-fluoro-4-({[6-(morpholin-4-yl)pyridin-3-yl]sulfonyl}amino)phenyl]-N-methyl-4-oxo-1,4-dihydro-1,8-naphthyridine-3-carboxamide;
2-amino-1-ethyl-7-(3-fluoro-4-{[(pyridin-2-ylmethyl)sulfonyl]amino}phenyl)-N-methyl-4-oxo-1,4-dihydro-1,8-naphthyridine-3-carboxamide;

11. Procédé de préparation d'un composé de formule (I) selon l'une quelconque des revendications 1 à 10, **caractérisé en ce que** l'on fait réagir un composé de formule (IXa) : 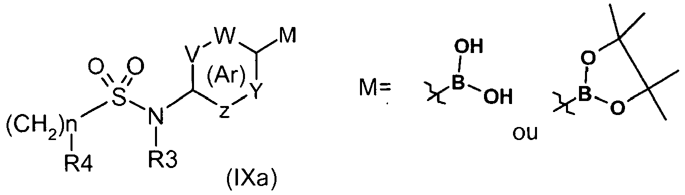 avec un composé de formule (VII), en présence d'un catalyseur de couplage et d'une base, 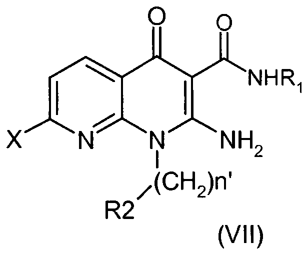 où **R1, R2, R3, R4, n, n', V, W, Y, Z et Ar** sont tels que définis dans la revendication 1, **X** représente un groupe partant et **M** est tel que défini ci-dessus.

12. Procédé de préparation d'un composé de formule (I) selon l'une quelconque des revendications 1 à 10, **caractérisé en ce que** l'on fait réagir un composé de formule (IXb) 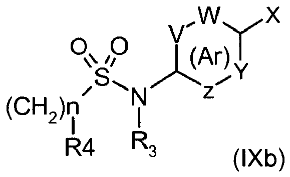 avec un composé de formule (VIII), 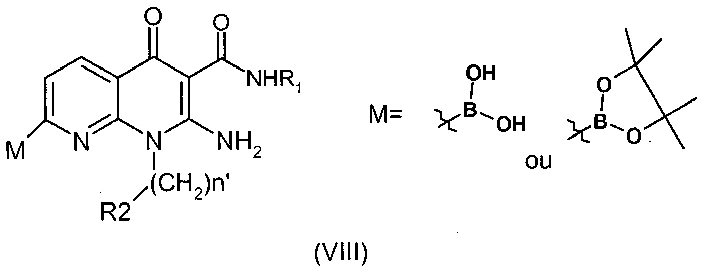 où R1, R2, R3, R4, n, n', V, W, Y, Z et Ar sont tels que définis dans la revendication 1, X représente un groupe partant et M est tel que défini ci-dessus.

13. Médicament, **caractérisé en ce qu'**il comprend un composé de formule (I) selon l'une quelconque des revendications 1 à 10, ou un sel d'addition de ce composé à un acide pharmaceutiquement acceptable, ou encore un un solvat du composé de formule (I).

14. Composition pharmaceutique, **caractérisée en ce qu'**elle comprend un composé de formule (I) selon l'une quelconque des revendications 1 à 10, ou un sel pharmaceutiquement acceptable, ou un solvat de ce composé, ainsi qu'au moins un excipient pharmaceutiquement acceptable.

15. Composé de formule (I) selon l'une quelconque des revendications 1 à 10 pour son utilisation dans le traitement et/ou la prévention des maladies prolifératives telles que les cancers, les leucémies chroniques ou aiguës, les lymphomes lymphocytaires, la maladie de Hodgkin, et les syndromes myeloprolifératifs, et les syndromes myelodysplasiques.

16. Composé de formule (I) selon l'une quelconque des revendications 1 à 10 pour son utilisation dans le traitement et/ou la prévention des maladies prolifératives telles que les cancers à tumeurs solides par exemple cancers du poumon (NSCLC), des os, du pancréas, de la peau, syndrome de Kaposi, les mélanomes intraoculaires, les cancers du sein, de l'utérus, du col de l'utérus, des ovaires, de l'endomètre, du vagin, de la vulve, de l'urètre, du pénis, de la prostate, les carcinomes des trompes de Fallope, les cancers tel que les GIST et de la région anale, du rectum, de l'intestin grêle, du colon, de l'estomac, de l'oesophage, des glandes endocrines, thyroïdes, parathyroïdes ou surrénales, les sarcomes des tissus mous, sarcomes d'Ewing, des ostésarcomes, dermatofibrosarcome et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases, les leucémies chroniques ou aigues, les lymphomes lymphocitaires, la maladie de Hodgkin et les syndromes myeloproliferatifs, et les syndromes myelodysplasiques.

17. Composé de formule (I) selon l'une quelconque des revendications 1 à 10 pour son utilisation dans le traitement et/ou la prévention de maladies prolifératives non-malignes telles que la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression.

18. Association d'au moins un composé de formule (I) selon l'une quelconque des revendications 1 à 10 avec au moins un agent de chimiothérapie.

## Patentansprüche

1. Verbindung der Formel (I): 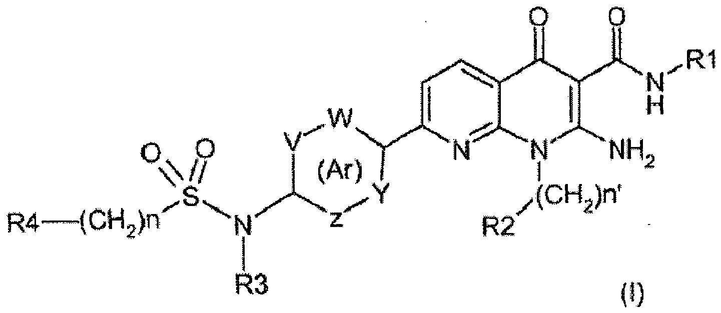 worin
• **n** für 0, 1, 2 oder 3 steht;
• **n'** für 0, 1, 2, 3 oder 4 steht;
• **R1** für eine Alkylgruppe steht;
• **R2** für:
(i) eine Cycloalkylgruppe,
(ii) eine Alkylgruppe oder
(iii)eine Alkoxygruppe
steht, wobei die Cycloalkyl-, Alkyl- oder Alkoxygruppen gegebenenfalls durch ein oder mehrere Halogenatome substituiert sind;
• **R3** für:
(i) ein Wasserstoffatom oder
(ii) eine -C(O)-Alkylgruppe
steht;
• **Ar** für einen 5- oder 6-gliedrigen Aryl- oder Heteroarylring stehen, wobei **Y, Z, V** und **W:**
(a) unabhängig voneinander für
(i) eine =CH-Gruppe,
(ii) eine =C(R5)-Gruppe, worin **R5** für:
o eine Alkylgruppe,
o ein Halogenatom oder
o eine Alkoxygruppe
steht,
(iii) ein aus dem Stickstoffatom, dem Schwefelatom oder dem Sauerstoffatom ausgewähltes Heteroatom
stehen,
(b) wobei höchstens eine der Variablen **Y, Z, V** und **W** fehlen kann,
mit der Maßgabe, dass dann, wenn **Ar** für ein aus Pyrrolyl, Imidazolyl, Pyrazolyl und Triazolylgruppen ausgewähltes Heteroaryl steht, mindestens eines der Stickstoffatome des Heteroaryls gegebenenfalls durch eine aus einer Alkylgruppe ausgewählte Gruppe **R6** substituiert sein kann,
• **R4** für eine Gruppe steht, die ausgewählt ist aus:
o einer Alkylgruppe,
o einer Alkoxyalkylgruppe,
o einer -NRR`-Gruppe, wobei **R** und **R'** gleich oder verschieden sein können und unabhängig voneinander für ein Wasserstoffatom, eine Alkylgruppe oder eine -(C3-C6)-Cycloalkylgruppe stehen,
○ einer Cycloalkylgruppe,
○ einer Alkenylgruppe,
○ einer Arylgruppe, wobei die Gruppe gegebenenfalls durch mindestens ein Halogenatom und/oder durch mindestens eine aus -(C1-C5)-Alkyl-, Halogenalkyl-, Nitril-, Halogenalkyloxy-, Alkoxy-, Nitro- und -NRR'-Gruppen ausgewählte Gruppe substituiert ist, wobei **R** und **R'** gleich oder verschieden sein können und unabhängig voneinander für ein Wasserstoffatom oder eine aus Alkylgruppen und -(C3-C6)-Cycloalkylgruppen ausgewählte Gruppe stehen,
○ einer Heteroarylgruppe, wobei die Gruppe mindestens ein aus dem Stickstoff- oder Schwefelatom ausgewähltes Heteroatom umfasst, wobei die Heteroarylgruppen gegebenenfalls durch mindestens eine aus Alkylgruppen und Heterocycloalkylgruppen mit mindestens einem aus dem Stickstoff- und Sauerstoffatom ausgewählten Heteroatom ausgewählte Gruppe substituiert sind;
mit der Maßgabe, dass dann, wenn die Heteroarylgruppe aus Pyrrolyl, Imidazolyl, Pyrazolyl und Triazolylgruppen ausgewählt ist, mindestens eines der Stickstoffatome des Heteroaryls gegebenenfalls durch eine aus einer Alkylgruppe ausgewählte Gruppe **R6** substituiert sein kann,
o einer Heterocycloalkylgruppe, die mindestens ein aus dem Stickstoff-, Schwefel- und Sauerstoffatom ausgewähltes Heteroatom umfasst und gegebenenfalls durch mindestens einen aus (i) Halogenatomen, (ii) Halogenalkylgruppen, (iii) linearen oder verzweigten Alkylgruppen und (iv) Cycloalkylgruppen ausgewählten Substituenten substituiert ist,
mit der Maßgabe, dass dann, wenn die Heterocycloalkylgruppen aus Pyrrolinyl, Pyrrolidinyl, Imidazolidinyl, Imidazolinyl, Pyrazolinyl, Pyrazolidinyl, Piperidinyl, Morpholinyl, Piperazinyl und Thiomorpholinyl ausgewählt sind, mindestens eines der Stickstoffatome des Heterocycloalkyls gegebenenfalls durch eine aus einer Alkylgruppe ausgewählte Gruppe **R6** substituiert sein kann,
in Säuren-, Basen- oder Säureadditionssalz- oder Basenadditionssalzform.

2. Verbindung der Formel (I) nach Anspruch 1, **dadurch gekennzeichnet, dass**:
**R1** für eine -(C1-C4)-Alkylgruppe steht,
wobei die Verbindungen in Basen- oder Säureadditionssalzform vorliegen.

3. Verbindung der Formel (I) nach einem der Ansprüche 1 oder 2, **dadurch gekennzeichnet, dass**:
**R2** für eine -(C1-C4)-Alkylgruppe steht,
wobei die Verbindungen in Basen- oder Säureadditionssalzform vorliegen.

4. Verbindung der Formel (I) nach einem der Ansprüche 1 bis 3, **dadurch gekennzeichnet, dass**:
**n'** für 1 steht,
wobei die Verbindungen in Basen- oder Säureadditionssalzform vorliegen.

5. Verbindungen der Formel (I) nach einem der Ansprüche 1 bis 4, **dadurch gekennzeichnet, dass**:
**R3** für ein Wasserstoffatom steht,
wobei die Verbindungen in Basen- oder Säureadditionssalzform vorliegen.

6. Verbindung der Formel (I) nach einem der Ansprüche 1 bis 5, **dadurch gekennzeichnet, dass**:
**Ar** für ein Phenyl steht,
wobei die Verbindungen in Basen- oder Säureadditionssalzform vorliegen.

7. Verbindung der Formel (I) nach einem der Ansprüche 1 bis 6, **dadurch gekennzeichnet, dass**:
**R4** für eine Gruppe steht, die ausgewählt ist aus:
○ einer Alkylgruppe;
○ einer -NRR`-Gruppe, wobei **R** und **R'** gleich ○ der verschieden sein können und unabhängig voneinander für ein Wasserstoffatom, eine Alkylgruppe oder eine -(C3-C6)-Cycloalkylgruppe stehen,
o einer Alkenylgruppe,
o einer Arylgruppe, wobei die Gruppe gegebenenfalls durch mindestens ein Halogenatom und/oder durch mindestens eine aus Alkoxy- und -NRR'-Gruppen ausgewählte Gruppe substituiert ist, wobei **R** und **R'** wie oben definiert sind,
o einer Heteroarylgruppe, wobei die Heteroarylgruppe gegebenenfalls durch mindestens eine aus Alkylgruppen und Heterocycloalkylgruppen mit mindestens einem aus dem Stickstoff- und Sauerstoffatom ausgewählten Heteroatom ausgewählte Gruppe substituiert ist;
mit der Maßgabe, dass dann, wenn die Heteroarylgruppe aus Pyrrolyl, Imidazolyl, Pyrazolyl und Triazolylgruppen ausgewählt ist, mindestens eines der Stickstoffatome des Heteroaryls gegebenenfalls durch eine Gruppe **R6** substituiert sein kann, wobei **R6** für eine aus einer Alkylgruppe ausgewählte Gruppe steht.

8. Verbindung der Formel (I) nach einem der Ansprüche 1 bis 7, **dadurch gekennzeichnet, dass R4** für eine aus Phenyl-, Pyridinyl- und Imidazolylgruppen ausgewählte Gruppe steht.

9. Verbindung der Formel (I) nach einem der Ansprüche 1 bis 8, **dadurch gekennzeichnet, dass Y, Z, V** und **W** jeweils für eine =CH-Gruppe und/oder eine =C(R5)-Gruppe stehen, wobei **R5** für ein Chlor- oder Fluoratom steht, wobei **Y, Z, V** und **W** somit in einer gegebenenfalls substituierten Phenylgruppe enthalten sind.

10. Verbindung der Formel (I) nach einem der Ansprüche 1 bis 9, ausgewählt aus den folgenden Verbindungen:
2-Amino-1-ethyl-7-(3-fluor-4-{[(pyridin-3-ylmethyl)sulfonyl]amino}phenyl)-N-methyl-4-oxo-1,4-dihydro-1,8-naphthyridin-3-carboxamid;
2-Amino-1-ethyl-7-(3-fluor-4-{[(3-fluorphenyl)sulfonyl]amino}phenyl)-N-methyl-4-oxo-1,4-dihydro-1,8-naphthyridin-3-carboxamid;
2-Amino-7-{4-[(ethenylsulfonyl)amino]-3-fluorphenyl}-1-ethyl-N-methyl-4-oxo-1,4-dihydro-1,8-naphthyridin-3-carboxamid;
2-Amino-7-[4-({[2-(dimethylamino)ethyl]sulfonyl}amino)-3-fluorphenyl]-1-ethyl-N-methyl-4-oxo-1,4-dihydro-1,8-naphthyridin-3-carboxamid;
2-Amino-7-(4-{[(3-aminobenzyl)sulfonyl]amino}-3-fluorphenyl)-1-ethyl-N-methyl-4-oxo-1,4-dihydro-1,8-naphthyridin-3-carboxamid;
2-Amino-1-ethyl-7-(3-fluor-4-{[(1-methyl-1H-imidazol-4-yl)sulfonyl]amino}phenyl)-N-methyl-4-oxo-1,4-dihydro-1,8-naphthyridin-3-carboxamid;
2-Amino-7-{4-[(butylsulfonyl)amino]-3-fluorphenyl}-1-ethyl-N-methyl-4-oxo-1,4-dihydro-1,8-naphthyridin-3-carboxamid;
2-Amino-7-(3-chlor-4{[(2,3-dichlorphenyl)sulfonyl]amino}phenyl)-1-ethyl-N-methyl-4-oxo-1,4-dihydro-1,8-naphthyridin-3-carboxamid;
2-Amino-7-(4-{[(2,5-dichlorphenyl)sulfonyl]amino}-3-fluorphenyl)-1-ethyl-N-methyl-4-oxo-1,4-dihydro-1,8-naphthyridin-3-carboxamid;
2-Amino-1-ethyl-N-methyl-4-oxo-7-{4-[(pyridin-3-ylsulfonyl)amino]phenyl}-1,4-dihydro-1,8-naphthyridin-3-carboxamid;
2-Amino-7-(4-{[(2,6-dichlorphenyl)sulfonyl]amino}-3-fluorphenyl)-1-ethyl-N-methyl-4-oxo-1,4-dihydro-1,8-naphthyridin-3-carboxamid;
2-Amino-7-(2-chlor-4-{[(2,5-dichlorphenyl)sulfonyl]amino}phenyl)-1-ethyl-N-methyl-4-oxo-1,4-dihydro-1,8-naphthyridin-3-carboxamid;
2-Amino-7-(2-chlor-4-{[(2,3-dichlorphenyl)sulfonyl]amino}phenyl)-1-ethyl-N-methyl-4-oxo-1,4-dihydro-1,8-naphthyridin-3-carboxamid;
2-Amino-7-(4-{[(2,3-dichlorphenyl)sulfonyl]amino}-2-fluorphenyl)-1-ethyl-N-methyl-4-oxo-1,4-dihydro-1,8-naphthyridin-3-carboxamid;
2-Amino-7-(4-{[(2,3-dichlorphenyl)sulfonyl]amino}-3-methylphenyl)-1-ethyl-N-methyl-4-oxo-1,4-dihydro-1,8-naphthyridin-3-carboxamid;
2-Amino-1-ethyl-N-methyl-7-{4-[(methylsulfonyl)amino]phenyl}-4-oxo-1,4-dihydro-1,8-naphthyridin-3-carboxamid;
2-Amino-7-(4-{[(2,3-dichlorphenyl)sulfonyl]amino}phenyl)-1-ethyl-N-methyl-4-oxo-1,4-dihydro-1,8-naphthyridin-3-carboxamid;
2-Amino-7-(4-{[(2,3-dichlorphenyl)sulfonyl]amino}-3-fluorphenyl)-1-ethyl-N-methyl-4-oxo-1,4-dihydro-1,8-naphthyridin-3-carboxamid;
2-Amino-7-(4-{[(2-chlorphenyl)sulfonyl]amino}phenyl)-1-ethyl-N-methyl-4-oxo-1,4-dihydro-1,8-naphthyridin-3-carboxamid;
2-Amino-1-ethyl-7-(3-fluor-4-{[(2-fluorphenyl)sulfonyl]amino}phenyl)-N-methyl-4-oxo-1,4-dihydro-1,8-naphthyridin-3-carboxamid;
2-Amino-7-(4-{[(4-chlorphenyl)sulfonyl]amino}-3-fluorphenyl)-1-ethyl-N-methyl-4-oxo-1,4-dihydro-1,8-naphthyridin-3-carboxamid;
2-Amino-7-(4-{[(3-chlorphenyl)sulfonyl]amino}-3-fluorphenyl)-1-ethyl-N-methyl-4-oxo-1,4-dihydro-1,8-naphthyridin-3-carboxamid;
2-Amino-7-(4-{[(3,4-difluorphenyl)sulfonyl]amino}-3-fluorphenyl)-1-ethyl-N-methyl-4-oxo-1,4-dihydro-1,8-naphthyridin-3-carboxamid;
2-Amino-1-ethyl-7-(3-fluor-4-{[(4-fluorphenyl)sulfonyl]amino}phenyl)-N-methyl-4-oxo-1,4-dihydro-1,8-naphthyridin-3-carboxamid;
2-Amino-1-ethyl-7-(3-fluor-4-{[(3-methoxyphenyl)sulfonyl]amino}phenyl)-N-methyl-4-oxo-1,4-dihydro-1,8-naphthyridin-3-carboxamid;
2-Amino-1-ethyl-7-[3-fluor-4-({[6-(morpholin-4-yl)pyridin-3-yl]sulfonyl}amino)phenyl]-N-methyl-4-oxo-1,4-dihydro-1,8-naphthyridin-3-carboxamid;
2-Amino-1-ethyl-7-(3-fluor-4-{[(pyridin-2-ylmethyl)sulfonyl]amino}phenyl)-N-methyl-4-oxo-1,4-dihydro-1,8-naphthyridin-3-carboxamid.

11. Verfahren zur Herstellung einer Verbindung der Formel (I) nach einem der Ansprüche 1 bis 10, **dadurch gekennzeichnet, dass** man eine Verbindung der Formel (IXa): 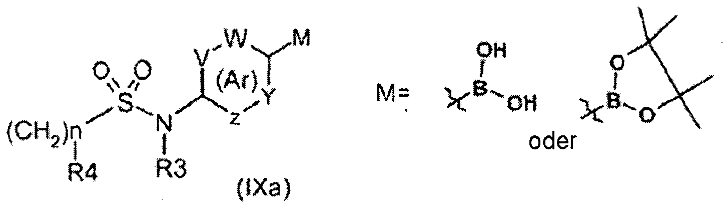 in Gegenwart eines Kupplungskatalysators und einer Base mit einer Verbindung der Formel (VII) 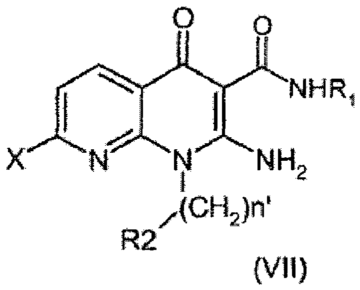 umsetzt, wobei **R1, R2, R3, R4, n, n', V, W, Y, Z** und **Ar** wie in Anspruch 1 definiert sind, **X** für eine Abgangsgruppe steht und **M** wie oben definiert ist.

12. Verfahren zur Herstellung einer Verbindung der Formel (I) nach einem der Ansprüche 1 bis 10, **dadurch gekennzeichnet, dass** man eine Verbindung der Formel (IXb) 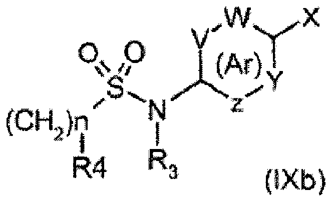 mit einer Verbindung der Formel (VIII) 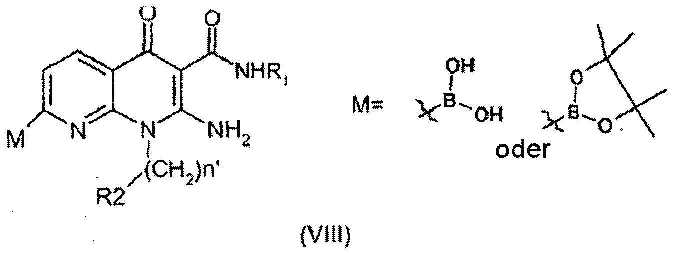 umsetzt, wobei **R1, R2, R3, R4, n, n', V, W, Y, Z** und **Ar** wie in Anspruch 1 definiert sind, **X** für eine Abgangsgruppe steht und **M** wie oben definiert ist.

13. Medikament, **dadurch gekennzeichnet, dass** es eine Verbindung der Formel (I) nach einem der Ansprüche 1 bis 10 oder ein Additionssalz dieser Verbindung mit einer pharmazeutisch unbedenklichen Säure oder auch ein Solvat der Verbindung der Formel (I) umfasst.

14. Pharmazeutische Zusammensetzung, **dadurch gekennzeichnet, dass** sie eine Verbindung der Formel (I) nach einem der Ansprüche 1 bis 10 oder ein pharmazeutisch unbedenkliches Salz oder ein Solvat dieser Verbindung sowie mindestens einen pharmazeutisch unbedenklichen Hilfsstoff umfasst.

15. Verbindung der Formel (I) nach einem der Ansprüche 1 bis 10 zur Verwendung bei der Behandlung und/oder Prävention von proliferativen Erkrankungen wie Krebserkrankungen, chronischen oder akuten Leukämien, lymphozytischen Lymphomen, Hodgkin-Krankheit, myeloproliferativen Syndromen und myelodysplastischen Syndromen.

16. Verbindung der Formel (I) nach einem der Ansprüche 1 bis 10 zur Verwendung bei der Behandlung und/oder Prävention von proliferativen Erkrankungen wie Krebserkrankungen mit soliden Tumoren, beispielsweise Krebserkrankungen der Lunge (NSCLC), der Knochen, der Bauchspeicheldrüse, der Haut, Kaposi-Sarkom, intraokulären Melanomen, Krebserkrankungen der Brust, der Gebärmutter, des Gebärmutterhalses, der Eierstöcke, des Endometriums, der Vagina, der Vulva, der Harnröhre, des Penis, der Prostata, Eileiterkarzinomen, Krebserkrankungen wie GIST und der Analregion, des Rektums, des Dünndarms, des Kolons, des Magens, der Speiseröhre, der endokrinen Drüsen, der Schilddrüse, der Nebenschilddrüse oder der Nebenniere, Weichteilsarkomen, Ewing-Sarkomen, Osteosarkomen, Dermatofibrosarkom und anderen Fibrosarkomen, Krebserkrankungen der Blase oder der Nieren, Neoplasmen des Zentralnervensystems, Tumoren der Wirbelsäule und Desmoiden, Hirnstammgliomen und Glioblastomen, Hypophysenadenom und Metastasen davon, chronischen oder akuten Leukämien, lymphozytischen Lymphomen, Hodgkin-Krankheit und myeloproliferativen Syndromen und myelodysplastischen Syndromen.

17. Verbindung der Formel (I) nach einem der Ansprüche 1 bis 10 zur Verwendung bei der Behandlung und/oder Prävention von nichtmalignen proliferativen Erkrankungen wie Restenose, Atherosklerose, Thrombose, Herzinsuffizienz, Herzhypertrophie, Pulmonalarterienhypertonie, Fibrose, diabetischer Nephropathie, Glomerulonephritis, chronischer Pyelonephritis, Hämangiomen, Autoimmunkrankheiten wie Psoriasis, Sklerodermatitis und Immunsuppression.

18. Kombination mindestens einer Verbindung der Formel (I) nach einem der Ansprüche 1 bis 10 mit einem Chemotherapeutikum.

## Claims

1. Compound corresponding to the formula (I): 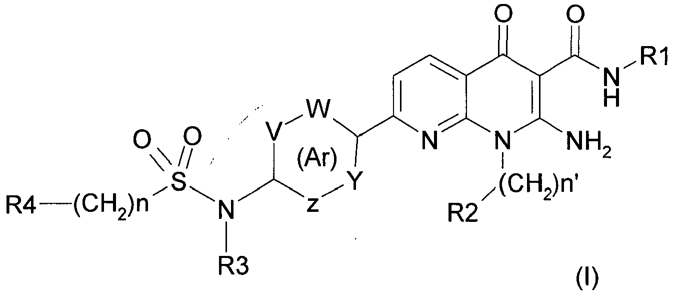 in which
• **n** represents 0, 1, 2 or 3;
• **n'** represents 0, 1, 2, 3 or 4;
• **R1** represents an alkyl group;
• **R2** represents:
(i) a cycloalkyl group,
(ii) an alkyl group, or
(iii) an alkoxy group,
said cycloalkyl, alkyl or alkoxy groups being optionally substituted with one or more halogen atoms;
• **R3** represents:
i) a hydrogen atom, or
ii) a -C(O)alkyl group;
• **Ar** represents a 5- or 6-membered aryl or heteroaryl ring in which **Y, Z, V and W:**
(a) represent, independently of each other,
(i) a =CH- group,
(ii) a =C(R5)- group in which **R5** represents:
o an alkyl group,
o a halogen atom, or
o an alkoxy group,
(iii) a heteroatom chosen from the nitrogen atom, the sulfur atom and the oxygen atom,
(b) at most one among **Y, Z, V and W** being optionally absent,
it being understood that, when **Ar** represents a heteroaryl chosen from pyrrolyl, imidazolyl, pyrazolyl and triazolyls, at least one of the nitrogen atoms of said heteroaryl may be optionally substituted with a group **R6** chosen from an alkyl group,
• **R4** represents a group chosen from:
○ an alkyl group,
○ an alkoxyalkyl group,
○ a group -NRR' with **R and R'**, which may be identical or different, representing, independently of each other, a hydrogen atom, an alkyl group or a -(C3-C6)cycloalkyl group,
○ a cycloalkyl group,
○ an alkenyl group,
○ an aryl group, said group being optionally substituted with at least one halogen atom, and/or with at least one group chosen from a -(C1-C5)alkyl, haloalkyl, nitrile, haloalkyloxy, alkoxy, nitro group and the groups -NRR' with **R and R'**, which may be identical or different, representing, independently of each other, a hydrogen atom or a group chosen from alkyl groups and -(C3-C6)cycloalkyl groups,
○ a heteroaryl group, said group comprising at least one heteroatom chosen from the nitrogen or sulfur atom, said heteroaryl groups being optionally substituted with at least one group chosen from alkyl groups and heterocycloalkyl groups comprising at least one heteroatom chosen from the nitrogen and oxygen atoms;
it being understood that, when the heteroaryl group is chosen from pyrrolyl, imidazolyl, pyrazolyl and triazolyls, at least one of the nitrogen atoms of said heteroaryl may be optionally substituted with a group **R6** chosen from an alkyl group,
o a heterocycloalkyl group comprising at least one heteroatom chosen from the nitrogen, sulfur and oxygen atoms and being optionally substituted with at least one substituent chosen from (i) halogen atoms, (ii) haloalkyl groups, (iii) linear or branched alkyl groups, and (iv) cycloalkyl groups,
it being understood that when the heterocycloalkyl groups are chosen from pyrrolinyl, pyrrolidinyl, imidazolidinyl, imidazolinyl, pyrazolinyl, pyrazolidinyl, piperidinyl, morpholinyl, piperazinyl and thiomorpholinyl, at least one of the nitrogen atoms of said heterocycloalkyl may be optionally substituted with a group **R6** chosen from an alkyl group,
in the form of an acid, a base or an addition salt with an acid or a base.

2. Compound of formula (I) according to Claim 1, **characterized in that**:
**R1** represents a -(C1-C4)alkyl group,
said compounds are in the form of a base or addition salts with an acid.

3. Compound of formula (I) according to either of Claims 1 and 2, **characterized in that**:
**R2** represents a -(C1-C4)alkyl group,
said compounds are in the form of a base or addition salts with an acid.

4. Compound of formula (I) according to any one of Claims 1 to 3, **characterized in that**:
**n'** represents 1,
said compounds are in the form of a base or addition salts with an acid.

5. Compound of formula (I) according to any one of Claims 1 to 4, **characterized in that**:
**R3** represents a hydrogen atom,
said compounds are in the form of a base or addition salts with an acid.

6. Compound of formula (I) according to any one of Claims 1 to 5, **characterized in that**:
**Ar** represents a phenyl,
said compounds are in the form of a base or addition salts with an acid.

7. Compound of formula (I) according to any one of Claims 1 to 6, **characterized in that**:
**R4** represents a group chosen from:
○ an alkyl group;
○ a group -NRR', with **R and R'**, which may be identical or different, representing, independently of each other, a hydrogen atom, an alkyl group or a -(C3-C6)cycloalkyl group,
o an alkenyl group,
o an aryl group, said group being optionally substituted with at least one halogen atom, and/or with at least one group chosen from alkoxy groups and the groups -NRR', with **R and R'** as defined above,
○ a heteroaryl group, said heteroaryl group being optionally substituted with at least one group chosen from alkyl groups and heterocycloalkyl groups comprising at least one heteroatom chosen from the nitrogen and oxygen atoms;
it being understood that, when said heteroaryl group is chosen from pyrrolyl, imidazolyl, pyrazolyl and triazolyls, at least one of the nitrogen atoms of said heteroaryl may be optionally substituted with a group **R6**, with **R6** representing a group chosen from an alkyl group.

8. Compound of formula (I) according to any one of Claims 1 to 7, **characterized in that R4** represents a group chosen from phenyl, pyridinyl and imidazolyl groups.

9. Compound of formula (I) according to any one of Claims 1 to 8, **characterized in that Y, Z, V and W** each represents a =CH group and/or a =C(R5)- group, with **R5** representing a chlorine or fluorine atom, **Y, Z, V and W** being thus in an optionally substituted phenyl group.

10. Compound of formula (I) according to any one of Claims 1 to 9, chosen from the following compounds:
2-amino-1-ethyl-7-(3-fluoro-4-{[(pyridin-3-ylmethyl)sulfonyl]amino}phenyl)-N-methyl-4-oxo-1,4-dihydro-1,8-naphthyridine-3-carboxamide;
2-amino-1-ethyl-7-(3-fluoro-4-{[(3-fluorophenyl)sulfonyl]amino}phenyl)-N-methyl-4-oxo-1,4-dihydro-1,8-naphthyridine-3-carboxamide;
2-amino-7-{4-[(ethenylsulfonyl)amino]-3-fluorophenyl}-1-ethyl-N-methyl-4-oxo-1,4-dihydro-1,8-naphthyridine-3-carboxamide;
2-amino-7-[4-({[2-(dimethylamino)ethyl]sulfonyl}amino)-3-fluorophenyl]-1-ethyl-N-methyl-4-oxo-1,4-dihydro-1,8-naphthyridine-3-carboxamide;
2-amino-7-(4-{[(3-aminobenzyl)sulfonyl]amino}-3-fluorophenyl)-1-ethyl-N-methyl-4-oxo-1,4-dihydro-1,8-naphthyridine-3-carboxamide;
2-amino-1-ethyl-7-(3-fluoro-4-{[(1-methyl-1H-imidazol-4-yl)sulfonyl]amino}phenyl)-N-methyl-4-oxo-1,4-dihydro-1,8-naphthyridine-3-carboxamide;
2-amino-7-{4-[(butylsulfonyl)amino]-3-fluorophenyl}-1-ethyl-N-methyl-4-oxo-1,4-dihydro-1,8-naphthyridine-3-carboxamide;
2-amino-7-(3-chloro-4-{[(2,3-dichlorophenyl)sulfonyl]amino}phenyl)-1-ethyl-N-methyl-4-oxo-1,4-dihydro-1,8-naphthyridine-3-carboxamide;
2-amino-7-(4-{[(2,5-dichlorophenyl)sulfonyl]amino}-3-fluorophenyl)-1-ethyl-N-methyl-4-oxo-1,4-dihydro-1,8-naphthyridine-3-carboxamide;
2-amino-1-ethyl-N-methyl-4-oxo-7-{4-[(pyridin-3-ylsulfonyl)amino]phenyl}-1,4-dihydro-1,8-naphthyridine-3-carboxamide;
2-amino-7-(4-{[(2,6-dichlorophenyl)sulfonyl]amino}-3-fluorophenyl)-1-ethyl-N-methyl-4-oxo-1,4-dihydro-1,8-naphthyridine-3-carboxamide;
2-amino-7-(2-chloro-4-{[(2,5-dichlorophenyl)sulfonyl]amino}phenyl)-1-ethyl-N-methyl-4-oxo-1,4-dihydro-1,8-naphthyridine-3-carboxamide;
2-amino-7-(2-chloro-4-{[(2,3-dichlorophenyl)sulfonyl]amino}phenyl)-1-ethyl-N-methyl-4-oxo-1,4-dihydro-1,8-naphthyridine-3-carboxamide;
2-amino-7-(4-{[(2,3-dichlorophenyl)sulfonyl]amino}-2-fluorophenyl)-1-ethyl-N-methyl-4-oxo-1,4-dihydro-1,8-naphthyridine-3-carboxamide;
2-amino-7-(4-{[(2,3-dichlorophenyl)sulfonyl]amino}-3-methylphenyl)-1-ethyl-N-methyl-4-oxo-1,4-dihydro-1,8-naphthyridine-3-carboxamide;
2-amino-1-ethyl-N-methyl-7-{4-[(methylsulfonyl)amino]phenyl}-4-oxo-1,4-dihydro-1,8-naphthyridine-3-carboxamide;
2-amino-7-(4-{[(2,3-dichlorophenyl)sulfonyl]amino}phenyl)-1-ethyl-N-methyl-4-oxo-1,4-dihydro-1,8-naphthyridine-3-carboxamide;
2-amino-7-(4-{[(2,3-dichlorophenyl)sulfonyl]amino}-3-fluorophenyl)-1-ethyl-N-methyl-4-oxo-1,4-dihydro-1,8-naphthyridine-3-carboxamide;
2-amino-7-(4-{[(2-chlorophenyl)sulfonyl]amino}phenyl)-1-ethyl-N-methyl-4-oxo-1,4-dihydro-1,8-naphthyridine-3-carboxamide;
2-amino-1-ethyl-7-(3-fluoro-4-{[(2-fluorophenyl)sulfonyl]amino}phenyl)-N-methyl-4-oxo-1,4-dihydro-1,8-naphthyridine-3-carboxamide;
2-amino-7-(4-{[(4-chlorophenyl)sulfonyl]amino}-3-fluorophenyl)-1-ethyl-N-methyl-4-oxo-1,4-dihydro-1,8-naphthyridine-3-carboxamide;
2-amino-7-(4-{[(3-chlorophenyl)sulfonyl]amino}-3-fluorophenyl)-1-ethyl-N-methyl-4-oxo-1,4-dihydro-1,8-naphthyridine-3-carboxamide;
2-amino-7-(4-{[(3,4-difluorophenyl)sulfonyl]amino}-3-fluorophenyl)-1-ethyl-N-methyl-4-oxo-1,4-dihydro-1,8-naphthyridine-3-carboxamide;
2-amino-1-ethyl-7-(3-fluoro-4-{[(4-fluorophenyl)sulfonyl]amino}phenyl)-N-methyl-4-oxo-1,4-dihydro-1,8-naphthyridine-3-carboxamide;
2-amino-1-ethyl-7-(3-fluoro-4-{[(3-methoxyphenyl)sulfonyl]amino}phenyl)-N-methyl-4-oxo-1,4-dihydro-1,8-naphthyridine-3-carboxamide;
2-amino-1-ethyl-7-[3-fluoro-4-({[6-(morpholin-4-yl)pyridin-3-yl]sulfonyl}amino)phenyl]-N-methyl-4-oxo-1,4-dihydro-1,8-naphthyridine-3-carboxamide;
2-amino-1-ethyl-7-(3-fluoro-4-{[(pyridin-2-ylmethyl)sulfonyl]amino}phenyl)-N-methyl-4-oxo-1,4-dihydro-1,8-naphthyridine-3-carboxamide.

11. Method for preparing a compound of formula (I) according to any one of Claims 1 to 10, **characterized in that** a compound of formula (IXa): 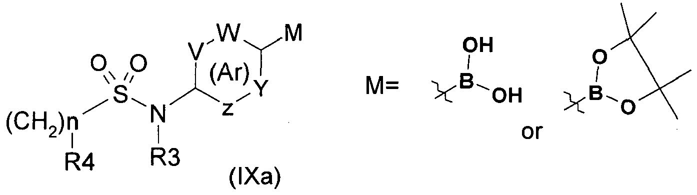 is reacted with a compound of formula (VII), in the presence of a coupling catalyst and a base, 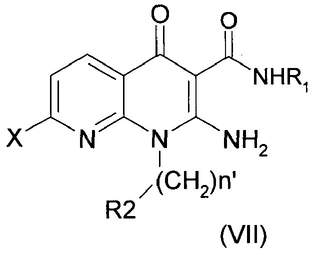 where **R1, R2, R3, R4, n, n', V, W, Y, Z and Ar** are as defined in claim 1, **X** represents a leaving group and **M** is as defined above.

12. Method for preparing a compound of formula (I) according to any one of Claims 1 to 10, **characterized in that** a compound of formula (IXb) 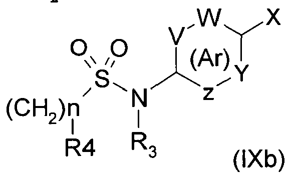 is reacted with a compound of formula (VIII), 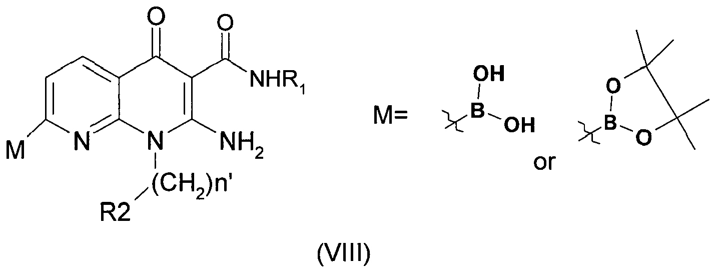 where **R1, R2, R3, R4, n, n', V, W, Y, Z and Ar** are as defined in claim 1, **X** represents a leaving group and **M** is as defined above.

13. Medicament, **characterized in that** it comprises a compound of formula (I) according to any one of Claims 1 to 10, or an addition salt of this compound with a pharmaceutically acceptable acid, or else a solvate of the compound of formula (I).

14. Pharmaceutical composition, **characterized in that** it comprises a compound of formula (I) according to any one of Claims 1 to 10, or a pharmaceutically acceptable salt, or a solvate of this compound, and at least one pharmaceutically acceptable excipient.

15. Compound of formula (I) according to any one of Claims 1 to 10, for its use in the treatment and/or prevention of proliferative diseases such as cancers, chronic or acute leukemias, lymphocytic lymphomas, Hodgkin's disease, and myeloproliferative syndromes, and myelodysplastic syndromes.

16. Compound of formula (I) according to any one of Claims 1 to 10, for its use in the treatment and/or prevention of proliferative diseases such as solid tumor cancers, for example cancers of the lung (NSCLC), of the bones, of the pancreas, of the skin, Kaposi's syndrome, intraocular melanomas, cancers of the breast, of the uterus, of the cervix, of the ovaries, of the endometrium, of the vagina, of the vulva, of the urethra, of the penis, of the prostate, fallopian tube carcinomas, cancers such as GISTs and of the anal region, of the rectum, of the small intestine, of the colon, of the stomach, of the esophagus, of the endocrine, thyroid, parathyroid or adrenal glands, soft tissue sarcomas, Ewing's sarcomas, ostesarcomas, dermatofibrosarcoma and other fibrosarcomas, cancers of the bladder or of the kidney, neoplasms of the central nervous system, vertebral column and desmoid tumors, brain stem gliomas and glioblastomas, pituitary adenomas and metastases thereof, chronic or acute leukemias, lymphocytic lymphomas, Hodgkin's disease and myeloproliferative syndromes, and myelodysplastic syndromes.

17. Compound of formula (I) according to any one of Claims 1 to 10, for its use in the treatment and/or prevention of nonmalignant proliferative diseases such as restenosis, atherosclerosis, thrombosis, heart failure, cardiac hypertrophy, pulmonary arterial hypertension, fibrosis, diabetic nephropathy, glomerulonephritis, chronic pyelonephritis, hemangiomas, autoimmune diseases such as psoriasis, sclerodermatitis, immunosuppression.

18. Combination of at least one compound of formula (I) according to any one of Claims 1 to 10 with at least one chemotherapeutic agent.
